(19) **Europäisches Patentamt / European Patent Office / Office européen des brevets**

(11) **EP 4 617 287 A1**

(12) **EUROPEAN PATENT APPLICATION**
published in accordance with Art. 153(4) EPC

(43) Date of publication:
**17.09.2025 Bulletin 2025/38**

(21) Application number: **23888009.0**

(22) Date of filing: **07.11.2023**

(51) International Patent Classification (IPC):
*C07K 19/00* (2006.01)      *C12N 15/62* (2006.01)
*C12N 5/10* (2006.01)      *C12N 15/85* (2006.01)
*A61K 38/17* (2006.01)      *A61P 7/04* (2006.01)
*A61P 43/00* (2006.01)

(52) Cooperative Patent Classification (CPC):
**A61K 38/17; A61K 45/06; A61K 47/68; A61P 7/00;
A61P 7/04; A61P 31/00; A61P 37/02; A61P 43/00;
C07K 19/00; C12N 5/10; C12N 15/62; C12N 15/85;
G01N 33/68**

(86) International application number:
**PCT/CN2023/130258**

(87) International publication number:
**WO 2024/099320 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 10.11.2022   CN 202211403394
22.09.2023   CN 202311235258

(71) Applicant: **Longbio Pharma (Suzhou) Co., Ltd.
Shanghai 201203 (CN)**

(72) Inventors:
- **MA, Haili
  Shanghai 201203 (CN)**
- **LIU, Heng
  Shanghai 201203 (CN)**

(74) Representative: **Bianchetti & Minoja with
Trevisan & Cuonzo IPS SRL
Via Plinio, 63
20129 Milano (IT)**

Remarks:
The complete document including Reference
Table(s) and the Sequence Listing(s) can be
downloaded from the EPO website

(54) **COMPLEMENT-INHIBITORY HYBRID PROTEIN MUTANT AND ANTIBODY FUSION PROTEIN THEREOF**

(57)    A hybrid protein containing a CFH functional unit and aDAFfunctional unt,and a mutant thereof, an encoding nucleicacid thereof, and a method and the use thereof for treating diseases or conditions related to a complement system,and a fusion proteinconstructed on the basis of the hybnd protein mutant and an antibody specifically binding to complement C5.Further provided area polynucleotide for encoding said fusion protein,an expression vector and a host cell,a pharmaceutical composition thereof, and amethod and the use thereof for treating C5-protein-related diseases.

EP 4 617 287 A1

**Description**

The present invention relates to a hybrid protein comprising a CFH functional unit and a DAF functional unit and a mutant thereof, an encoding nucleic acid thereof, and methods and a use thereof for treating a disease or disorder related to the complement system; the invention further relates to a fusion protein constructed based on the hybrid protein mutant and an antibody that specifically binds to complement C5. The present invention also relates to a polynucleotide, encoding the fusion protein, an expression vector, and a host cell, and a pharmaceutical composition thereof, and a method and use for treating C5 protein-related diseases.

**Background technique**

The complement system is consisted of more than 30 types of soluble protein molecules and is part of the natural immune system. Its components include more than 30 types of molecules such as intrinsic complement components, various regulatory factors, and complement receptors. The complement system can be activated through three relatively independent but interrelated pathways, thereby exerting various biological effects such as opsonizing phagocytosis, cell lysis, mediating inflammation, immune regulation and clearing immune complexes, including enhancing phagocytosis, enhancing chemotaxis of cell phagocytosis, increasing vascular permeability, neutralizing viruses, cytolysis, regulating immune responses, etc. While complement activation provides a valuable first line of defense against potential pathogens, complement activation that promotes protective inflammatory responses may also present itself as a potential threat to the host. Complement activation and its deposition on target structures can also indirectly cause cell or tissue destruction. Complement activation products that mediate tissue damage are produced at various points in the complement pathway. Inappropriate complement activation on host tissues plays an important role in the pathology of many autoimmune and inflammatory diseases.

Complement proteins include proteins represented as C1 to C9, and these proteins are continuously activated through three different pathways (classical pathway, lectin pathway, alternative pathway) to elicit an immune response. Complement C5 is the key component of complement and plays an important role in inflammation and cell killing. This protein consists of alpha and beta polypeptide chains linked by disulfide bonds. Activating peptide C5a is an anaphylatoxin that induces inflammatory responses in various cells via C5aR (CD88) and C5L2 (GPR77). It has potent spasmogenic and chemotactic activities. It is derived from $\alpha$ polypeptide by cleavage with C5 convertase. The C5b macromolecular cleavage product can form a complex with C6 complement components, and this complex is the basis for the formation of the membrane attack complex (MAC), which includes additional complement components. The complement system can be highly cytotoxic to host cells if it is not properly controlled or if it is overactivated.

Numerous studies have shown that complement C5 is related to various diseases, such as various diseases related to the hemolytic activity of human complement.

The anti-C5 monoclonal antibody eculizumab (Soliris (registered trademark)) exhibits high affinity for complement C5 and inhibits complement activation by inhibiting the cleavage of C5 into C5a/C5b and the accompanying formation of the membrane attack complex. Thus, eculizumab exhibits an inhibitory effect on hemolysis and therefore serves as a therapeutic agent for paroxysmal nocturnal hemoglobinuria and atypical hemolytic uremic syndrome. In addition, eculizumab is known as a therapeutic agent for generalized myasthenia gravis (gMG).

In the in vitro hemolysis inhibitory activity test, although eculizumab showed good hemolysis inhibition effect in the classical pathway, its activity in blocking the complement alternative pathway (AP) was insufficient, so its efficacy could not fully meet the needs of patients. For example, some patients with PNH who have undergone eculizumab (Soliris) treatment will still be unable to get rid of blood transfusion dependence due to extravascular hemolysis.

In addition, the complement system is also tightly regulated by a range of proteins (complement regulatory proteins) because lack of regulation or inappropriate activation of complement activation can lead to damage to host tissues. Among them, complement activation regulatory factor (RCA) family proteins are mainly responsible for complement regulation. RCA proteins include membrane proteins such as decay-accelerating factor (DAF; CD55), membrane cofactor protein (MCP; CD46) and complement receptor 1 (CR1; CD35), as well as fluid-phase proteins such as Factor H (FH or CFH) and C4b-binding protein (C4BP). The RCA protein structure consists of complement control protein repeat (CCP) modules, of which 2-4 consecutive modules contribute to regulatory functions known as decay-accelerating activity (DAA) and cofactor activity (CFA). RCA proteins act by targeting the C3/C5 convertase, the central enzyme of the complement pathway. RCA proteins bind to these convertases or their non-catalytic subunits to inactivate these enzymes. The DAA was displayed as the irreversible dissociation of the invertase into subunits after binding to the RCA protein, whereas the CFA was displayed as the cleavage and inactivation of the invertase via the recruitment of serine protease Factor I (FI) after the binding RCA protein to the non-catalytic subunits (C3b/C4b), thereby preventing the formation of C3 convertase. Factor H is an opsonin and ligand for complement receptors 2 and 3, acting as a cofactor for Factor I in the catalyzed cleavage of C3b to iC3b, and inhibiting C3b amplification. It accelerates the irreversible dissociation of C3 convertase C3bBb in the alternative pathway, and may also compete with Factor B for C3b binding during the formation of proconvertase. Factor H is a soluble complement regulator essential for protecting surfaces, including extracellular matrix ECM. FH binds to the peptide hormone adrenomedullin and may prevent its degradation. The FH plays a role in managing cellular senescence, stress, or injury through interactions with C-reactive proteins, pentameric proteins, DNA, histones, annexin II, the malondialdehyde acetaldehyde adduct of proteins, and oxidized lipids. FHL1 also has factor I cofactor activity and C3bBb attenuation acceleration activity (The Complement FactsBook. Edited by: Scott Barnum and Theresa Schein, Copyright © 2018 Elsevier Ltd. All rights reserved. https://doi.org/10.1016/C2015-0-06595-9, chapter 30).

DAF intrinsically protects host cells from autologous complement attack by preventing the formation and accelerating the decay of classical and alternative C3 and C5 convertases, thereby inhibiting the cleavage of C3 and C5. When purified DAF is added to cells, it can be incorporated into the cell membrane, thus displaying functional activity. DAF has also been found to regulate T cell tolerance and thus negatively regulate several animal models of autoimmune diseases (The Complement FactsBook. Edited by: Scott Barnum and Theresa Schein, Copyright © 2018 Elsevier Ltd. All rights reserved. https://doi.org/10.1016/C2015-0-06595-9, chapter 25).

Therefore, there is still a need in the art to develop new proteins with stronger complement regulatory activity, and fusion proteins based on anti-C5 antibody and the new complement regulatory proteins, especially to optimize the complement regulatory proteins by mutation, and fusion proteins obtained based on the preferred proteins, which have improved property and developability, e.g., have better efficacy, or have improved solubility and purity while maintaining activity, etc.

**Description of the Invention**

The invention relates to a hybrid protein and a mutant thereof with better complement regulatory activity, and this hybrid protein and the mutant thereof can have advantages such as better therapeutical effect.

In some embodiments, the hybrid protein and a mutant thereof of the present invention further comprise less CCP of DAF on the basis of comprising CPP1/SCR1 of CFH, so that a mutant of the hybrid protein having a smaller molecular weight can be obtained. The hybrid proteins and mutants thereof, when in the therapeutic applications, can be administered at the same mass concentration with higher molarity doses, and thus have the potential of better therapeutic efficacy or longer dosing intervals, as compared to other typically used complement regulatory proteins with larger molecular weight (especially compared to hybrid proteins that also comprise other CCPs of CFH), e.g. for use in such as ophthalmic, brain medications. In other cases, smaller molecular weights are also more conducive to tissue penetration, such as tumor tissue, blood-brain barrier, and the like.

In other embodiments, the hybrid proteins and mutants thereof of the invention have better complement-inhibiting activity, including the activity in inhibiting the Alternative Pathway (AP), the Classical Pathway (CP) and/or C3b deposition. In some embodiments, the hybrid proteins and mutants thereof is effective to block human complement hemolytic activity. In some embodiments, the mutant of the hybrid protein of the invention is effective in inhibiting hemolysis of the human complement classical pathway and/or hemolysis of the human complement alternative pathway. In some embodiments, a mutant of the hybrid protein of the present invention is effective in inhibiting C3b deposition.

Further, the invention relates to a hybrid protein of CFH and DAF, wherein CCP1/SCR1 of CFH is hybridized with CCP of DAF (e.g., CCP3-4/SCR3-4 of DAF) to form the hybrid protein.

In some embodiments, the invention relates to a mutant of the hybrid protein having improved purity or improved aggregate characteristics or an improved expression amount compared to the parent hybrid protein. For example, the mutant of the hybrid protein of the invention has a reduced (major) aggregate proportion or increased purity compared to the parent hybrid protein. For another example, the mutant of the hybrid protein of the invention has an increased expression amount compared to the parent hybrid protein. As another example, the mutant of the hybrid protein of the invention has a reduced (major) aggregate proportion while has an improved expression amount compared to the parent hybrid protein. In some embodiments, the invention relates to a mutant of the hybrid protein having a reduced aggregate proportion, an increased expression amount, and a substantially comparable activity (e.g., complement inhibitory activity) compared to the parent protein (e.g., the parent hybrid protein).

In some embodiments, the invention relates to a fusion protein comprising an anti-C5 antibody, comprising an anti-C5 antibody, and a complement regulatory protein optimized by mutation (e.g., a hybrid protein of a complement regulatory protein, e.g., the hybrid protein mutant of the invention). In some embodiments, compared with an anti-C5 antibody, the fusion protein (1) further improves the complement classical pathway (CP) blocking activity and enhances drug efficacy; (2) significantly improves the complement alternative pathway (AP) blocking activity. In a further embodiment, the fusion protein has a stronger activity in inhibiting the deposition of C3b on the cell surface than the C5 antibody fusion protein known in the art, wherein C3b deposition is considered to be the main reason for developing extravascular hemolysis in PNH patients. In some embodiments, the fusion proteins of the invention have improved solubility while maintaining their activity.

In some embodiments, the present invention relates to the following specific embodiments:

1. A hybrid protein mutant, wherein the parent hybrid protein comprises or consists of

(a) CCP1 of human complement factor H (CFH),
(b) CCP3 and CCP4 of human decay-accelerating factor (DAF), and

optionally a signal peptide and/or a tag;

wherein compared to the parent hybrid protein, the mutant comprises one or more mutations selected from the group consisting of:

mutations at position 56, 59, 82, 86, 107, 109, and/or 110, e.g., substitutions, e.g., substitutions to an amino acid that is more hydrophilic than the original amino acid; and/or mutations comprised at the C-terminus, e.g., addition of one or more amino acids at the C-terminus;
wherein the positions of amino acid mutations are numbered corresponding to the amino acid positions as set forth in SEQ ID NO:20.

2. The hybrid protein mutant of embodiment 1, wherein the optimized hybrid protein comprises substitutions at position 59, 82 and 110 numbered corresponding to the amino acid positions as set forth in SEQ ID NO:20, and/or addition of one or more amino acids at the C-terminus.

3. The hybrid protein mutant of embodiment 2 comprising substitutions at position 56, 86, 107 and/or 109 numbered corresponding to the amino acid positions as set forth in SEQ ID NO:20.

4. The hybrid protein mutant of embodiment 2 comprising substitutions at position 56, 59, 82 and/or 110 numbered corresponding to the amino acid positions as set forth in SEQ ID NO:20, e.g., substitutions at 59, 82 and 110, or substitutions at 56, 59, 82 and 110.

5. The hybrid protein mutant of any one of embodiments 1-4, wherein the mutation(s) are selected from the group consisting of:

substitutions of the amino acids at position 56, 59, 82, 86, 107, 109 and/or 110 numbered corresponding to the amino acid position as set forth in SEQ ID NO:20, to amino acids which is more hydrophilic than the original amino acids, for example to D, E, S, Y or T; or
addition of 1-5 amino acids at the C-terminus, for example 2 amino acids.

6. The hybrid protein mutation of any one of embodiments 1-5, wherein the mutation(s) are selected from the group consisting of:
56D/E, 59D/S, 82D, 86Y, 107E, 109D/E, 110T or an addition of the amino acid KS at the C-terminus, or a combination thereof.

7. The hybrid protein mutant of embodiment 6, comprising

56E;
59D/S;
82D;
86Y;
107E;
109D/E;
110T;
59D-82D-110T;
59S-82D-110T;
59D-82D-110T-56E;
59S-82D-110T-56E, or
an addition of the amino acids KS at the C-terminus.

8. The hybrid protein mutant of any one of embodiments 1-7, wherein the CCP3 and CCP4 of DAF are directly linked together to form CCP3-4.

9. The hybrid protein mutant of any one of embodiments 1-8, wherein the CCP1 of human CFH comprises or consists of the amino acid sequence at positions 19-82 of the human CFH protein, optionally the CCP1 comprises a V62I mutation, wherein the amino acid positions are numbered corresponding to the amino acid positions as set forth in SEQ ID NO:3.

10. The hybrid protein mutant of any one of embodiments 1-9, wherein the CCP1 of human CFH comprises or consists of the amino acid sequence at positions 19-84 of the human CFH protein, optionally, CCP1 comprises a V62I mutation, wherein the amino acid positions are numbered corresponding to the amino acid positions as set forth in SEQ ID NO:3.

11. The hybrid protein mutant of any one of embodiments 1-10, wherein the CCP3 of human DAF comprises or consists of the amino acid sequence at positions 161-222 of the human DAF protein, and/or the CCP4 of human DAF comprises or consists of the amino acid sequence at positions 223-285 of the human DAF protein, wherein the amino acid positions are numbered corresponding to the amino acid positions as set forth in SEQ ID NO: 1.

12. The hybrid protein mutant of any one of embodiments 1-11, wherein the CCP3 of human DAF comprises or consists of the amino acid sequence at positions 163-222 of the human DAF protein, and/or the CCP4 of human DAF comprises or consists of the amino acid sequence at positions 223-285 of the human DAF protein, wherein the amino acid positions are numbered corresponding to the amino acid positions as set forth in SEQ ID NO: 1.

13. The hybrid protein mutant of any one of embodiments 1-12, wherein the CCP3-4 of human DAF comprises or consists of the amino acid sequence at positions 161-285 of the human DAF protein, wherein the amino acid positions are numbered corresponding to the amino acid positions as set forth in SEQ ID NO: 1.

14. The hybrid protein mutant of any one of embodiments 1-13, wherein the CCP3-4 of human DAF comprises or consists of the amino acid sequence at positions 163-285 of the human DAF protein, wherein the amino acid positions are numbered corresponding to the amino acid positions as set forth in SEQ ID NO: 1.

15. The hybrid protein mutant of any one of embodiments 1-14, wherein

> (1) the CCP1 of human CFH comprises or consists of the amino acid sequences at positions 19-82 of the human CFH protein, and the CCP3 of human DAF comprises or consists of the amino acid sequence at positions 161-222 of the human DAF protein, and the CCP4 of human DAF comprises or consists of the amino acid sequence at positions 223-285 of the human DAF protein;
> (2) the CCP1 of human CFH comprises or consists of the amino acid sequences at positions 19-84 of the human CFH protein, and the CCP3 of human DAF comprises or consists of the amino acid sequence at positions 163-222 of the human DAF protein, and the CCP4 of human DAF comprises or consists of the amino acid sequence at positions 223-285 of the human DAF protein;
> (3) the CCP1 of human CFH comprises or consists of the amino acid sequence at positions 19-82 of the human CFH protein, and the CCP3-4 of human DAF comprises or consists of the amino acid sequence at positions 161-285 of the human DAF protein; or
> (4) the CCP1 of human CFH comprises or consists of the amino acid sequence at positions 19-84 of the human CFH protein, and the CCP3-4 of human DAF comprises or consists of the amino acid sequence at positions 163-285 of the human DAF protein;

wherein the amino acid positions of the human CFH protein are numbered corresponding to the amino acid positions as set forth in SEQ ID NO: 3 and the amino acid positions of the human DAF protein are numbered corresponding to the amino acid positions as set forth in SEQ ID NO: 1.

16. The hybrid protein mutant of any one of embodiments 1-15, wherein the CCP1 of human CFH has the V62I mutation.

17. The hybrid protein mutant of any oen of embodiments 1-16, wherein the human CFH protein is a human native CFH protein or comprises

> (i) the amino acid sequence as set forth in SEQ ID NO: 3 or 5;
> (ii) the amino acid sequence encoded by the nucleotide sequence as set forth in SEQ ID NO: 4 or 6;
> (iii) the amino acid sequence having at least 95%, 96%, 97%, 98%, or 99% identity to the amino acid sequence as set forth in (i) or (ii);

or consists of the amino acid sequence as set forth in any one of (i) to (iii).

18. The hybrid protein mutant of any one of embodiments 1-17, wherein the human DAF protein is a native human DAF protein or comprises

> (i) the amino acid sequence as set forth in SEQ ID NO: 1;
> (ii) the amino acid sequence encoded by the nucleotide sequence as set forth in SEQ ID NO: 2;
> (iii) the amino acid sequence having at least 95%, 96%, 97%, 98%, or 99% identity to the amino acid sequence as set forth in (i) or (ii);

or consists of the amino acid sequence as set forth in any one of (i) to (iii).

19. The hybrid protein mutant of any one of embodiments 1-18, wherein

the CCP1 of human CFH comprises or consists of the amino acid sequence as set forth in SEQ ID NO: 12, 13, 14, or 15, or an amino acid sequence having at least 95%, 96%, 97%, 98%, or 99% identity to the amino acid sequence as set forth in SEQ ID NO: 12, 13, 14, or 15;

the CCP3 of human DAF comprises or consists of the amino acid sequence as set forth in SEQ ID NO: 7 or 8, or an amino acid sequence having at least 95%, 96%, 97%, 98%, or 99% identity to the amino acid sequence as set forth in SEQ ID NO: 7 or SEQ ID NO: 8;

the CCP4 of human DAF comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9; and/or

the CCP3-4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.

20. The hybrid protein mutant of any one of embodiments 1-19, wherein the tag is, for example, a purification tag such as a hexahistidine tag or a biotin marker, comprising, e.g., the amino acid sequence as set forth in SEQ ID NO: 16.

21. The hybrid protein mutant of any one of embodiments 1-20, wherein the signal peptide is a secretory signal peptide, comprising, e.g., the amino acid sequence as set forth in SEQ ID NO: 17.

22. The hybrid protein mutant of any one of embodiments 1-21, wherein the parent hybrid protein comprises or consists of the amino acid sequence of any one of SEQ ID NOs: 18-33, or an amino acid sequence having at least a 95%, 96%, 97%, 98%, or 99% identity to the amino acid sequence.

23. The hybrid protein mutant of embodiment 22, wherein the parent hybrid protein comprises or consists of the amino acid sequence of SEQ ID NO:20, or an amino acid sequence having at least a 95%, 96%, 97%, 98%, or 99% identity to the amino acid sequence.

24. The hybrid protein mutant of any one of embodiments 1-23, comprising or consisting of the amino acid sequence of any one of SEQ ID NOs: 53-54, 56-58, 60, 62, 64, 66, 68, 70, 72, 74, 76, 78, 80, 82, 84, 86, 88-96, or an amino acid sequence having at least a 95%, 96%, 97%, 98%, or 99% identity to the amino acid sequence.

25. The hybrid protein mutant of any one of embodiments 1-23,

(1) comprising an amino acid sequence having at least 95%, 96%, 97%, 98%, 99% identity with the amino acid sequence as set forth in SEQ ID NO:60, and comprising an addition of KS at the C-terminus;

(2) comprising an amino acid sequence having at least 95%, 96%, 97%, 98%, 99% identity with the amino acid sequence as set forth in SEQ ID NO:62, and comprising an addition of KS at the C-terminus and a mutation of 56E;

(3) comprising an amino acid sequence having at least 95%, 96%, 97%, 98%, 99% identity with the amino acid sequence as set forth in SEQ ID NO:64, and comprising an addition of KS at the C-terminus and a mutation of 59D;

(4) comprising an amino acid sequence having at least 95%, 96%, 97%, 98%, 99% identity with the amino acid sequence as set forth in SEQ ID NO:66, and comprising an addition of KS at the C-terminus and a mutation of 59S;

(5) comprising an amino acid sequence having at least 95%, 96%, 97%, 98%, 99% identity with the amino acid sequence as set forth in SEQ ID NO:68, and comprising an addition of KS at the C-terminus and a mutation of 82D;

(6) comprising an amino acid sequence having at least 95%, 96%, 97%, 98%, 99% identity with the amino acid sequence as set forth in SEQ ID NO:70, and comprising an addition of KS at the C-terminus and a mutation of 86Y;

(7) comprising an amino acid sequence having at least 95%, 96%, 97%, 98%, 99% identity with the amino acid sequence as set forth in SEQ ID NO:72, and comprising an addition of KS at the C-terminus and a mutation of 107E;

(8) comprising an amino acid sequence having at least 95%, 96%, 97%, 98%, 99% identity with the amino acid sequence as set forth in SEQ ID NO:74, and comprising an addition of KS at the C-terminus and a mutation of 109D;

(9) comprising an amino acid sequence having at least 95%, 96%, 97%, 98%, 99% identity with the amino acid sequence as set forth in SEQ ID NO:76, and comprising an addition of KS at the C-terminus and a mutation of 109E;

(10) comprising an amino acid sequence having at least 95%, 96%, 97%, 98%, 99% identity with the amino acid sequence as set forth in SEQ ID NO:78, and comprising an addition of KS at the C-terminus and a mutation of 110T;

(11) comprising an amino acid sequence having at least 95%, 96%, 97%, 98%, 99% identity with the amino acid sequence as set forth in SEQ ID NO:80, and comprising KS at the C-terminus and a mutation of 59D-82D-110T;

(12) comprising an amino acid sequence having at least 95%, 96%, 97%, 98%, 99% identity with the amino acid sequence as set forth in SEQ ID NO:82, and comprising KS at the C-terminus and a mutation of 59S-82D-110T;

(13) comprising an amino acid sequence having at least 95%, 96%, 97%, 98%, 99% identity with the amino acid sequence as set forth in SEQ ID NO:84, and comprising KS at the C-terminus and a mutation of 59D-82D-110T-56E;

(14) comprising an amino acid sequence having at least 95%, 96%, 97%, 98%, 99% identity with the amino acid sequence as set forth in SEQ ID NO:86, and comprising KS at the C-terminus and a mutation of 59S-82D-110T-56E.

26. A fusion protein comprising an anti-C5 antibody or antigen-binding fragment thereof and the hybrid protein mutant of any one of embodiments 1-25, wherein the antibody or antigen-binding fragment thereof is linked to the hybrid protein mutant with or without a linker.

27. The fusion protein of embodiment 26, wherein the one or more hybrid protein mutants (at their N-terminus or at their C-terminus) are respectively connected to the N-terminus and/or C-terminus of the heavy chain and/or light chain of the anti-C5 antibody, with or without a linker.

28. The fusion protein of any one of embodiments 1-27, wherein the anti-C5 antibody is a humanized or chimeric antibody.

29. The fusion protein of any one of embodiments 1-28, wherein the anti-C5 antibody is a monoclonal antibody.

30. The fusion protein of any one of embodiments 1-29, wherein the antigen-binding fragment of the anti-C5 antibody is selected from Fab, Fab', Fab'-SH, Fv, singel chain antibody(e.g., scFv), $(Fab')_2$, single domain antibody e.g., VHH, dAb(domain antibody), or a linear antibody.

31. The fusion protein of any one of embodiments 1-30, wherein the anti-C5 antibody or antigen-binding fragment thereof comprises an Fc region, preferably, wherein the Fc region is connected at its C-terminus to the N-terminus of the hybrid protein, with or without a linker.

32. The fusion protein of any one of embodiments 1-31, wherein the linker is selected from one or more glycine $(G)_n$, GS, $G_nS$, $G_nS_n$, $(G_nS)_n$ or $(GSG)_n$ or $(G_4S)_n$, wherein n is an integer equal to or greater than 1, for example, n is an integer of 2, 3, 4, 5, 6 or 7, for example, the linker is G, GSG or $G_4S$.

33. The fusion protein of any one of embodiments 1-32, comprising the full-length anti-C5 antibody and the hybrid protein, wherein

the anti-C5 antibody is connected at the C-terminus of its Fc region to the N-terminus of the hybrid protein to form the heavy chain of the fusion protein (with or without a linker);
the light chain of the anti-C5 antibody forms the light chain of the fusion protein.

34. The fusion protein of any one of embodiments 1-33, wherein said anti-C5 antibody or antigen-binding fragment thereof is selected from

i) the anti-C5 antibody or the antigen-binding fragment thereof disclosed in CN113754763A; or
ii) Eculizumab, Ravulizumab, Pozelimab, Crovalimab, Tesidolumab or the antigen-binding fragment thereof.

35. The fusion protein of any one of embodiments 1-33, wherein the anti-C5 antibody or antigen-binding fragment thereof comprising three complementarity determining regions (CDRs) of a heavy chain variable region (VH), HCDR1, HCDR2, and HCDR3, and three CDRs of a light chain variable region (VL), LCDR1, LCDR2, and LCDR3, wherein the HCDR1, HCDR2, and HCDR3 are the three CDRs contained in the VH as set forth in SEQ ID NO: 39; and the LCDR1, LCDR2, and LCDR3 are the three CDRs contained in the VL as set forth in SEQ ID NO: 45.

36. The fusion protein of any one of embodiments 1-33, wherein the anti-C5 antibody or antigen-binding fragment thereof comprising:

- HCDR1 comprising or consisting of the amino acid sequence as set forth in SEQ ID NO: 40,
- HCDR2 comprising or consisting of the amino acid sequence as set forth in SEQ ID NO: 41,
- HCDR3 comprising or consisting of the amino acid sequence as set forth in SEQ ID NO: 42,
- LCDR1 comprising or consisting of the amino acid sequence as set forth in SEQ ID NO: 46,
- LCDR2 comprising or consisting of the amino acid sequence as set forth in SEQ ID NO: 47, and
- LCDR3 comprising or consisting of the amino acid sequence as set forth in SEQ ID NO: 48.

37. The fusion protein of embodiment 35 or 36, wherein the anti-C5 antibody or antigen-binding fragment thereof comprises a heavy chain variable region VH, wherein the heavy chain variable region

(i) comprises or consists of an amino acid sequence having at least 90%, 91%, 92%, 93%, 94%, 95%, 96%, 97%, 98%, or 99% identity with the amino acid sequence of SEQ ID NO: 39; or
(ii) comprises or consists of the amino acid sequence of SEQ ID NO: 39.

38. The fusion protein of any one of embodiments 35-37, wherein the anti-C5 antibody or antigen-binding fragment thereof comprises a light chain variable region VL, wherein the light chain variable region

(i) comprises or consists of an amino acid sequence having at least 90%, 91%, 92%, 93%, 94%, 95%, 96%, 97%, 98%, or 99% identity with the amino acid sequence of SEQ ID NO: 45; or
(ii) comprises or consists of the amino acid sequence of SEQ ID NO: 45.

39. The fusion protein of embodiment 35 or 36, wherein the anti-C5 antibody or antigen-binding fragment thereof comprises a heavy chain variable region VH and a light chain variable region VL, wherein

- the heavy chain variable region comprises or consists of the amino acid sequence of SEQ ID NO: 39 or an amino acid sequence having at least 90%, 91%, 92%, 93%, 94%, 95%, 96%, 97%, 98%, or 99% identity therewith; and
- the light chain variable region comprises or consists of the amino acid sequence of SEQ ID NO: 45 or an amino acid sequence having at least 90%, 91%, 92%, 93%, 94%, 95%, 96%, 97%, 98%, or 99% identity therewith.

40. The fusion protein of embodiment 35 or 36, wherein the anti-C5 antibody or antigen-binding fragment thereof comprises a heavy chain variable region VH and a light chain variable region VL, wherein the VH comprises or consists of the amino acid sequence of SEQ ID NO: 39 and the VL comprises or consists of the amino acid sequence of SEQ ID NO: 45.

41. The fusion protein of any one of embodiments 35-40, wherein the anti-C5 antibody or antigen-binding fragment thereof further comprises a heavy chain constant region HC, e.g., the heavy chain constant region (HC) is the heavy chain constant region of IgG1, IgG2, IgG3, or IgG4, preferably the heavy chain constant region of IgG2 or of IgG4, or the heavy chain constant region of an IgG2/IgG4 hybrid form.

42. The fusion protein of embodiment 41, the heavy chain constant region

(i) comprises or consists of an amino acid sequence having at least 90%, 91%, 92%, 93%, 94%, 95%, 96%, 97%, 98%, or 99% identity with SEQ ID NO: 43; or
(ii) comprises or consists of the amino acid sequence of SEQ ID NO: 43; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 43.

43. The fusion protein of embodiment 41 or 42, wherein the heavy chain constant region comprises one or more mutations enhancing FcRn binding, e.g., YTE mutaion (M252Y/S254T/T256E), LA mutation(M428L/N434A), or LS mutation (M428L/N434S)), preferably comprises LA mutation; and/or comprises mutations enhancing stability, e.g., S228P.

44. The fusion protein of any one of embodiments 35-43, wherein the anti-C5 antibody or antigen-binding fragment thereof comprises a light chain constant region, e.g., said light chain constant region is a lambda or a kappa constant region.

45. The antibody or antigen-binding fragment thereof according to embodiment 44, wherein the light chain constant region:

(i) comprises or consists of an amino acid sequence having at least 90%, 91%, 92%, 93%, 94%, 95%, 96%, 97%, 98%, or 99% identity with the amino acid sequence of SEQ ID NO: 49; or
(ii) comprises or consists of the amino acid sequence of SEQ ID NO: 49; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 49.

46. The fusion protein of any one of embodiments 35-45, wherein the anti-C5 antibody or antigen-binding fragment thereof comprises a heavy chain, wherein the heavy chain

(i) comprises or consists of an amino acid sequence having at least 90%, 91%, 92%, 93%, 94%, 95%, 96%, 97%, 98%, or 99% identity with the amino acid sequence of SEQ ID NO: 44; or
(ii) comprises or consists of the amino acid sequence of SEQ ID NO: 44; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 44, preferably the amino acid change(s) occurs outside the CDR region, preferably, the amino acid change(s) occurs outside the heavy chain variable regions.

47. The fusion protein of any one of embodiments 35-46, wherein the anti-C5 antibody or antigen-binding fragment thereof comprises a light chain, wherein the light chain

(i) comprises or consists of an amino acid sequence having at least 90%, 91%, 92%, 93%, 94%, 95%, 96%, 97%, 98%, or 99% identity with the amino acid sequence of SEQ ID NO: 50; or
(ii) comprises or consists of the amino acid sequence of SEQ ID NO: 50; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 50, preferably the amino acid change(s) occurs outside the CDR region, preferably, the amino acid change(s) occurs outside the heavy chain variable regions.

48. The fusion protein of embodiment 46 or 47, wherein the anti-C5 antibody or antigen-binding fragment thereof comprises a heavy chain and a light chain, wherein

- the heavy chain comprises or consists of the amino acid sequence of SEQ ID NO: 44 or an amino acid sequence having at least 85%, 90%, 91%, 92%, 93%, 94%, 95%, 96%, 97%, 98%, or 99% identity therewith; and
- the light chain comprises or consists of the amino acid sequence of SEQ ID NO: 50 or an amino acid sequence having at least 85%, 90%, 91%, 92%, 93%, 94%, 95%, 96%, 97%, 98%, or 99% identity therewith.

49. The fusion protein of embodiment 48, wherein the anti-C5 antibody or antigen binding fragment thereof comprises a heavy chain and a light chain, wherein the heavy chain comprises or consists of the amino acid sequence as set forth in SEQ ID NO: 44 and the light chain comprises or consists of the amino acid sequence as set forth in SEQ ID NO: 50.
50. The fusion protein of embodiment 33, wherein the fusion protein comprises two heavy chains and two light chains.
51. The fusion protein of embodiment 33 or 50, wherein the heavy chain of the fusion protein comprises or consists of the amino acid sequence of SEQ ID NO:61, 63, 65, 67, 69, 71, 73, 75, 77, 79, 81, 83, 85, or 87, or an amino acid sequence having at least 85%, 90%, 91%, 92%, 93%, 94%, 95%, 96%, 97%, 98% or 99% identity thereto; and/or the light chain of the fusion protein comprises or consists of the amino acid sequence of SEQ ID NO:50, or an amino acid sequence having at least 85%, 90%, 91%, 92%, 93%, 94%, 95%, 96%, 97%, 98% or 99% identity thereto.
52. A nucleic acid molecule encoding a hybrid protein mutant of any one of embodiments 1 to 25 or a fusion protein of any one of embodiments 26 to 51.
53. An expression vector comprising the nucleic acid molecule of embodiment 52, preferably the expression vector is pcDNA3.1.
54. A host cell comprising the nucleic acid molecule according to embodiment 52 or the expression vector according to embodiment 53, preferably, the host cell is prokaryotic or eukaryotic, such as a CHO cell, a 293 cell, such as an Expi293 cell.
55. A method for preparing the hybrid protein mutant of any one of embodiments 1-25 or the fusion protein of any one of embodiments 26-51, the method comprising culturing a host cell of embodiment 54 under conditions suitable for expression of the hybrid protein mutant or fusion protein, and optionally further comprising isolating the hybrid protein mutant or fusion protein from the host cell or the host cell culture medium, and/or purifying the hybrid protein mutant or fusion protein.
56. An immunoconjugate comprising the fusion protein of any one of embodiments 26-51, and other agents, such as anti-hemolytic drugs or labels.
57. A pharmaceutical composition or medicament or formulation comprising the hybrid protein mutant of any one of embodiments 1-25 or the fusion protein of any one of embodiments 26-51, and optionally a pharmaceutical excipient.
58. A pharmaceutical combination product comprising

the hybrid protein mutant of any one of embodiments 1-25 or the fusion protein of any one of embodiments 26-51; and
the additional therapeutic agents.

59. A method for preventing or treating a disease or condition associated with the complement system in a subject, comprising administering to the subject an effective amount of the hybrid protein mutant of any one of embodiments 1-25 or the fusion protein of any one of embodiments 26-51; or the immunoconjugate of embodiment 56 or the pharmaceutical composition or formuation of embodiment 57; or the pharmaceutical combination product of embodiment 58.

60. The method of embodiment 59, wherein the disease or condition is caused by abnormal activation of the complement system or dysregulation of the complement system or is a complement C5-associated disease or condition.

61. The method of embodiment 60, wherein the abnormal activation of the complement system or dysregulation of the complement system is due to, for example, microbial infection or an increase in autoimmune antibodies, or due to a reduction, loss, disability, or functional interference or blockage of complement regulatory proteins.

62. The method of embodiment 60, wherein the complement C5-associated disease or condition includes a disease phenotype caused by unregulated C5 function, such as due to dysregulated C5 activation, such as increased C5 activation.

63. The method of embodiment 60, wherein the complement C5-associated disease or condition is a disease or condition in which the subject has (e.g., elevated levels, such as nucleic acid or protein levels) complement C5 protein (e.g., compared to healthy subjects) or the subject has (e.g., elevated levels, such as nucleic acid or protein levels) complement C5 protein in the blood or blood cells (e.g., compared to the blood or blood cells of healthy subjects).

64. The method of embodiment 63, wherein the complement system-associated disease or disorder is selected from a disease requiring hemolysis inhibition, such as a disease requiring inhibition of hemolysis of the classical pathway of the complement immunity and/or the alternative pathway of the complement immunity; or a disease requiring inhibition of C3b deposition activity, such as dense deposition disease DDD.

65. A method for detecting the presence of complement C5 in a biological sample, comprising contacting the biological sample with the fusion protein according to any one of embodiments 26-51, under conditions that allow binding to complement C5, and detecting whether a complex is formed between the antibody or antigen-binding fragment thereof or the fusion protein and complement C5, wherein the formation of a complex indicates the presence of complement C5.

**Description of Figures:**

Figure 1 shows the crystal structures of the CFH CCP1-4 and DAF CCP1-4 and and the structural model of the hybrid protein.

Figure 2 shows the SDS-PAGE electrophoresis pattern of each protein to be tested.

Figure 3 shows the results of SEC-HPLC purity analysis of the hybrid protein.

Figure 4 shows the CP inhibitory activity of each protein to be tested.

Figure 5 shows the AP inhibitory activity of each protein to be tested.

Figure 6 shows that the activity to inhibit the deposition of C3b on the surface of red blood cells of each protein to be tested.

Figure 7 shows the dissociation properties of the anti-C5 antibodies and the complement C5 in the ForteBio assay at different pH.

Figure 8 shows the activity of the anti-C5 antibodies to inhibit the hemolysis of human C5 transgenic mouse serum CP.

Figure 9 shows a schematic diagram of the fusion protein structure of the antibody and the hybrid protein.

Figure 10 shows the prediction of potential amino acid sites that mediate protein aggregation in hybrid proteins by computer-aided design software (DS2020).

Figure 11 shows AP inhibitory activity of fusion proteins comprising different mutant hybrid proteins (the fusion proteins are identified by the mutations contained in the mutant hybrid proteins).

Figure 12 shows CP inhibitory activity of fusion proteins comprising different mutant hybrid proteins (the fusion proteins are identified by the mutations contained in the mutant hybrid proteins).

Figure 13 shows AP and CP inhibitory activity of the fusion proteins comprising hybrid proteins comprising different mutation combinations (the fusion proteins are identified by the mutations contained in the mutant hybrid proteins).

Figure 14 shows a comparison of AP inhibitory activity of the fusion proteins comprising the hybrid protein comprising 59D/82D/110T/56E mutation combination with that of Eculizumab and the fusion protein comprising CFH CCP1-5 (the fusion proteins are identified by the mutations contained in the mutant hybrid protein).

Figure 15 shows a comparison of CP inhibitory activity of the fusion proteins comprising the hybrid protein comprising 59D/82D/110T/56E mutation combination with that of Eculizumab and the fusion protein comprising CFH CCP1-5 (the fusion proteins are identified by the mutations contained in the mutant hybrid protein).

Figure 16 shows a comparison of C3b deposition inhibitory activity of the fusion proteins comprising the hybrid protein comprising 59D/82D/110T/56E mutation combination with that of Eculizumab and the fusion protein comprising CFH CCP1-5 (rabbit erythrocyte method) (the fusion proteins are identified by the mutations contained in the mutant hybrid protein).

Figure 17 shows a comparison of C3b deposition inhibition activity of the fusion proteins comprising the hybrid protein comprising 59D/82D/110T/56E mutation combination with that of Eculizumab and the fusion protein comprising CFH CCP1-5 (Wieslab method) (the fusion proteins are identified by the mutations contained in the mutant hybrid protein).

Figure 18 shows a comparison of LP inhibitory activity of the fusion proteins comprising the hybrid protein comprising 59D/82D/110T/56E mutation combination with that of Eculizumab and the fusion protein comprising CFH CCP1-5 (Wieslab method) (the fusion proteins are identified by the mutations contained in the mutant hybrid protein).

Figure 19 A-H show purity results and major aggregate characteristics of the hybrid protein mutants detected by HPLC-SEC.

Figure 20 shows a comparison of the ratio (%) of major aggregates and the monomer purity (%) of the hybrid protein mutants.

Figure 21 shows a comparison of expression amount (relative expression amount) of the hybrid protein mutants.

Figure 22 shows a comparison of AP and CP inhibitory activity of the hybrid protein mutants.

## Detailed description of the invention:

### I. Definition

It is to be understood that this invention is not limited to the specific methodologies, protocols, and reagents described herein, as these may vary. It should also be understood that the terminology used herein is for describing particular embodiments only, and is not intended to limit the scope of the present invention, which will be limited only by the appended claims. Unless otherwise defined, all technical and scientific terms used herein have the same meaning as commonly understood by one of ordinary skill in the art to which this invention belongs.

### ➢ Decay-accelerating Factor (DAF)

Decay-accelerating Factor (DAF, CD55) is a membrane-associated regulatory protein that protects autologous cells from activation of autologous complement on their surfaces. DAF acts by rapidly dissociating C3 and C5 convertases (central enzymes of the cascade). DAF has the most potent attenuation-accelerating activity of proteins associated with complement regulation and acts on classical pathway enzymes (C4b2a and C4b2a3b) and alternative pathway enzymes (C3bBb and C3bBbC3b). However, DAF has no cofactor function.

Structural analysis of the DAF shows that, starting from its N-terminus, it consists of a unit of 4 to 60 amino acids in length, followed by a fragment (STP) rich in highly O-glycosylated serine (S) and threonine (T), followed by a post-translationally added glycosylphosphatidylinositol (GPI) anchor.

In some embodiments, the DAF is a human DAF having the following accession numbers: Genbank accession numbers M31516, M15799, M64653, 572858, or M643567.

In some embodiments, the DAF is human DAF. In some embodiments, the human DAF is native human DAF. In some embodiments, the amino acid sequence of native human DAF is set forth in SEQ ID NO: 1, with the various modules as set forth in Table 1 below. Four 60 amino acid long repeating units termed complement control protein repeats (CCP) or Short Consensus Repeats (SCR). CCP1 includes amino acids 35-96; CCP2 includes amino acids 96-160; CCP3 includes amino acids 161-222 and CCP4 includes amino acids 223-285. They provide all the regulatory activities of DAF. The highly O-glycosylated region serves as a cushion that positions the CCP at an appropriate distance suspended above the surface membrane. The GPI anchor allows DAF to move freely on the plasma membrane, making it possible to inactivate invertase complexes anywhere they are assembled. In this context, reference to the amino acid positions of the modules of DAF is made to the amino acid position numbering corresponding to that as set forth in SEQ ID NO: 1.

Table 1: Protein modules of DAF divided by UniProt way)

| The amino acid position of SEQ ID NO:1 | Module |
|---|---|
| 1-34 | Lead sequence |
| 35-96 | CCP1 |
| 96-160 | CCP2 |
| 161-222 | CCP3 |
| 223-285 | CCP4 |
| 287-356 | STP |

In some embodiments, the nucleotide sequence of the cDNA encoding DAF is set forth in SEQ ID NO: 2.

➤ **Factor H (CFH or FH)**

"Complement Factor H", "Factor H", "FH", "CFH protein" or "CFH" are used interchangeably and refer to a protein of approximately 150 kDa that is a member of the regulators of the complement activation family and is a complement control protein. CFH is a large soluble glycoprotein that circulates in human plasma and serves to regulate the alternative pathway of the complement system, ensuring that the complement system is directed towards pathogens or other dangerous substances and does not damage host tissues.

Factor H is predominantly monomeric but can be weakly self-associated (KD = 28 $\mu$M) and may be oligomeric in the presence of glycosaminoglycans or high concentrations of metal ions. CFH consists of 20 homologous units called complement control protein repeats (CCPs) (SCR or sushi domains), some of which function for cell attachment, while other repeats function to eliminate C3b from the cell surface. Each of the 20 SCRs is about 60 amino acids in length, arranged head to tail, comprising 4 cysteine residues, forming 2 disulfide bonds for each module. SCR 19 and 20 are involved in C3b binding.

The splice variant FHL-1 consists of the first 7 CCPs followed by the C-terminal sequence Ser-Pro-Leu-Thr. Each CCP comprises approximately 60 residues, including four invariant cysteines, forming $Cys^I$-$Cys^{III}$, $Cys^{II}$-$Cys^{IV}$ disulfides. Adjacent modules are connected by sequences of three to eight residues.

The images from negative staining electron microscopy show that FH molecules adopt a variety of conformations, but are mainly folded in half; analytical ultracentrifugation, small angle X-ray scattering (deposition of the module in PDB, e.g., 3GAV), and chemical cross-linking also indicated that the module chain itself was bent. The following high resolution structure of several FH fragments was determined, alone or in complex with other molecules (PDB identifier is provided in parenthesis below): CCP 1-2 (2RLP), 2-3 (2RLQ), 1-4 (2WII), 5, 6-7 (e.g., 2W80, 2YBY), 7 (2JGW and 2JGX), 6-8 (2UWN and 2V8E), 9 (4K12), 10-11 (4B2R), 11-12 (4B2S), 12-13 (2KMS), 15 (1HFI), 16 (1HCC), 15-16 (1HFH), 18-20 (3SWO), 19-20 (e.g., 2BZM, 2G7I and 4ONT). Each CCP resembles a prolate spheroid having a major axis of about 4 nm and a minor axis of about 2 nm and comprises beta strands in anti-parallel patches that are approximately aligned with the major axis, with the N- and C-termini located at either end of its long axis, facilitating an end-to-end arrangement of tandem CCPs with variable inter-module contact, tilt, and twists.

In some embodiments, the CFH is a human CFH, e.g., a native human CFH. In some embodiments, the amino acid sequence of CFH has the following accession numbers: HGNC: HGNC: 4883, Ensembl: ENSG00000000971, HPRD: 00601, MIM:134370 or Vega: OTTHUMG00000035607.

In some embodiments, the amino acid sequence of CFH is set forth in SEQ ID NO: 3. In some embodiments, the amino acid positions corresponding to each module of the CFH are as set forth in Table 2 below.

Table 2: Protein modules of CFH (divided by UniProt way)

| The amino acid position of SEQ ID NO:3 | Module |
|---|---|
| 1-18 | Lead peptide |
| 19-82 | CCP1 |
| 83-143 | CCP2 |
| 144-207 | CCP3 |
| 208-264 | CCP4 |
| 265-322 | CCP5 |
| 324-386 | CCP6 |
| 387-444 | CCP7 |
| 446-507 | CCP8 |
| 515-566 | CCP9 |
| 567-625 | CCP10 |
| 628-686 | CCP11 |
| 689-746 | CCP12 |
| 751-805 | CCP13 |
| 809-866 | CCP14 |

(continued)

| The amino acid position of SEQ ID NO:3 | Module |
|---|---|
| 868-928 | CCP15 |
| 929-986 | CCP16 |
| 987-1045 | CCP17 |
| 1046-1104 | CCP18 |
| 1107-1165 | CCP19 |
| 1170-1230 | CCP20 |

In some embodiments, the nucleotide sequence of the cDNA encoding CFH is set forth in SEQ ID NO: 4. In some embodiments, the protein sequence encoding the splice variant FHL-1 of CFH is set forth in SEQ ID NO: 5. In some embodiments, the nucleotide sequence of the cDNA encoding the splice variant FHL-1 of CFH is set forth in SEQ ID NO: 6.

In this context, reference to the amino acid positions of the modules of CFH is made to the amino acid position numbering corresponding to that as set forth in SEQ ID NO: 3.

➢ **Other Definitions**

Wherever appropriate, terms used in the singular may also include the plural and vice versa.

The term "about" when used in conjunction with a numerical value means that it encompasses a range of numerical values with a lower limit that is 5% less than the specified numerical value and an upper limit that is 5% greater than the specified numerical value.

As used herein, the term "and/or" means any one of the optional items or two or more or all of the optional items.

As used herein, the term "comprise" or "include" or any part of the speech thereof means including the elements, integers or steps mentioned, but not excluding any other elements, integers or steps. In this context, when the term "comprise" or "include" is used, unless otherwise specified, it also covers the situation consisting of the elements, integers or steps mentioned. For example, when referring to a protein "comprising" a specific sequence, it is also intended to cover the protein consisting of the specific sequence.

When referring to "first" and "second" in this article, it is only to distinguish between two domains or two chains, and does not indicate the position of the two domains in any way.

As used herein, the term "hybrid protein" is used interchangeably with "chimeric polypeptide" and refers to a larger polypeptide formed by the fusion of at least two heterologous polypeptide sequences, optionally through a linker. Hybrid proteins can be produced by recombinant expression. When referring to a hybrid protein, it may also include mutants or variants of the hybrid protein.

The terms "complement C5" or "C5 protein" or "complement C5 protein" or "C5 complement protein" are used interchangeably and also refer to a complement C5 protein in different species. Human complement C5 (Uniprot entry P01031) is a secreted multidomain glycoprotein consisting of an $\alpha$ chain (999 amino acids) and a $\beta$ chain (655 amino acids) connected by a disulfide bridge. The peptide bond between Arg751 and Leu752 of the $\alpha$ chain is cleaved by the C5 convertase, producing a small 74 amino acid long C5a fragment and a large C5b fragment (1580 amino acids). The conversion of C5 to C5b involves a large conformational change and results in subsequent C6 binding. For example, in some embodiments, human C5 has a sequence as set forth in SEQ ID NO:38. The term "antigen" refers to a molecule that triggers an immune response. This immune response may involve antibody production or activation of specific immune cells, or both. The skilled person will understand that any macromolecule, including essentially all proteins or peptides, can be used as an antigen. In addition, the antigen can be derived from recombinant or genomic DNA. As used herein, the term "epitope" refers to the part of the antigen that specifically interacts with the antibody molecule.

As used herein, the term "antigen binding region" refers to the part of the fusion protein that binds to a specific antigen. The antigen binding region can be, for example, an antibody or immunoglobulin itself or an antibody fragment. Such an antigen binding region may or may not have a tertiary structure independent of the rest of the fusion protein, and may or may not bind to its antigen as a separate entity.

When referring to "the antigen binding region is derived from an antibody", it means that the binding domain constituting the antigen binding region is or is derived from the binding domain of the antibody that specifically binds to the antigen, for example, the heavy chain variable region and/or the light chain variable region of the antigen binding region is or is derived from the heavy chain variable region and/or the light chain variable region of the antibody, or 1, 2, 3, 4, 5 or 6 CDRs of the target binding region are CDRs of the antibody.

The term "derived from" means that the fragment in the antigen binding region is substantially the same as the fragment of the antibody from which it is derived, but has a mutation, such as substitution, deletion or addition, at one or more sites. In a specific embodiment, the mutation is not in the CDR of the antibody.

The terms "whole antibody", "full-length antibody", "complete antibody" and "intact antibody" are used interchangeably herein to refer to a naturally occurring glycoprotein comprising at least two heavy chains (H) and two light chains (L) interconnected by disulfide bonds. Each heavy chain consists of a heavy chain variable region (abbreviated herein as VH) and a heavy chain constant region. The heavy chain constant region consists of three domains CH1, CH2 and CH3. Each light chain consists of a light chain variable region (abbreviated herein as VL) and a light chain constant region. The light chain constant region consists of one domain CL. The VH region and the VL region can be further divided into hypervariable regions (complementarity determining regions (CDRs) with more conserved regions (framework regions (FRs)) interposed therebetween. Each VH and VL consists of three CDRs and four FRs, arranged in the following order from the amino terminus to the carboxyl terminus: FR1, CDR1, FR2, CDR2, FR3, CDR3, FR4. The constant region is not directly involved in the binding of the antibody to the antigen, but exhibits a variety of effector functions. In some embodiments, the heavy chain constant region HC of the antibody of the present invention is the heavy chain constant region of IgG1, IgG2, IgG3 or IgG4, preferably the heavy chain constant region of IgG1. The term "antibody fragment" includes a portion of a complete antibody. In a preferred embodiment, the antibody fragment is an antigen binding fragment.

The term "antigen binding fragment" of an antibody is a portion or segment of a full-length or whole antibody that has fewer amino acid residues than a whole antibody or a full-length antibody, but it can bind to an antigen or compete with a full-length antibody (i.e., the full-length antibody from which the antigen binding fragment is derived) for binding to an antigen. Antigen binding fragments can be prepared by recombinant DNA technology, or by enzymatic or chemical cleavage of a complete antibody. Antigen binding fragments include, but are not limited to, Fab, Fab', F(ab')2, Fv, single-chain Fv, diabody Diabody, single domain antibody (sdAb), nanobody. For example, Fab fragments can be obtained by digesting full-length antibodies with papain. In addition, F(ab')2, which is a dimer of Fab' and a divalent antibody fragment, is produced by digesting the complete antibody below the disulfide bond in the hinge region with pepsin. F(ab')2 can be reduced under neutral conditions by destroying the disulfide bond in the hinge region, thereby converting the F(ab')2 dimer into a Fab' monomer. The Fab' monomer is basically a Fab fragment with a hinge region. The Fv fragment consists of the VL and VH domains of a single arm of an antibody. The two domains VL and VH of the Fv fragment can be encoded by independent genes, but recombinant methods can also be used to connect the two domains using a synthetic connecting peptide so that they are produced as a single protein chain, and in the single protein chain, the VL region and the VH region are paired to form a single-chain Fv (scFv).

The term "variable region" or "variable domain" refers to the domain of an antibody heavy or light chain that is involved in binding the antibody to an antigen. The variable regions of the heavy and light chains of natural antibodies generally have similar structures, with each domain containing four conserved framework regions (FRs) and three complementarity determining regions.

The "complementarity determining region" or "CDR region" or "CDR" or "hypervariable region" of an antibody is a region of an antibody variable domain (VH or VHH) that is highly variable in sequence and forms structurally defined loops ("hypervariable loops") and/or contains antigen contact residues ("antigen contact points"). CDRs are primarily responsible for binding to antigen epitopes. The CDRs of the heavy and light chains are numbered sequentially from the N-terminus and are usually referred to as CDR1, CDR2 and CDR3. The CDRs located in the variable domain of the antibody heavy chain are also referred to as HCDR1, HCDR2 and HCDR3, while the CDRs located in the variable domain of the antibody light chain are referred to as LCDR1, LCDR2 and LCDR3. In a given light chain variable region or heavy chain variable region amino acid sequence, its CDR sequence can be determined using a variety of schemes known in the art, such as: Chothia based on the three-dimensional structure of the antibody and the topology of the CDR loop, Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (International Immunogenetics Information System, World Wide Web imgt.cines.fr/), and North CDR definition based on affinity propagation clustering using a large number of crystal structures (North et al., "A New Clustering of Antibody CDR Loop Conformations", Journal of Molecular Biology, 406, 228-256 (2011)).

Unless otherwise specified, in the present invention, the term "CDR" or "CDR sequence" encompasses CDR sequences determined in any of the above ways.

CDRs can also be determined based on the same Kabat numbering position as the reference CDR sequence. Unless otherwise specified, in the present invention, when referring to residue positions in the variable region of an antibody (including heavy chain variable region residues and light chain variable region residues), it refers to the numbering position according to the Kabat numbering system (Kabat et al., Sequences of Proteins of Immunological Interest, 5th Ed. Public Health Service, National Institutes of Health, Bethesda, Md. (1991)).

In one embodiment, the CDRs in the antibody molecule of the present invention are determined by the Chothia numbering convention.

The term "Fc domain" or "Fc region" is used herein to define the C-terminal region of an immunoglobulin heavy chain containing at least a portion of the constant region. The term includes native sequence Fc regions and variant Fc regions. A natural immunoglobulin "Fc domain" or "Fc region" comprises two or three constant domains, i.e., a CH2 domain, a CH3 domain, and an optional CH4 domain. For example, in a natural antibody, an immunoglobulin Fc domain comprises the second and third constant domains (CH2 domain and CH3 domain) of the heavy chain derived from IgG, IgA, and IgD class antibodies; or comprises the second, third, and fourth constant domains (CH2 domain, CH3 domain, and CH4 domain) of two heavy chains derived from IgM and IgE class antibodies. Unless otherwise specified herein, the amino acid residues in the Fc region or heavy chain constant region are numbered according to the EU numbering system (also referred to as the EU index) as described in Kabat et al., Sequences of Proteins of Immunological Interes, 5th edition, Public Health Service, National Institutes of Health, Bethesda, MD, 1991. In this context, the terms "Fc region", "Fc part" and "Fc fragment" do not include the heavy chain variable region VH and light chain variable region VL of the immunoglobulin, as well as the heavy chain constant region CH1 and light chain constant region CL, but may include the hinge region at the N-terminus of the heavy chain constant region in some cases. Examples of "effector functions" of immunoglobulins include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen-presenting cells, downregulation of cell surface receptors (such as B cell receptors) and B cell activation.

The term "chimeric antibody" is an antibody molecule in which (a) the constant region or a portion thereof is altered, replaced or exchanged so that the antigen binding site is linked to a constant region of a different or altered class, with a different effector function and/or of a different species, or to a completely different molecule (e.g., enzyme, toxin, hormone, growth factor, drug, etc.) that imparts new properties to the chimeric antibody; or (b) the variable region or a portion thereof is altered, replaced or exchanged with a variable region having a different or altered antigen specificity. For example, a mouse antibody can be modified by replacing its constant region with a constant region from a human immunoglobulin. As a result of the replacement with a human constant region, the chimeric antibody can retain its specificity in recognizing an antigen while having reduced immunogenicity in humans, as compared to the original mouse antibody.

A "humanized antibody" is an antibody that retains the antigen-specific reactivity of a non-human antibody (e.g., a mouse monoclonal antibody) while being less immunogenic when administered to humans as a therapeutic. This can be achieved, for example, by retaining the non-human antigen binding site and replacing the remainder of the antibody with their human counterparts (i.e., replacing the constant region and the portion of the variable region that does not participate in binding with the corresponding portion of a human antibody).

As used herein, the term "fusion protein" refers to a larger polypeptide formed by the fusion of at least two heterologous polypeptide sequences, optionally via a linker. Fusion proteins can be produced by recombinant expression.

In this context, antibody constant regions or antibody constant domains, including CH1, CL and Fc domains and CH2, CH3 and optional CH4 domains constituting the Fc domain, can be selected according to the intended function of the antibody molecule. For example, the constant region can be an IgA, IgD, IgE, IgG or IgM region, in particular an immunoglobulin constant domain of human IgG, for example, a constant domain of human IgG1, IgG2, IgG3 or IgG4, preferably a constant domain of human IgG2, IgG4 or IgG2/4 hybrid form. The immunoglobulin constant region can have a native sequence or a variant sequence.

The term "heavy chain constant region of hybrid form" refers to a fragment of a heavy chain constant region, such as the constant region domains (e.g., CH1, CH2, CH3 and optionally CH4) constituting the constant region or a portion thereof are derived from different IgG, IgA and IgD class antibodies, respectively. For example, heavy chain constant region of an IgG2/IgG4 hybrid form refers to, for example, the CH1 and part of the CH2 of the constant region are from IgG2, and part of the CH2 and CH3 are from IgG4, such as the heavy chain constant region of the C5 antibody drugs Soliris and Ultomiris.

As used herein, the term "linker" refers to any molecule that enables direct connection of different parts of a bispecific binding molecule. Examples of linkers that establish covalent connections between different molecular parts include peptide linkers and non-protein polymers, including but not limited to polyethylene glycol (PEG), polypropylene glycol, polyoxyalkylene or copolymers of polyethylene glycol and polypropylene glycol. In some embodiments, the linker is a peptide linker (also called a "connecting peptide"), which refers to a short amino acid sequence composed of amino acids, such as glycine (G) and/or serine (S) and/or threonine residues (T) used alone or in combination, or a hinge region from an immunoglobulin, for connecting the amino acid sequence of the first part of the fusion protein molecule to the second part of the molecule. For example, a peptide linker can connect the antibody molecule and the polypeptide of the fusion protein molecule. For example, a peptide linker can also connect a part of an antibody to another part of an antibody, such as connecting a light chain variable region to a heavy chain variable region. Preferably, the peptide linker has such a length that it is enough to connect two entities in a manner that they maintain their conformations relative to each other so as not to hinder the desired activity. Useful linkers also include glycine-alanine polymers, glycine-serine polymers, alanine-serine polymers and other flexible linkers. In one embodiment, the connecting peptide has a length of 1-50 amino acids, for

EP 4 617 287 A1

example, 1,2,3,4,5,6,7,8,9,10 or more amino acid lengths. In one embodiment, the linker is one or more glycines, for example Gn, wherein n=an integer of 1-10, for example 1,2,3,4,5 or 6. In one embodiment, the linker is, for example, a glycine-serine polymer, such as GS, GSG, $G_nS_n$, $(G_nS)_n$, or $(G_4S)n$, where n = an integer from 1 to 10, such as 1, 2, 3, 4, 5, or 6. In one embodiment, the connecting peptide is selected from one or more glycine, or "(GSG)n" or $(G_4S)n$, wherein n is an integer equal to or greater than 1, for example, n is an integer of 2, 3, 4, 5, 6 or 7. Suitable flexible connecting peptides can be rationally designed using computer programs to simulate the three-dimensional structure of proteins and peptides, or by phage display methods.

As used herein, the terms "anti", "binding," or "specific binding" mean that the binding is selective for the antigen and can be distinguished from unwanted or non-specific interactions. The ability of an antigen binding site to bind to a specific antigen can be determined by enzyme-linked immunosorbent assay (ELISA) or conventional binding assays known in the art, such as by radioimmunoassay (RIA) or thin-layer biofilm interferometry or MSD assay or surface plasmon resonance (SPR).

"Affinity" or "binding affinity" refers to the intrinsic binding affinity that reflects the interaction between members of a binding pair. The affinity of a molecule X for its partner Y can be generally represented by a dissociation constant (KD), which is the ratio of the dissociation rate constant and the association rate constant (kdis and kon, respectively). Affinity can be measured by common methods known in the art. One specific method for measuring affinity is the ForteBio kinetic binding assay herein.

In this context, the amino acid mutation may be an amino acid substitution, deletion, insertion, and addition. Any combination of substitution, deletion, insertion and addition may be made to obtain an optimized mutein with the desired properties (e.g. increased solubility). Amino acid deletions and insertions include those at the amino and/or carboxy terminus of the polypeptide sequence, as well as those within the polypeptide sequence. Several amino acids may be added at the C-terminus of the hybrid protein. In some embodiments, preferred amino acid mutations are amino acid substitutions, such as single amino acid substitutions, or a combination of several amino acid substitutions.

In this context, when referring to the amino acid position to be mutated in the optimization of the hybrid protein, it is determined by referring to the amino acid sequence set forth in SEQ ID NO: 20. Corresponding amino acid positions on hybrid proteins or polypeptides having other amino acid sequences can be determined by amino acid sequence alignment with SEQ ID NO: 20. For example, when referring to "L56", it is meant leucine L at position 56 of SEQ ID NO:20, or the amino acid residue at the corresponding position of the amino acid sequence of other hybrid proteins by alignment.

In this context, when referring to a hybrid protein mutant or a mutation thereof, single amino acid substitution is described in the following manner: [original amino acid residue/position/amino acid residue for substitution] or [position/amino acid residue for substitution]. For example, a substitution of the leucine at position 56 (or other corresponding amino acid) to a glutamic acid can be represented as L56E or as 56E. When there are a variety of alternative amino acid substitutions (e.g., D, S) at a given position (e.g., position L59), the amino acid substitution can be represented as: L59/D/S or 59/D/S. Accordingly, individual single amino acid substitutions may be linked by a plus sign "+" or "-" to indicate a combination mutation at a plurality of given positions. For example, the combination mutation of 59S, 82D, 110T can be represented as: 59S +82D +110T or 59S-82D-110T.

As used herein, "hydrophilic amino acids or residues" refers to amino acids or residues having side chains that exhibit hydrophobicity less than zero according to the normalized consensus hydrophobicity scale of Eisenberg et al (Eisenberg et al.,J.Mol.Biol.,179:125-142[1984]). Exemplary hydrophilic amino acids are selected from Thr (T), Ser (S), L-His (H), Glu (E), Asn (N), Gln (Q), Asp (D), Lys (K), and Arg (R).

As used herein, "hydrophobic amino acids or residues" refers to amino acids or residues having side chains that exhibit hydrophobicity greater than zero according to the normalized consensus hydrophobicity scale of Eisenberg et al (Eisenberg et al., J.Mol.Biol.,179:125-142[1984]). Exemplary hydrophobic amino acids are selected from Pro (P), Ile (I), Phe (F), Val (V), Leu (L), Trp (W), Met (M), Ala (A), or Tyr (Y).

"An amino acid which is more hydrophilic than the original amino acid" means that the substituted amino acid is more hydrophilic than the original amino acid, and is not necessarily "a hydrophilic amino acid", and for example, F may be substituted for Y, provided that Y is more hydrophilic than F.

"Percenage (%) of identity" of an amino acid sequence refers to the percentage of amino acid residues in a candidate sequence that are identical to the amino acid residues of the specific amino acid sequence as set forth in this specification, after aligning the candidate sequence with the specific amino acid sequence as set forth in this specification and introducing gaps, if necessary, to achieve the maximum percent sequence identity, and without taking into account any conservative substitutions included as part of sequence identity. In some embodiments, the present invention contemplates variants of a protein or polypeptide of the present invention that have a substantial degree of identity, e.g., at least 80%, 85%, 90%, 95%, 97%, 98%, or 99% or more identity, relative to the polypeptide or protein specifically disclosed herein. The variants may comprise conservative changes.

For polypeptide sequences, "conservative changes" include substitutions, deletions, or additions to the polypeptide sequence that do not substantially change the desired functional activity of the polypeptide sequence. For example, conservative substitutions often result in the replacement of an amino acid with a chemically similar amino acid.

Conservative substitution tables providing functionally similar amino acids are well known in the art. 8 groups of amino acids conservatively substituted for each other are listed as follows: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M) ), Valine (V); 6) Phenylalanine (F), Tyrosine (Y), Tryptophan (W); 7) Serine (S), Threonine (T); and 8) Cysteine (C), Methionine (M). In some embodiments, the term "conservative sequence change" is used to refer to amino acid modifications that do not significantly affect or alter the activity of the parent hybrid protein. For example, conservatively modified variants retain at least 80%, 85%, 90%, 95%, 98%, 99%, or more, e.g., 100-110% or more activity relative to the parent polypeptide or hybrid protein.

The term "host cell" refers to a cell into which an exogenous polynucleotide has been introduced, including the progeny of such cells. Host cells include "transformants" and "transformed cells," which include primary transformed cells and progeny derived therefrom. Host cells are any type of cell system that can be used to produce hybrid proteins of the present invention, including eukaryotic cells, such as mammalian cells, insect cells, yeast cells; and prokaryotic cells, such as E. coli cells. Host cells include cultured cells, as well as cells within transgenic animals, transgenic plants, or cultured plant tissue or animal tissue.

The term "expression vector" refers to a vector comprising a recombinant polynucleotide comprising expression control sequences operably linked to the nucleotide sequence to be expressed. The expression vector comprises sufficient cis-acting elements for expression; other elements for expression can be provided by the host cell or in an in vitro expression system. Expression vectors include all those known in the art, including cosmids, plasmids (e.g., naked or included in liposomes), and viruses (e.g., lentiviruses, retroviruses, adenoviruses, viruses, and adeno-associated viruses) into which recombinant polynucleotides are incorporated.

The terms "individual" or "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, individuals are human.

The term "treating" includes administering a composition or hybrid polypeptide to prevent or delay the onset of symptoms, complications, or biochemical indication of a disease, alleviate symptoms, or prevent or inhibit further progression of the disease, condition, or disorder. The term "prevention" includes the inhibition of the occurrence or progression of a disease or disorder or symptoms of a particular disease or disorder.

The term "pharmaceutical excipient" refers to diluents, adjuvants (such as Freund's adjuvant (complete and incomplete)), excipients, carriers or stabilizers, etc., which are administered with the active substance.

The term "pharmaceutical composition" refers to a composition that is in a form effective to permit the biological activity of the active ingredients comprised therein and does not comprise additional ingredients that would have unacceptable toxicities to the subject to whom the composition is administered.

The term "effective amount" refers to an amount or dosage of a hybrid protein of the present invention or a nucleic acid encoding the same or a composition or combination thereof that, when administered to a patient in single or multiple doses, produces the desired effect in a patient in need of treatment or prevention.

A "therapeutically effective amount" refers to an amount effective to achieve the desired therapeutic result, at the required doses and for the required period of time. A therapeutically effective amount is also an amount in which any toxic or detrimental effects of the hybrid protein or composition or combination are outweighed by the therapeutically beneficial effects. A "therapeutically effective amount" preferably inhibits a measurable parameter or improves a measurable parameter by at least about 40%, even more preferably at least about 50%, 55%, 60%, 65%, 70%, 75%, 80%, 85%, 90% or even 100%, relative to an untreated subject.

A "prophylactically effective amount" refers to an amount effective to achieve the desired prophylactic result, at the required dosage and for the required period of time. Generally, the prophylactically effective amount will be less than the therapeutically effective amount because the prophylactic dose is administered in the subject before or at an earlier stage of the disease.

As used herein, the term "complement C5-associated disease or condition" refers to a disease or condition in which unregulated C5 function may result in a disease phenotype, for example, due to dysregulated C5 activation, such as increased C5 activation.

As used herein, the term "label" refers to a compound or composition that is directly or indirectly conjugated or fused to an agent (such as a polynucleotide probe or antibody) and facilitates the detection of the agent to which it is conjugated or fused. The label itself can be detectable (e.g., a radioisotope label or a fluorescent label) or can catalyze a chemical alteration of a detectable substrate compound or composition in the case of an enzymatic label. The term is intended to cover direct labeling of a probe or antibody by coupling (i.e., physically linking) a detectable substance to the probe or antibody and indirect labeling of a probe or antibody by reaction with another reagent that is directly labeled. In some embodiments, the label is His or biotin.

"Subject/patient/individual sample" refers to a collection of cells or fluids obtained from a patient or subject or individual. The source of tissue or cell sample can be solid tissue, like from fresh, frozen and/or preserved organ or tissue sample or biopsy sample or puncture sample; blood or any blood component; body fluid, such as tears, vitreous humor, cerebrospinal fluid, amniotic fluid (amniotic fluid), peritoneal fluid (ascites), or interstitial fluid; cells from any time of pregnancy or development of the subject. In some embodiments, the sample is blood or serum. In some embodiments, the tissue sample may contain compounds that are not naturally intermixed with the tissue in nature, such as preservative, anticoagulant, buffer, fixing agent, nutrient, antibiotic, and the like.

These and other aspects and embodiments of the present invention are described in the accompanying drawings (Brief Description of the Drawings immediately follows) and the following detailed description of the present invention and are illustrated in the following examples. Any or all features discussed above and throughout this application may be combined in various embodiments of the present invention. The following examples further illustrate the present invention, however, it is to be understood that the examples are described by way of illustration rather than limitation and that various modifications may be made by those skilled in the art.

### III. Hybrid proteins or mutants thereof

The present invention relates to a hybrid protein of CFH and DAF, which comprises at least one functional unit derived from CFH and at least one functional unit derived from DAF.

### A. DAF functional unit

In certain embodiments, the hybrid protein of the present invention preferably comprises a functional unit from DAF. Such functional units are capable of dissociating the C3 and C5 convertases and/or accelerating the decay-accelerating activity against C3 convertases in the classical pathway and/or in the alternative pathway. In some embodiments, the DAF functional unit comprises CCP3 and 4 of the DAF.

The amino acid sequence of such CCPs can be identical to the native or naturally occurring amino acid sequence of DAF. Alternatively, the amino acid sequence of such CCPs may be slightly altered, particularly at the amino or carboxy terminus. This change occurs when the restriction enzyme site is incorporated into the polynucleotide encoding the CCP. Such changes also occur when an amino acid is deleted from the N-terminus or C-terminus of the functional unit. For example, in some embodiments, 1-2 amino acids may be deleted at the N-terminus of CCP3. For another example, in some embodiments, 1-2 amino acids may be deleted at the C-terminus of CCP4.

Some amino acid substitutions, preferably conservative substitutions, in the sequence may also be introduced without affecting functional activity. Conservative substitutions may be made, for example, by substituting charged amino acids for one another or substituting hydrophilic amino acids for one another, hydrophobic amino acids for one another, and amino acids of similar mass for one another.

In one embodiment, the DAF functional unit comprises an amino acid sequence having least 95%, 96%, 97%, 98%, or 99% identity with CCP 3 and/or 4 of the human DAF protein (or CCP3-4 directly linked together by the C-terminus of CCP3 and the N-terminus of CCP4). In some embodiments, the DAF functional unit comprises CCP3 and/or 4 of the human DAF protein (CCP3-4 directly linked together by the C-terminus of CCP3 and the N-terminus of CCP4). In some embodiments, the DAF functional unit comprises CCP3 and 4 of the human DAF protein. In some embodiments, the DAF function unit is CCP3-4, which is directly linked together by the C-terminus of CCP3 and the N-terminus of CCP4.

In some embodiments, the human DAF protein is a native human DAF protein. In some embodiments, the native human DAF protein comprises or consists of the amino acid sequence shown as SEQ ID NO: 1. DAF comprises or consists of the amino acid sequence encoded by the DNA sequence as set forth in SEQ ID NO: 2.

In some embodiments, CCP3 comprises or consists of amino acids 161-222 of DAF. In some embodiments, 1-2 amino acids of the N-terminus of CCP3 may be deleted for attachment of other functional units, e.g., CCP3 comprises or consists of amino acids 163-222 of DAF.

In some embodiments, CCP3 comprises or consists of the amino acid sequence as set forth in SEQ ID NO: 7 or 8, or an amino acid sequence having at least 95%, 96%, 97%, 98%, or 99% identity to the amino acid sequence as set forth in SEQ ID NO: 7 or SEQ ID NO: 8.

SEQ ID NO: 7:

KSCPNPGEIRNGQIDVPGGILFGATISFSCNTGYKLFGSTSSFCLISGSSVQWSDPLPECR
E

SEQ ID NO: 8:
CPNPGEIRNGQIDVPGGILFGATISFSCNTGYKLFGSTSSFCLISGSSVQWSDPLPECRE.

In some embodiments, CCP4 comprises or consists of amino acids 223-285 of DAF.

In some embodiments, CCP4 comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9.

SEQ ID NO: 9:
IYCPAPPQIDNGIIQGERDHYGYRQSVTYACNKGFTMIGEHSIYCTVNNDEGEWSGPPP ECRG.

In some embodiments, CCP3-4 comprises or consists of amino acids 161-285 or amino acids 163-285 of DAF. In some embodiments, the DAF functional unit CCP3-4 comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.

SEQ ID NO: 10: Human DAF CCP3-4

KSCPNPGEIRNGQIDVPGGILFGATISFSCNTGYKLFGSTSSFCLISGSSVQWSDPLPECR EIYCPAPPQIDNGIIQGERDHYGYRQSVTYACNKGFTMIGEHSIYCTVNNDEGEWSGPPPEC RG

SEQ ID NO: 11: Human DAF CCP3-4 (2 amino acids are missing from the N-terminus of CCP3)

CPNPGEIRNGQIDVPGGILFGATISFSCNTGYKLFGSTSSFCLISGSSVQWSDPLPECREIY CPAPPQIDNGIIQGERDHYGYRQSVTYACNKGFTMIGEHSIYCTVNNDEGEWSGPPPECRG

When referring to the amino acid position of DAF, the amino acid sequence position corresponds to the amino acid position of SEQ ID NO: 1.

## B. CFH functional unit

In certain embodiments, the hybrid protein of the present invention comprises functional units from CFH. This functional unit is capable of dissociating the alternative pathway C3 convertase C3bBb and/or binding to C3b. In a preferred embodiment, the CFH functional unit comprises CCP1 of CFH.

The amino acid sequence of the CCP of such CFH may be identical to the native or naturally occurring amino acid sequence of CFH. Alternatively, the amino acid sequence of such CCPs may be slightly altered, particularly at the amino or carboxy terminus. This change occurs when the restriction enzyme site is incorporated into the polynucleotide encoding the CCP. Such changes also occur when amino acids are deleted from or added to the N-terminus or C-terminus of the functional unit.

For example, in some embodiments, 1-2 amino acids may be deleted at the C-terminus of CCP1.

In some embodiments, 1-2 amino acids of CCP2 can also be added to the C-terminus of CCP1 to attach functional units of DAF, thus, "CCP1" as described herein encompasses the CCP1 with amino acids (e.g., 1-2 amino acids of CCP2) added at the C-terminus. For example, in the case where 1-2 amino acids are deleted from the N-terminus of the functional unit of DAF, 1-2 amino acids of CCP2 may be added to the C-terminus of CCP1 for linking the functional unit of DAF. In some embodiments, KS are deleted from the CCP3 of DAF, and RP are added to the C-terminus of CCP1 of CFH, both of which are linked to obtain a hybrid protein.

Some amino acid substitutions, preferably conservative substitutions, in the sequence may also be introduced without affecting functional activity. Conservative substitutions may be made, for example, by substituting charged amino acids for one another or substituting hydrophilic amino acids for one another, hydrophobic amino acids for one another, and amino acids of similar mass for one another. For example, CCP1 of the CFH of the present invention can comprise a V62I mutation.

In one embodiment, the CFH functional unit comprises an amino acid sequence having at least 95%, 96%, 97%, 98%, or 99% identity to CCP1 of the human CFH protein. In some embodiments, the CFH functional unit comprises CCP1 of the human CFH protein.

In some embodiments, the human CFH protein is a native human CFH protein. In some embodiments, the native human CFH protein comprises or consists of an amino acid sequence as set forth in SEQ ID NO: 3 or 5. In some embodiments, the CFH comprises or consists of the amino acid sequence encoded by the DNA sequence as set forth in SEQ ID NO: 4 or 6.

In some embodiments, CCP1 comprises or consists of amino acids 19-82 of CFH. In some embodiments, CCP1 comprises or consists of amino acids 19-to 84 of CFH.

In some embodiments, the CFH functional unit comprises or consists of the amino acid sequence as set forth in SEQ ID NO: 12, 13, 14, or 15, or an amino acid sequence having at least 95%, 96%, 97%, 98%, or 99% identity to the amino acid sequence as set forth in SEQ ID NO: 12, 13, 14, or 15.

SEQ ID NO: 12: Human CFH CCP1 (the C-terminus of CCP1 is added with two N-terminal amino acids RP of CCP2)

EDCNELPPRRNTEILTGSWSDQTYPEGTQAIYKCRPGYRSLGNVIMVCRKGEWVALNP

LRKCQKRP

SEQ ID NO: 13: Human CFH CCP1 (CCP1)

EDCNELPPRRNTEILTGSWSDQTYPEGTQAIYKCRPGYRSLGNVIMVCRKGEWVALNP
LRKCQK

SEQ ID NO: 14: Human CFH CCP1 (the C-terminus of CCP1 is added with two N-terminal amino acids RP of CCP2 and comprises V62I)

EDCNELPPRRNTEILTGSWSDQTYPEGTQAIYKCRPGYRSLGNIIMVCRKGEWVALNPL
RKCQKRP

SEQ ID NO:15: Human CFH CCP1 (CCP1 comprises V62I)

EDCNELPPRRNTEILTGSWSDQTYPEGTQAIYKCRPGYRSLGNIIMVCRKGEWVALNPL
RKCQK

When referring to the amino acid position of CFH, the amino acid sequence position corresponds to the amino acid position of SEQ ID NO: 3.

## C. Other units

Optionally, the hybrid protein or mutant thereof of the present invention may further comprise a tag, preferably of about 2-10 amino acids, added to the amino or carboxy terminus, e.g. the carboxy terminus, of the hybrid protein or mutant thereof. Typically, such additions are made to stabilize the protein or to facilitate secretory expression or purification of the hybrid protein or mutant thereof. Such tags are known in the art. Representative examples of such tags include sequences encoding a series of histidine residues (e.g., 2-10 histidines, e.g., 2, 3, 4, 5, 6, or 7 histidines), an epitope tag FLAG, herpes simplex glycoprotein D, beta-galactosidase, a maltose-binding protein, or glutathione S-transferase.

In some embodiments, the tag is a histidine residue, which may be added at the amino-terminus or the carboxy-terminus, e.g., the carboxy-terminus, of the hybrid protein or mutant thereof.

In some embodiments, the tag is a 6×His tag of GHHHHHH (SEQ ID NO: 16).

Optionally, the hybrid protein of the present invention may also include a signal peptide, such as MGWSCIILFLVATATGVHS (SEQ ID NO: 17).

The present invention also encompasses hybrid proteins or mutants thereof in which one or more amino acids are altered by post-translational processes or synthetic methods. Examples of such modifications include, but are not limited to, glycosylation, iodination, myristoylation, and PEGylation.

## D. Examples of hybrid proteins

In some embodiments, the hybrid protein of the present invention comprises at least one functional unit from CFH and at least one functional unit from DAF.

In some embodiments, the hybrid proteins of the present invention comprise (i) CCP1 of human complement factor H (CFH) and (ii) CCP3 and/or CCP4 of human Decay Accelerating Factor (DAF).

In some embodiments, the hybrid protein of the present invention comprises CCP1 of CFH and CCP3 and CCP4 of DAF. In some embodiments, the hybrid protein of the present invention consists of CCP1 of CFH and CCP3 and CCP4 of DAF. In some embodiments, the hybrid protein of the present invention comprises or consists of CCP1 of CFH and CCP3-4 of DAF.

In some embodiments, 1-2 amino acids (e.g., 1-2 amino acids at the N-terminus of CCP2) are added to the C-terminus of CCP1 of CFH. In some embodiments, 1-2 amino acids are deleted from the N-terminus of CCP3 or CCP3-4 of DAF.

In some embodiments, the hybrid protein of the present invention comprises CCP1 of CFH and CCP3-4 of DAF, wherein 1-2 N-terminal amino acids of CCP2 are added to the C-terminus of CCP1 and 1-2 amino acids are deleted correspondingly from the N-terminus of CCP3 of DAF. In some specific embodiments, the hybrid protein of the present invention comprises CCP1 of CFH and CCP3-4 of DAF, wherein the N-terminal amino acid RP of CCP2 are added to the C-terminus of CCP1 and the amino acid KS are deleted correspondingly from the N-terminus of CCP3 of DAF.

In some embodiments, CCP1 of CFH in a hybrid protein of the invention can comprise a V62I mutation.

In some embodiments, the hybrid protein of the present invention further comprises a signal peptide at the N-terminus, such as the amino acid sequence as set forth in SEQ ID NO: 17, and/or a tag at the C-terminus, such as a histidine tag, such as a 6×His-tag, such as GHHHHHH.

In some embodiments, CCP3 of DAF comprises or consists of amino acids 161-222 of the DAF protein and/or CCP4 comprises or consists of amino acids 223-285 of the DAF protein, wherein the amino acid positions are numbered corresponding to the amino acid positions as set forth in SEQ ID NO: 1. In some embodiments, the CCP3 of DAF is deleted by 2 amino acids from its N-terminus, for example comprising or consisting of amino acids 163-222 of the DAF protein, wherein said amino acid positions are numbered corresponding to the amino acid positions as set forth in SEQ ID NO: 1. In some embodiments, the CCP3-4 of the DAF comprises or consists of amino acids 161-285 of the DAF protein, wherein the amino acid positions are numbered corresponding to the amino acid positions as set forth in SEQ ID NO: 1. In some embodiments, the CCP3-4 of DAF is deleted at its N-terminus by 2 amino acids, for example comprising or consisting of amino acids 163-285 of the DAF protein, wherein said amino acid positions are numbered corresponding to the amino acid positions as set forth in SEQ ID NO: 1.

In some embodiments, CCP1 of CFH comprises or consists of amino acids 19-82 of the CFH protein, wherein the amino acid positions are numbered corresponding to the amino acid positions as set forth in SEQ ID NO: 3. In some embodiments, CCP1 of CFH comprises or consists of amino acids 19-84 of the CFH protein, wherein the amino acid positions are numbered corresponding to the amino acid positions as set forth in SEQ ID NO: 3.

In some embodiments, CCP3-4 of DAF comprises or consists of amino acids 161-285 of the DAF protein, wherein the amino acid positions are numbered corresponding to the amino acid positions as set forth in SEQ ID NO: 1; and CCP1 of CFH comprises or consists of amino acids 19-82 of the CFH protein, wherein the amino acid positions are numbered corresponding to the amino acid positions as set forth in SEQ ID NO: 3.

In some embodiments, CCP3-4 of DAF comprises or consists of amino acids 163-285 of the DAF protein, wherein the amino acid positions are numbered corresponding to the amino acid positions as set forth in SEQ ID NO: 1; and CCP1 of CFH comprises or consists of amino acids 19-84 of the CFH protein, wherein the amino acid positions are numbered corresponding to the amino acid positions as set forth in SEQ ID NO: 3.

In some specific embodiments, the DAF is a human DAF protein, such as a native human DAF protein. In some specific embodiments, the DAF protein comprises or consists of

(i) the amino acid sequence as set forth in SEQ ID NO: 1;
(ii) the amino acid sequence encoded by the nucleotide sequence as set forth in SEQ ID NO: 2;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

In some specific embodiments, CFH is a human CFH protein, such as a native human CFH protein. In some specific embodiments, the CFH protein comprises

(i) the amino acid sequence as set forth in SEQ ID NO: 3 or 5;
(ii) the amino acid sequence encoded by the nucleotide sequence as set forth in SEQ ID NO: 4 or 6;
(iii) the amino acid sequence having at least 95%, 96%, 97%, 98%, or 99% identity to the amino acid sequence as set forth in (i) or (ii); or

consists of the amino acid sequence as set forth in any one of (i) to (iii).

In some embodiments, the hybrid protein of the present invention comprises or consists of the amino acid sequence of any one of SEQ ID NOs: 18-21, or an amino acid sequence having at least 95%, 96%, 97%, 98%, or 99% identity to the amino acid sequence.

> Human CFH CCP1 or SCR1 - Human DAF/CD55 CCP3-4 or SCR3-4 (in bold):

EDCNELPPRRNTEILTGSWSDQTYPEGTQAIYKCRPGYRSLGNVIMVCRKGEWVALNPLRK
CQKRP**CPNPGEIRNGQIDVPGGILFGATISFSCNTGYKLFGSTSSFCLISGSSVQWSDPLP
ECREIYCPAPPQIDNGIIQGERDHYGYRQSVTYACNKGFTMIGEHSIYCTVNNDEGEW
SGPPPECRG**(SEQ ID NO:18)

EDCNELPPRRNTEILTGSWSDQTYPEGTQAIYKCRPGYRSLGNVIMVCRKGEWVALNPLRK
CQKKS**CPNPGEIRNGQIDVPGGILFGATISFSCNTGYKLFGSTSSFCLISGSSVQWSDPLP
ECREIYCPAPPQIDNGIIQGERDHYGYRQSVTYACNKGFTMIGEHSIYCTVNNDEGEW
SGPPPECRG**(SEQ ID NO:19)

> Human CFH CCP1 or SCR1 - Human DAF/CD55 CCP3-4 or SCR3-4 (in bold), comprising a V62I mutation:

EDCNELPPRRNTEILTGSWSDQTYPEGTQAIYKCRPGYRSLGN**I**IMVCRKGEWVALNPLRK
CQKKS**CPNPGEIRNGQIDVPGGILFGATISFSCNTGYKLFGSTSSFCLISGSSVQWSDPLP
ECREIYCPAPPQIDNGIIQGERDHYGYRQSVTYACNKGFTMIGEHSIYCTVNNDEGEW
SGPPPECRG** (SEQ ID NO:20)

EDCNELPPRRNTEILTGSWSDQTYPEGTQAIYKCRPGYRSLGN**I**IMVCRKGEWVALNPLRK
CQKRP**CPNPGEIRNGQIDVPGGILFGATISFSCNTGYKLFGSTSSFCLISGSSVQWSDPLP
ECREIYCPAPPQIDNGIIQGERDHYGYRQSVTYACNKGFTMIGEHSIYCTVNNDEGEW
SGPPPECRG** (SEQ ID NO:21)

In some embodiments, the hybrid protein of the present invention comprises or consists of the amino acid sequence according to any one of SEQ ID NOs: 22-25, or an amino acid sequence having at least 95%, 96%, 97%, 98%, or 99% identity to the amino acid sequence.

> Human CFH CCP1 or SCR1 - Human DAF/CD55 CCP3-4 or SCR3-4 (in bold) - 6×His (italics):

EDCNELPPRRNTEILTGSWSDQTYPEGTQAIYKCRPGYRSLGNVIMVCRKGEWVALNPLRK

CQKRP**CPNPGEIRNGQIDVPGGILFGATISFSCNTGYKLFGSTSSFCLISGSSVQWSDPLP
ECREIYCPAPPQIDNGIIQGERDHYGYRQSVTYACNKGFTMIGEHSIYCTVNNDEGEW
SGPPPECRG***GHHHHHH* (SEQ ID NO:22)

EDCNELPPRRNTEILTGSWSDQTYPEGTQAIYKCRPGYRSLGNVIMVCRKGEWVALNPLRK
CQKKS**CPNPGEIRNGQIDVPGGILFGATISFSCNTGYKLFGSTSSFCLISGSSVQWSDPLP
ECREIYCPAPPQIDNGIIQGERDHYGYRQSVTYACNKGFTMIGEHSIYCTVNNDEGEW
SGPPPECRG***GHHHHHH* (SEQ ID NO:23)

> Human CFH CCP1 or SCR1 - Human DAF/CD55 CCP3-4 or SCR3-4 (in bold) - 6×His (italics):
comprising a V62I mutation:

EDCNELPPRRNTEILTGSWSDQTYPEGTQAIYKCRPGYRSLGNIIMVCRKGEWVALNP
LRKCQKKS**CPNPGEIRNGQIDVPGGILFGATISFSCNTGYKLFGSTSSFCLISGSSVQWS
DPLPECREIYCPAPPQIDNGIIQGERDHYGYRQSVTYACNKGFTMIGEHSIYCTVNNDE
GEWSGPPPECRG***GHHHHHH*(SEQ ID NO:24)

EDCNELPPRRNTEILTGSWSDQTYPEGTQAIYKCRPGYRSLGN**I**IMVCRKGEWVALNPLRK CQKRP**CPNPGEIRNGQIDVPGGILFGATISFSCNTGYKLFGSTSSFCLISGSSVQWSDPLP ECREIYCPAPPQIDNGIIQGERDHYGYRQSVTYACNKGFTMIGEHSIYCTVNNDEGEW SGPPPECRG***GHHHHHH*(SEQ ID NO:25)

In some embodiments, the hybrid protein of the present invention comprises or consists of the amino acid sequence according to any one of SEQ ID NOs: 26-29, or an amino acid sequence having at least 95%, 96%, 97%, 98%, or 99% identity to the amino acid sequence.

> Signal peptide - <u>human CFH CCP1 or SCR1</u> - human DAF/CD55 CCP3-4 or SCR3-4 (in bold) - 6×His (italic):

MGWSCIILFLVATATGVHS<u>EDCNELPPRRNTEILTGSWSDQTYPEGTQAIYKCRPGYRSLGN VIMVCRKGEWVALNPLRKCQKRP</u>**CPNPGEIRNGQIDVPGGILFGATISFSCNTGYKLFGS TSSFCLISGSSVQWSDPLPECREIYCPAPPQIDNGIIQGERDHYGYRQSVTYACNKGFT MIGEHSIYCTVNNDEGEWSGPPPECRG***GHHHHHH* (SEQ ID NO:26)

MGWSCIILFLVATATGVHS<u>EDCNELPPRRNTEILTGSWSDQTYPEGTQAIYKCRPGYRSLGN VIMVCRKGEWVALNPLRKCQKK</u>**SCPNPGEIRNGQIDVPGGILFGATISFSCNTGYKLFGS TSSFCLISGSSVQWSDPLPECREIYCPAPPQIDNGIIQGERDHYGYRQSVTYACNKGFT MIGEHSIYCTVNNDEGEWSGPPPECRG***GHHHHHH* (SEQ ID NO:27)

> Signal peptide - human <u>CFH CCP1 or SCR1</u> - human DAF/CD55 CCP3-4 or SCR3-4 (in bold) - 6×His (italic): comprising a V62I mutation:

MGWSCIILFLVATATGVHS<u>EDCNELPPRRNTEILTGSWSDQTYPEGTQAIYKCRPGYRSLGN**I** IMVCRKGEWVALNPLRKCQKK</u>**SCPNPGEIRNGQIDVPGGILFGATISFSCNTGYKLFGST SSFCLISGSSVQWSDPLPECREIYCPAPPQIDNGIIQGERDHYGYRQSVTYACNKGFTMI GEHSIYCTVNNDEGEWSGPPPECRG***GHHHHHH*(SEQ ID NO:28)

MGWSCIILFLVATATGVHS<u>EDCNELPPRRNTEILTGSWSDQTYPEGTQAIYKCRPGYRSLGN**I** IMVCRKGEWVALNPLRKCQKRP</u>**CPNPGEIRNGQIDVPGGILFGATISFSCNTGYKLFGST**

**SSFCLISGSSVQWSDPLPECREIYCPAPPQIDNGIIQGERDHYGYRQSVTYACNKGFTMI GEHSIYCTVNNDEGEWSGPPPECRG***GHHHHHH*(SEQ ID NO:29)

In some embodiments, the hybrid protein of the present invention comprises or consists of the amino acid sequence according to any one of SEQ ID NOs: 30-33, or an amino acid sequence having at least 95%, 96%, 97%, 98%, or 99% identity to the amino acid sequence.

> Signal peptide - human <u>CFH CCP1 or SCR1</u> - human DAF/CD55 CCP3-4 or SCR3-4 (in bold):

MGWSCIILFLVATATGVHS<u>EDCNELPPRRNTEILTGSWSDQTYPEGTQAIYKCRPGYRSLGN VIMVCRKGEWVALNPLRKCQKRP</u>**CPNPGEIRNGQIDVPGGILFGATISFSCNTGYKLFGS TSSFCLISGSSVQWSDPLPECREIYCPAPPQIDNGIIQGERDHYGYRQSVTYACNKGFT MIGEHSIYCTVNNDEGEWSGPPPECRG**(SEQ ID NO:30)

MGWSCIILFLVATATGVHS<u>EDCNELPPRRNTEILTGSWSDQTYPEGTQAIYKCRPGYRSLGN VIMVCRKGEWVALNPLRKCQKK</u>**SCPNPGEIRNGQIDVPGGILFGATISFSCNTGYKLFGS TSSFCLISGSSVQWSDPLPECREIYCPAPPQIDNGIIQGERDHYGYRQSVTYACNKGFT MIGEHSIYCTVNNDEGEWSGPPPECRG**(SEQ ID NO:31)

> Signal peptide - human <u>CFH CCP1 or SCR1</u> - human DAF/CD55 CCP3-4 or SCR3-4 (in bold): comprising a V62I mutation:

MGWSCIILFLVATATGVHS<u>EDCNELPPRRNTEILTGSWSDQTYPEGTQAIYKCRPGYRSL GNIIMVCRKGEWVALNPLRKCQKK</u>**SCPNPGEIRNGQIDVPGGILFGATISFSCNTGYKLF GSTSSFCLISGSSVQWSDPLPECREIYCPAPPQIDNGIIQGERDHYGYRQSVTYACNKGF TMIGEHSIYCTVNNDEGEWSGPPPECRG**(SEQ ID NO:32)

MGWSCIILFLVATATGVHS<u>EDCNELPPRRNTEILTGSWSDQTYPEGTQAIYKCRPGYRSL GNIIMVCRKGEWVALNPLRKCQKR</u>**PCPNPGEIRNGQIDVPGGILFGATISFSCNTGYKLF GSTSSFCLISGSSVQWSDPLPECREIYCPAPPQIDNGIIQGERDHYGYRQSVTYACNKGF TMIGEHSIYCTVNNDEGEWSGPPPECRG**(SEQ ID NO:33)

**E. Hybrid protein mutants**

The invention also relates to hybrid protein mutants with optimized property obtained by introducing mutation(s) based on the above defined hybrid protein. Thus, the above-defined hybrid protein of the present invention may be used as a parent to obtain a hybrid protein mutant.

The fusion protein comprising the optimized hybrid protein can maintain the activity of the original hybrid protein and has better solubility compared to the hybrid protein without introducing the mutation(s) for optimization.

In some embodiments, the hybrid proteins and mutants thereof of the invention have better complement inhibitory activity, including activity inhibiting the Alternative Pathway (AP), the Classical Pathway (CP), and/or C3b deposition.

In some embodiments, the hybrid proteins or mutants thereof of the invention are effective in blocking hemolytic activity of human complement.

In some embodiments, the invention relates to a mutant of the hybrid protein having improved purity or aggregate characteristics or expression amount compared to the parent hybrid protein. For example, a mutant of the hybrid proteins of the invention has a reduced (major) aggregate proportion or increased purity compared to the parent hybrid protein. As another example, a mutant of the hybrid proteins of the invention has a reduced major aggregate proportion while has an improved expression amount compared to the parent hybrid protein. For another example, a mutant of a hybrid protein of the invention has an increased expression amount compared to the parent hybrid protein. In some embodiments, the invention relates to a mutant of the hybrid protein having a reduced (major) aggregate proportion, an increased expression amount, and a substantially comparable activity (e.g., complement inhibitory activity) compared to the parent hybrid protein.

In some embodiments, the hybrid protein mutants of the present invention are capable of reducing aggregate production, improving aggregate formation or increasing protein purity as compared to the hybrid protein. In some embodiments, the hybrid protein mutants of the invention have comparable or increased complement inhibitory activity compared to the hybrid protein. In some embodiments, the hybrid protein mutants of the present invention have comparable protein expression amount, or even increased protein expression amount, as compared to the hybrid protein.

In some embodiments, any combination of substitutions, deletions, insertions, and additions can be made to obtain a final mutein construct with desired properties (e.g., a reduced percentage of aggregates of the hybrid protein). Amino acid deletions or insertions include those at the amino and/or carboxy terminus of the polypeptide sequence, as well as those within the polypeptide sequence. In some embodiments, preferred amino acid mutations are amino acid substitutions, such as a single amino acid substitution or substitution of an amino acid sequence segment.

In some embodiments, a mutant of the hybrid protein of the invention is effective in inhibiting hemolysis of the human complement classical pathway and/or hemolysis of the human complement alternative pathway. In some embodiments, a mutant of the hybrid protein of the present invention is effective in inhibiting C3b deposition.

In some embodiments, the hybrid protein mutants of the present invention comprise at least one mutation as compared to the parent hybrid protein; optionally said mutation

improves the formation of major aggregates;
increases the monomer purity of the mutant;
increases the protein expression amount; and/or
does not significantly cause a decrease in complement inhibitory activity (e.g., a decrease in AP inhibitory activity).

In some embodiments, the parent hybrid protein of the invention may be the hybrid protein described herein, e.g., the hybrid protein described in Section II-D.

In some embodiments, an optimized hybrid protein, i.e., a hybrid protein mutant, can be obtained by introducing one or more mutations for optimization into the hybrid protein.

As used herein, a " mutation for optimization " refers to a mutation that further optimizes the hybrid protein in terms of properties, such as solubility of a fusion protein comprising the hybrid protein. The amino acid positions indicated when referring to the mutations for optimization herein are the positions numbered corresponding to the amino acid positions as set forth in SEQ ID NO:20, whether or not indicated.

In some embodiments, mutations for optimization refers to mutating an amino acid in the hybrid protein to a more hydrophilic amino acid that is structurally similar, e.g., mutating a hydrophobic amino acid (e.g., one that predicted by the software to mediate the formation of aggregates) to a more hydrophilic amino acid that is structurally similar, e.g., mutating a hydrophobic amino acid to a hydrophilic amino acid. In some embodiments, the mutation for optimization comprises the addition of one or more (e.g., 1-5, e.g., 1, 2, 3, 4, or 5) amino acids, e.g., hydrophilic amino acids, e.g., lysine or serine, at the carboxy terminus.

In one embodiment, the mutation for optimization is selected from, for example, mutations at position 56, 59, 82, 86, 107, 109 and/or 110 numbered corresponding to the amino acid positions as set forth in SEQ ID NO:20, e.g., a substitution, or an addition of one or more(e.g., 1-5, e.g., 1, 2, 3, 4 or 5) amino acids at the C-terminus.

In some embodiments, the optimized hybrid protein comprises

(1) additions of one or more (e.g., 1-5, such as 1, 2, 3, 4, or 5) amino acids (e.g., hydrophilic amino acids) at the C-terminus;
(2) substitutions (e.g., a substitution to an amino acid that is more hydrophilic than the original amino acid, e.g., a more hydrophilic amino acid that is structurally similar; or a substitution to a hydrophilic amino acid, e.g., a hydrophilic amino acid that is structurally similar to the amino acid to be substituted) at one or more amino acid positions selected from: positions 56, 59, 82, 86, 107, 109 or 110 numbered correspond to the amino acid positions as set forth in SEQ ID NO:20.

In some embodiments, the optimized hybrid protein comprises

(1) addition of one or more (e.g., 1-5, such as 1, 2, 3, 4, or 5) amino acids at the C-terminus;
(2) substitutions at positions 59, 82 and 110 numbered corresponding to the amino acid positions as set forth in SEQ ID NO:20; and optionally
(3) substitution(s( at positions 56, 86, 107, and/or 109 numbered corresponding to the amino acid positions as set forth in SEQ ID NO:20.

In some embodiments, the optimized hybrid protein comprises

(1) addition of one or more (e.g., 1-5, such as 1, 2, 3, 4, or 5) amino acids at the C-terminus; and
(2) substitutions at positions 56, 59, 82, and 110 numbered corresponding to the amino acid positions as set forth in SEQ ID NO:20.

In some embodiments, the amino acid added at the C-terminus is KS.

In some embodiments, the substitution may replace the original amino acid with an amino acid that is more hydrophilic than the original amino acid, for example, replacing F with Y.

In some embodiments, the substitution may be a substitution of an amino acid to a hydrophilic amino acid, such as D, E, S or T.

In some embodiments, the substitution at position 56 is 56E; a substitution at position 59 is 59D or 59S, a substitution at position 82 is 82D; a substitution at position 86 is 86Y; a substitution at position 107 is 107E; a substitution at position 109 is 109D or 109E, and/or a substitution at position 110 is 110T.

In some embodiments, the optimized hybrid protein comprises a mutation selected from the group consisting of: 56E, 59D/S, 82D, 86Y, 107E, 109D/E, 110T or the addition of the amino acids KS at the C-terminus, or a combination thereof.

In some embodiments, the optimized hybrid protein comprises a combination of mutations selected from the group consisting of:

(1) the addition of the amino acids KS at the C-terminus; and
(2) a substitution or combination of substitutions having any one of:

56E;
59D/S;
82D;
86Y;
107E;
109D/E;
110T;
59D/82D/110T;
59S/82D/110T;
59D/82D/110T/56E; or
59S/82D/110T/56E.

In some embodiments, the hybrid protein mutant of the invention comprises or consists of the amino acid sequence of any one of SEQ ID NOs: 53-54, 56-58, 60, 62, 64, 66, 68, 70, 72, 74, 76, 78, 80, 82, 84, 86, 88-96, or the amino acid sequence having at least a 95%, 96%, 97%, 98%, or 99% identity to the amino acid sequence.

>F1D34-KS(SEQ ID NO: 60)

EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRGKS

>F1D34-KS-L56E(SEQ ID NO: 62)

EDCNELPPRRNTEILTGSWSDQTYPEGTQAIYKCRPGYRSLGNIIMVCRKGEWVAENPLRKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRGKS

>F1D34-KS-L59D(SEQ ID NO: 64)

EDCNELPPRRNTEILTGSWSDQTYPEGTQAIYKCRPGYRSLGNIIMVCRKGEWVALNPDRKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRGKS

>F1D34-KS-L59S(SEQ ID NO: 66)

EDCNELPPRRNTEILTGSWSDQTYPEGTQAIYKCRPGYRSLGNIIMVCRKGEWVALNPSRKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRGKS

>F1D34-KS-G82D(SEQ ID NO: 68)

EDCNELPPRRNTEILTGSWSDQTYPEGTQAIYKCRPGYRSLGNIIMVCRKGEWVALNPLRKCQKKSCPNP
GEIRNGQIDVPDGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRGKS

> F1D34-KSF86Y (SEQ ID NO:70)

EDCNELPPRRNTEILTGSWSDQTYPEGTQAIYKCRPGYRSLGNIIMVCRKGEWVALNPLRKCQKKSCPNP
GEIRNGQIDVPGGILYGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRGKS

> F1D34-KS-F107E (SEQ ID NO:72)

EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSECLISGSSVQWSDPLPECREIYCPAPPQIDNGIIQGER
DHYGYRQSVTYACNKGFTMIGEHSIYCTVNNDEGEWSGPPPECRGKS

> F1D34-KS-L109D (SEQ ID NO:74)

EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFCDISGSSVQWSDPLPECREIYCPAPPQIDNGIIQGER

DHYGYRQSVTYACNKGFTMIGEHSIYCTVNNDEGEWSGPPPECRGKS

> F1D34-KS-L109E(SEQ ID NO:76)

EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFCEISGSSVQWSDPLPECREIYCPAPPQIDNGIIQGER
DHYGYRQSVTYACNKGFTMIGEHSIYCTVNNDEGEWSGPPPECRGKS

>F1D34-KS-I110T(SEQ ID NO: 78)

EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFCLTSGSSVQWSDPLPECREIYCPAPPQIDNGIIQGER
DHYGYRQSVTYACNKGFTMIGEHSIYCTVNNDEGEWSGPPPECRGKS

> F1D34-KS-59D/82D/110T(SEQ ID NO:80)

EDCNELPPRRNTEILTGSWSDQTYPEGTQAIYKCRPGYRSLGNIIMVCRKGEWVALNPDRKCQKKSCPNP
GEIRNGQIDVPDGILFGATISFSCNTGYKLFGSTSSFCLTSGSSVQWSDPLPECREIYCPAPPQIDNGIIQGER
DHYGYRQSVTYACNKGFTMIGEHSIYCTVNNDEGEWSGPPPECRGKS

> F1D34-KS-59S/82D/110T (SEQ ID NO:82)

EDCNELPPRRNTEILTGSWSDQTYPEGTQAIYKCRPGYRSLGNIIMVCRKGEWVALNPSRKCQKKSCPNP
GEIRNGQIDVPDGILFGATISFSCNTGYKLFGSTSSFCLTSGSSVQWSDPLPECREIYCPAPPQIDNGIIQGER
DHYGYRQSVTYACNKGFTMIGEHSIYCTVNNDEGEWSGPPPECRGKS

> F1D34-KS-59D/82D/110T/56E (SEQ ID NO:84)

EDCNELPPRRNTEILTGSWSDQTYPEGTQAIYKCRPGYRSLGNIIMVCRKGEWVAENPDRKCQKKSCPNP
GEIRNGQIDVPDGILFGATISFSCNTGYKLFGSTSSFCLTSGSSVQWSDPLPECREIYCPAPPQIDNGIIQGER
DHYGYRQSVTYACNKGFTMIGEHSIYCTVNNDEGEWSGPPPECRGKS

> F1D34-KS-59S/82D/110T/56E (SEQ ID NO:86)

EDCNELPPRRNTEILTGSWSDQTYPEGTQAIYKCRPGYRSLGNIIMVCRKGEWVAENPSRKCQKKSCPNP
GEIRNGQIDVPDGILFGATISFSCNTGYKLFGSTSSFCLTSGSSVQWSDPLPECREIYCPAPPQIDNGIIQGER
DHYGYRQSVTYACNKGFTMIGEHSIYCTVNNDEGEWSGPPPECRGKS

In some embodiments, the hybrid protein mutants of the invention

(1) comprising an amino acid sequence having at least 95%, 96%, 97%, 98%, 99% identity with the amino acid sequence as set forth in SEQ ID NO:60, and comprising an addition of KS at the C-terminus;
(2) comprising an amino acid sequence having at least 95%, 96%, 97%, 98%, 99% identity with the amino acid sequence as set forth in SEQ ID NO:62, and comprising an addition of KS at the C-terminus and a mutation of 56E;

(3) comprising an amino acid sequence having at least 95%, 96%, 97%, 98%, 99% identity with the amino acid sequence as set forth in SEQ ID NO:64, and comprising an addition of KS at the C-terminus and a mutation of 59D;

(4) comprising an amino acid sequence having at least 95%, 96%, 97%, 98%, 99% identity with the amino acid sequence as set forth in SEQ ID NO:66, and comprising an addition of KS at the C-terminus and a mutation of 59S;

(5) comprising an amino acid sequence having at least 95%, 96%, 97%, 98%, 99% identity with the amino acid sequence as set forth in SEQ ID NO:68, and comprising an addition of KS at the C-terminus and a mutation of 82D;

(6) comprising an amino acid sequence having at least 95%, 96%, 97%, 98%, 99% identity with the amino acid sequence as set forth in SEQ ID NO:70, and comprising an addition of KS at the C-terminus and a mutation of 86Y;

(7) comprising an amino acid sequence having at least 95%, 96%, 97%, 98%, 99% identity with the amino acid sequence as set forth in SEQ ID NO:72, and comprising an addition of KS at the C-terminus and a mutation of 107E;

(8) comprising an amino acid sequence having at least 95%, 96%, 97%, 98%, 99% identity with the amino acid sequence as set forth in SEQ ID NO:74, and comprising an addition of KS at the C-terminus and a mutation of 109D;

(9) comprising an amino acid sequence having at least 95%, 96%, 97%, 98%, 99% identity with the amino acid sequence as set forth in SEQ ID NO:76, and comprising an addition of KS at the C-terminus and a mutation of 109E;

(10) comprising an amino acid sequence having at least 95%, 96%, 97%, 98%, 99% identity with the amino acid sequence as set forth in SEQ ID NO:78, and comprising an addition of KS at the C-terminus and a mutation of 110T;

(11) comprising an amino acid sequence having at least 95%, 96%, 97%, 98%, 99% identity with the amino acid sequence as set forth in SEQ ID NO:80, and comprising KS at the C-terminus and a mutation of 59D-82D-110T;

(12) comprising an amino acid sequence having at least 95%, 96%, 97%, 98%, 99% identity with the amino acid sequence as set forth in SEQ ID NO:82, and comprising KS at the C-terminus and a mutation of 59S-82D-110T;

(13) comprising an amino acid sequence having at least 95%, 96%, 97%, 98%, 99% identity with the amino acid sequence as set forth in SEQ ID NO:84, and comprising KS at the C-terminus and a mutation of 59D-82D-110T-56E;

(14) comprising an amino acid sequence having at least 95%, 96%, 97%, 98%, 99% identity with the amino acid sequence as set forth in SEQ ID NO:86, and comprising KS at the C-terminus and a mutation of 59S-82D-110T-56E.

In some embodiments, the hybrid protein mutants of the invention comprise or consist of the amino acid sequence set forth in any one of SEQ ID NOs: 53-54, 56-58, 60, 62, 64, 66, 68, 70, 72, 74, 76, 78, 80, 82, 84, 86, 88-96.

In some embodiments, the hybrid protein mutant of the present invention further comprises a signal peptide at the N-terminus, such as the amino acid sequence as set forth in SEQ ID NO: 17, and/or a tag at the C-terminus, such as a histidine tag, such as a 6×His-tag, such as GHHHHHH.

## III. Fusion protein

In some embodiments, the present invention relates to a fusion protein comprising an anti-C5 antibody or an antigen-binding fragment thereof, and a hybrid protein or mutant thereof of the present invention.

In some embodiments, the fusion protein comprises an anti-C5 antibody or an antigen-binding fragment thereof and a hybrid protein or mutant thereof, wherein the one or more hybrid proteins or mutants thereof (at their N-terminus or at their C-terminus) are respectively connected to the N-terminus and/or C-terminus of the heavy chain and/or light chain of the anti-C5 antibody or its antigen-binding fragment, with or without a linker.

In some embodiments, the fusion protein comprises an anti-C5 antibody or an antigen-binding fragment thereof and a hybrid protein or mutant thereof, wherein the one or two hybrid proteins or mutants thereof (e.g., at their N-terminus) are respectively connected to the C-terminus of the heavy chain of the anti-C5 antibody or its antigen-binding fragment.

In some embodiments, the fusion protein comprises a full-length anti-C5 antibody.

In some embodiments, in the fusion protein, the anti-C5 antibody or its antigen-binding fragment is connected to the hybrid protein or mutant thereof with or without a linker. In some embodiments, the linker is selected from one or more glycine (G)n, GS, $G_nS$, $G_nS_n$, $(G_nS)_n$ or $(GSG)_n$ or $(G_4S)_n$, wherein n is an integer equal to or greater than 1, for example, n is an integer of 2, 3, 4, 5, 6 or 7. In some embodiments, the linker is G, GSG or $G_4S$.

In some embodiments, the anti-C5 antibody or its antigen-binding fragment or fusion protein comprises an Fc region. In some embodiments, the fusion protein comprises a dimeric Fc formed by dimerization of the Fc region. In some embodiments, the first and second Fc regions are the same. In other embodiments, the first Fc region and the second Fc region are different, and the two are paired and heterodimerized. In some embodiments, the Fc region of the fusion protein is connected at its C-terminus to the hybrid protein or mutant thereof of the present invention (e.g., its N-terminus), for example, the first Fc region and/or the second Fc region are connected to the hybrid protein or mutant thereof of the present invention via or without a linker.

The Fc region fragment suitable for the anti-C5 antibody or fusion protein can be any antibody Fc region. The Fc region can include a native sequence Fc region and a variant Fc region. The native sequence Fc domain covers various naturally occurring immunoglobulin Fc sequences, such as Fc regions of various Ig subtypes and their allotypes (Gestur Vidarsson et al., IgG subclasses and allotypes: from structure to effector functions, 20 October 2014, doi: 10.3389/fimmu.2014.00520.). For example, the Fc region of the antibody of the present invention can comprise two or three constant domains, namely, a CH2 domain, a CH3 domain, and an optional CH4 domain. **In** some embodiments, the antibody Fc region may also have an IgG hinge region or a portion of an IgG hinge region at the N-terminus, for example, an IgG1 hinge region or a portion of an IgG1 hinge region. Mutations may be contained in the hinge region.

Preferably, the Fc region contained in the antibody of the fusion protein of the present invention comprises: CH2-CH3 from N-terminus to C-terminus, or comprises: hinge region-CH2-CH3 from N-terminus to C-terminus. In some embodiments, the Fc region suitable for the antibody or fusion protein of the present invention is human IgG Fc, for example, human IgG1 Fc, human IgG2 Fc, human IgG3 or human IgG4 Fc, such as human IgG2 or human IgG4 Fc region, or a Fc region of human IgG2/IgG4 hybrid form (e.g., part of CH2 is from IgG2, part of CH2 and CH3 are from IgG4, such as the Fc region of C5 antibody drugs Soliris and Ultomiris).

The Fc region in the fusion protein of the present invention can be mutated to obtain the desired properties. Mutations to the Fc region are known in the art.

In one embodiment, the Fc region comprises a mutation that increases antibody stability, particularly the stability of an antibody of IgG4 form, such as S228P.

In one embodiment, the Fc region is modified in terms of the properties of the effector function of the Fc region (e.g., the complement activation function of the Fc region). In one embodiment, the effector function has been reduced or eliminated relative to the wild-type isotype Fc region. In one embodiment, the effector function is reduced or eliminated by a method selected from the following: using an Fc isotype that naturally has reduced or eliminated effector function, and Fc region modification.

In one preferred embodiment, the Fc region has reduced effector function mediated by the Fc region, such as reduced or eliminated ADCC or ADCP or CDC effector function, for example, comprising mutations that achieve the above functions.

As will be appreciated by those skilled in the art, according to the intended use of the fusion protein molecule of the invention, the fusion protein molecule of the invention may also comprise modifications in the Fc domain that alter the binding affinity to one or more Fc receptors. In one embodiment, the Fc receptor is an Fcγ receptor, in particular a human Fcγ receptor.

In some embodiments, the Fc region comprises mutations that reduce binding to Fcγ receptors. For example, in some embodiments, the Fc region used in the present invention has L234A/L235A mutations (LALA mutations, e.g., in the IgG1 Fc region) that reduce binding to Fcγ receptors.

In one embodiment, the Fc region comprises mutations that extend the half-life of the antibody (e.g., in vivo half-life), for example, the Fc region comprises mutations that increase binding to the FcRn receptor. For example, in some embodiments, the Fc region used in the present invention has a mutation for increasing binding to the FcRn receptor, such as one or more of a YTE mutation (M252Y/S254T/T256E), a LA mutation (M428L/N434A), or a LS mutation (M428L/N434S). In some embodiments, the Fc region used in the present invention has a LA mutation, a YTE mutation, and a LS mutation that reduce binding to the Fcγ receptor.

In one embodiment, the Fc region comprises or consists of an amino acid sequence of SEQ ID NO:59 or an amino acid sequence having at least 90% identity thereto, such as 95%, 96%, 97%, 99% or more identity thereto.

In some embodiments, the Fc region comprising a mutation that increases binding to the FcRn receptor comprises an amino acid sequence having at least 90% identity, such as 95%, 96%, 97%, 99% or more identity with SEQ ID NO:59, and comprises M428L and N434A.

In some embodiments, the Fc region suitable for the present invention comprises an amino acid sequence having at least 90% identity, such as 95%, 96%, 97%, 99% or more identity with SEQ ID NO: 59 and comprises S228P, M428L and N434A mutations.

In the case where the Fc regions are different, mutations that facilitate heterodimerization may be contained in the Fc region comprised by the fusion protein of the present invention. In one embodiment, mutations are introduced in the CH3 regions of the two Fc regions. Methods for promoting heterodimerization of the Fc region are known in the art. For example, the CH3 region of the first Fc region and the CH3 region of the second Fc region are engineered in a complementary manner so that each CH3 region (or a heavy chain comprising it) can no longer homodimerize with itself but is forced to heterodimerize with the other CH3 region which is engineered in a complementary manner (so that the first and second CH3 regions heterodimerize and no homodimers are formed between the two first CH3 regions or the two second CH3 regions). Preferably, based on the Knob-in-Hole technology, corresponding knob mutations and hole mutations are introduced into the first monomer Fc region and the second monomer Fc region, respectively. This technology is described, for example, in Merchant, A. M., et al. (1998). "An efficient route to human bispecific IgG." Nat Biotechnol 16(7): 677-681.

In some embodiments of the present invention, the present invention provides a fusion protein comprising a full-length anti-C5 antibody and a hybrid protein or a mutant thereof, wherein the hybrid protein or the mutant thereof is connected to the anti-C5 antibody (with or without a linker).

In some embodiments of the present invention, the present invention provides a fusion protein comprising a full-length anti-C5 antibody and a hybrid protein or a mutant thereof, wherein the hybrid protein is connected to the anti-C5 antibody heavy chain at its C-terminus or N-terminus (with or without a linker).

In some embodiments of the present invention, the present invention provides a fusion protein comprising a full-length anti-C5 antibody and a hybrid protein or a mutant thereof, wherein

the anti-C5 antibody is connected at the C-terminus of its Fc region to the N-terminus of the hybrid protein or the mutant thereof to form the heavy chain of the fusion protein (with or without a linker);

the light chain of the anti-C5 antibody forms the light chain of the fusion protein;

For example, the linker is selected from one or more glycines $(G)_n$, GS, $G_nS$, $G_nS_n$, $(G_nS)_n$ or $(GSG)_n$ or $(G4S)_n$, wherein n is an integer equal to or greater than 1, for example, n is an integer of 2, 3, 4, 5, 6 or 7, for example, the linker is G, GSG or $G_4S$.

In some embodiments, the fusion protein comprises two heavy chains and two light chains, preferably, the two heavy chains are identical and/or the two light chains are identical.

In some embodiments, the heavy chain of the fusion protein comprises or consists of the amino acid sequence as set forth in SEQ ID NO: 55, 61, 63, 65, 67, 69, 71, 73, 75, 77, 79, 81, 83, 85 or 87, or an amino acid sequence having at least 85%, 90%, 91%, 92%, 93%, 94%, 95%, 96%, 97%, 98% or 99% identity thereto.

In some embodiments, the light chain of the fusion protein comprises or consists of the amino acid sequence as set forth in SEQ ID NO:50, or an amino acid sequence having at least 85%, 90%, 91%, 92%, 93%, 94%, 95%, 96%, 97%, 98% or 99% identity thereto.

In some embodiments, the fusion protein comprises a heavy chain and a light chan, wherein the heavy chain comprises or consists of the amino acid sequence as set forth in SEQ ID NO: 55, 61, 63, 65, 67, 69, 71, 73, 75, 77, 79, 81, 83, 85 or 87, or an amino acid sequence having at least 85%, 90%, 91%, 92%, 93%, 94%, 95%, 96%, 97%, 98% or 99% identity thereto, and the light chain comprises or consists of the amino acid sequence as set forth in SEQ ID NO:50, or an amino acid sequence having at least 85%, 90%, 91%, 92%, 93%, 94%, 95%, 96%, 97%, 98% or 99% identity thereto.

In some embodiments, the fusion protein comprises a heavy chain and a light chain, wherein the heavy chain comprises or consists of the amino acid sequence as set forth in SEQ ID NO: 55, 61, 63, 65, 67, 69, 71, 73, 75, 77, 79, 81, 83, 85 or 87, and the light chain comprises or consists of the amino acid sequence as set forth in SEQ ID NO:50.

In some embodiments, the fusion protein of the present invention also comprises a signal peptide (e.g., at its N-terminus, such as at the N-terminus of the heavy chain), such as the amino acid sequence as set forth in SEQ ID NO: 17.

**IV**. **Anti-CS Antibody**

In some embodiments, the anti-C5 antibody or antigen-binding fragment thereof suitable for the fusion protein of the present invention can be any anti-C5 antibody or antigen-binding fragment thereof.

In some embodiments, the anti-C5 antibody or antigen-binding fragment thereof is an antibody or antigen-binding fragment thereof that specifically binds to C5, such as the anti-C5 antibody or antigen-binding fragment thereof disclosed in CN113754763A, or Eculizumab, Ravulizumab, Pozelimab, Crovalimab, Tesidolumab or antigen-binding fragment thereof. In some embodiments, the anti-C5 antibody or antigen-binding fragment thereof comprises 1, 2, 3, 4, 5, or 6 CDRs of an antibody that specifically binds to C5, such as the anti-C5 antibody disclosed in CN113754763A, Eculizumab, Ravulizumab, Pozelimab, Crovalimab, or Tesidolumab. In some embodiments, the anti-C5 antibody or antigen-binding fragment thereof comprises 1, 2, or 3 heavy chain variable region CDRs, i.e., HCDR1, HCDR2, and HCDR3, of an antibody that specifically binds to C5, such as the anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises 1, 2 or 3 light chain variable region CDRs, i.e., LCDR1, LCDR2 and LCDR3, of an antibody that specifically binds to C5, such as the anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises 3 heavy chain variable region CDRs and 3 light chain variable region CDRs of the antibody that specifically binds to C5, such as the anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises a heavy chain variable region of an antibody that specifically binds to C5, such as the anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises a light chain variable region of a known antibody that specifically binds to C5, such as an anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises a heavy chain variable region and a light chain variable region of an antibody that specifically binds to C5, such as the anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises a heavy chain of an antibody that specifically binds to C5, such as the anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises a light chain of an antibody that specifically binds to C5, such as the anti-C5 antibody disclosed in CN113754763A, Eculizumab, Ravulizumab, Pozelimab, Tesidolumab, or Crovalimab. In some embodiments, the anti-C5 antibody or its antigen-binding fragment comprises a heavy chain and a light chain of an antibody that specifically binds to C5, such as the anti-C5 antibody disclosed in CN113754763A, Eculizumab, Ravulizumab, Pozelimab, Tesidolumab, or Crovalimab.

In some embodiments, the anti-C5 antibody applicable for the fusion protein of the invention is an anti-C5 antibody, as defined below, which, for example, has a longer duration of in vivo efficacy, or dissociates from C5 faster under acidic conditions than that under neutral conditions.

In some embodiments, the anti-C5 antibody applicable for the fusion protein of the invention is able to effectively inhibit the classical pathway of human complement or the alternative pathway of human complement (for example, inhibit hemolysis of the pathway). In some embodiments, the C5 antibody of the present invention can effectively inhibit the classical pathway of human complement and the alternative pathway of human complement (for example, inhibit hemolysis of the pathway). In some embodiments, the C5 antibody of the present invention has the activity of persistently inhibiting the classical pathway of human complement or the alternative pathway of human complement (for example, inhibiting hemolysis of the pathway). In some embodiments, the C5 antibody of the present invention has the activity of persistently inhibiting the classical pathway and the alternative pathway of human complement (e.g., inhibiting hemolysis of the pathway).

In some embodiments, the anti-C5 antibody applicable for the fusion protein of the invention has pH-dependent antigen binding specificity. In some embodiments, the anti-C5 antibody has a weakened ability to bind to an antigen at a pH under acidic conditions (i.e., a faster dissociation rate), for example, the pH is less than about 7, 6.5, or 6, for example, the pH is between about 4-7.0, 4.5-6.5, or 4.5-6.0. In some embodiments, the anti-C5 antibody has a higher dissociation rate under acidic conditions (e.g., the pH is less than about 7, 6.5, or 6, e.g., the pH is between about 4-7.0, 4.5-6.5, 4.5-6.0, 5.0-6.0, 5.5-6.0, e.g., about pH 5.8) than that under neutral conditions (e.g., a pH of about 7 or between about 7-8, e.g., about pH 7.4). In some embodiments, the antibody has a prolonged drug half-life, for example, a prolonged antibody drug half-life in vivo.

In some embodiments, the antibody or antigen binding fragment thereof has increased stability.

In some embodiments, the anti-C5 antibody or antigen-binding fragment thereof comprises three complementary determining regions (HCDRs) from the heavy chain variable region, HCDR1, HCDR2, and HCDR3.

In some embodiments, the anti-C5 antibody or antigen-binding fragment thereof comprises three complementary determining regions (LCDRs) from the light chain variable region, LCDR1, LCDR2, and LCDR3.

In some embodiments, the anti-C5 antibody or antigen-binding fragment thereof comprises three complementary determining regions (HCDRs) from the heavy chain variable region and three complementary determining regions (LCDRs) from the light chain variable region.

In some aspects, the anti-C5 antibody or antigen-binding fragment thereof comprises a heavy chain variable region (VH). In some aspects, the anti-C5 antibody or antigen-binding fragment thereof comprises a light chain variable region (VL). In some aspects, the anti-C5 antibody or antigen-binding fragment thereof comprises a heavy chain variable region (VH) and a light chain variable region (VL). In some embodiments, the heavy chain variable region comprises three complementary determining regions (CDRs) from the heavy chain variable region, HCDR1, HCDR2, and HCDR3. In some embodiments, the light chain variable region comprises three complementary determining regions (CDRs) from the light chain variable region, LCDR1, LCDR2, and LCDR3.

In some embodiments, the anti-C5 antibody or antigen-binding fragment thereof further comprises an antibody heavy chain constant region HC. In some embodiments, the anti-C5 antibody or antigen-binding fragment thereof further comprises an antibody light chain constant region LC. In some embodiments, the anti-C5 antibody or antigen-binding fragment thereof further comprises a heavy chain constant region HC and a light chain constant region LC.

In some embodiments, the heavy chain variable region of the anti-C5 antibody

(i) comprises or consists of an amino acid sequence having at least 90%, 91%, 92%, 93%, 94%, 95%, 96%, 97%, 98%, or 99% identity with the amino acid sequence of SEQ ID NO: 39; or

(ii) comprises or consists of the amino acid sequence of SEQ ID NO: 39; or

(iii) comprises or consists of an amino acid sequence having one or more (preferably no more than 10, more preferably no more than 5, 4, 3, 2, 1) amino acid changes (preferably amino acid substitutions, more preferably amino acid conservative substitutions) compared to the amino acid sequence of SEQ ID NO: 39, and preferably, the amino acid changes do not occur in the CDR region.

In some embodiments, the light chain variable region of the anti-C5 antibody

(i) comprises or consists of an amino acid sequence having at least 90%, 91%, 92%, 93%, 94%, 95%, 96%, 97%, 98%, or 99% identity with the amino acid sequence of SEQ ID NO: 45; or

(ii) comprises or consists of the amino acid sequence of SEQ ID NO: 45; or

(iii) comprises or consists of an amino acid sequence having one or more (preferably no more than 10, more preferably no more than 5, 4, 3, 2, 1) amino acid changes (preferably amino acid substitutions, more preferably amino acid conservative substitutions) compared to the amino acid sequence of SEQ ID NO: 45, and preferably, the amino acid changes do not occur in the CDR region.

In some embodiments, the three complementary determining regions (HCDRs) from the heavy chain variable region of the anti-C5 antibody, HCDR1, HCDR2 and HCDR3 are selected from

(i) the three complementary determining regions HCDR1, HCDR2 and HCDR3 contained in the VH as set forth in SEQ ID NO: 39, or

(ii) a sequence containing at least one and no more than 5, 4, 3, 2 or 1 amino acid changes (preferably amino acid substitutions, preferably conservative substitutions) in the three HCDR regions relative to the sequence of any one of (i),

for example, the CDRs are determined by the Chothia scheme.

In some embodiments, the three complementary determining regions (LCDRs) from the light chain variable region of the anti-C5 antibody, LCDR1, LCDR2 and LCDR3 are selected from

(i) the three complementary determining regions LCDR1, LCDR2 and LCDR3 contained in the VL as set forth in SEQ ID NO: 45, or

(ii) a sequence containing at least one and no more than 5, 4, 3, 2 or 1 amino acid changes (preferably amino acid substitutions, preferably conservative substitutions) in the three LCDR regions relative to the sequence of any one of (i),

for example, the CDRs are determined by the Chothia scheme.

In some embodiments, the HCDR1 comprises or consists of the amino acid sequence of SEQ ID NO: 40, or the HCDR1 comprises an amino acid sequence having one, two or three changes (preferably amino acid substitutions, preferably conservative substitutions) compared with the amino acid sequence of SEQ ID NO: 40.

In some embodiments, the HCDR2 comprises or consists of the amino acid sequence of SEQ ID NO: 41, or the HCDR2 comprises an amino acid sequence having one, two or three changes (preferably amino acid substitutions, preferably conservative substitutions) compared to the amino acid sequence of SEQ ID NO: 41.

In some embodiments, the HCDR3 comprises or consists of the amino acid sequence of SEQ ID NO: 42, or the HCDR3 comprises an amino acid sequence having one, two or three changes (preferably amino acid substitutions, preferably conservative substitutions) compared to the amino acid sequence of SEQ ID NO: 42.

In some embodiments, the LCDR1 comprises or consists of the amino acid sequence of SEQ ID NO: 46, or the LCDR1 comprises an amino acid sequence having one, two or three changes (preferably amino acid substitutions, preferably conservative substitutions) compared to the amino acid sequence of SEQ ID NO: 46.

In some embodiments, the LCDR2 comprises or consists of the amino acid sequence of SEQ ID NO: 47, or the LCDR2 comprises an amino acid sequence having one, two or three changes (preferably amino acid substitutions, preferably conservative substitutions) compared to the amino acid sequence of SEQ ID NO: 47.

In some embodiments, the LCDR3 comprises or consists of the amino acid sequence of SEQ ID NO: 48, or the LCDR3 comprises an amino acid sequence having one, two or three changes (preferably amino acid substitutions, preferably conservative substitutions) compared to the amino acid sequence of SEQ ID NO: 48.

In some specific embodiments of the present invention, the anti-C5 antibody or antigen-binding fragment thereof comprises VH and VL, wherein the VH comprises or consists of the amino acid sequence as set forth in SEQ ID NO:39 or an amino acid sequence having at least 90%, 91%, 92%, 93%, 94%, 95%, 96%, 97%, 98% or 99% identity thereto, and the VL comprises or consists of the amino acid sequence as set forth in SEQ ID NO:45 or an amino acid sequence having at least 90%, 91%, 92%, 93%, 94%, 95%, 96%, 97%, 98% or 99% identity thereto or consists of the amino acid sequence.

In some specific embodiments of the present invention, the anti-C5 antibody or antigen-binding fragment thereof comprises the HCDR1 comprising or consisting of the amino acid sequence as set forth in SEQ ID NO:40, the HCDR2 comprising or consisting of the amino acid sequence as set forth in SEQ ID NO:41, the HCDR3 comprising or consisting of the amino acid sequence as set forth in SEQ ID NO:42; the LCDR1 comprising or consisting of the amino acid sequence as set forth in SEQ ID NO:46, the LCDR2 comprising or consisting of the amino acid sequence as set forth in SEQ ID NO:47, and the LCDR3 comprising or consisting of the amino acid sequence as set forth in SEQ ID NO:48.

In some specific embodiments of the present invention, the anti-C5 antibody or antigen-binding fragment thereof comprises the VH and the VL, wherein VH comprises or consists of the amino acid sequence as set forth in SEQ ID NO:39, and VL comprises or consists of the amino acid sequence as set forth in SEQ ID NO:45.

In some embodiments of the present invention, the anti-C5 antibody or antigen-binding fragment thereof comprises a heavy chain constant region. In some embodiments, the heavy chain constant region is from IgG1, IgG2, IgG3 or IgG4, preferably the heavy chain constant region of IgG2 or the heavy chain constant region of IgG4, or a heavy chain constant region of an IgG2/IgG4 hybrid form (for example, CH1 region, hinge region and part of CH2 region are from IgG2, and part of CH2 region and CH3 region are from IgG4, such as the heavy chain constant region of C5 antibody drugs Soliris and Ultomiris). In some embodiments, the heavy chain constant region

(i) comprises or consists of an amino acid sequence having at least 90%, 91%, 92%, 93%, 94%, 95%, 96%, 97%, 98%, or 99% identity with SEQ ID NO: 43; or

(ii) comprises or consists of the amino acid sequence of SEQ ID NO: 43; or

(iii) comprises or consists of an amino acid sequence having one or more (preferably no more than 10, more preferably no more than 5, 4, 3, 2, or 1) amino acid changes (preferably substitutions, more preferably conservative substitutions) compared to SEQ ID NO: 43.

In some embodiments of the present invention, the anti-C5 antibody or antigen-binding fragment thereof comprises a light chain constant region. In some embodiments, the light chain constant region of the anti-C5 antibody is a lambda or kappa light chain constant region, such as a Kappa light chain constant region. In some embodiments, the light chain constant region

(i) comprises or consists of an amino acid sequence having at least 90%, 91%, 92%, 93%, 94%, 95%, 96%, 97%, 98%, or 99% identity with the amino acid sequence of SEQ ID NO: 49; or

(ii) comprises or consists of the amino acid sequence of SEQ ID NO: 49; or

(iii) comprises or consists of an amino acid sequence having one or more (preferably no more than 10, more preferably no more than 5, 4, 3, 2, or 1) amino acid changes (preferably substitutions, more preferably conservative substitutions) compared to SEQ ID NO: 49.

In some embodiments of the present invention, the anti-C5 antibody or antigen-binding fragment thereof comprises a heavy chain. In some embodiments of the present invention, the anti-C5 antibody or antigen-binding fragment thereof comprises a light chain. In some embodiments of the present invention, the anti-C5 antibody or antigen-binding fragment thereof comprises a heavy chain and a light chain.

In some specific embodiments, the heavy chain of the anti-C5 antibody

(i) comprises or consists of an amino acid sequence having at least 90%, 91%, 92%, 93%, 94%, 95%, 96%, 97%, 98%, or 99% identity with the amino acid sequence of SEQ ID NO: 44; or

(ii) comprises or consists of the amino acid sequence of SEQ ID NO: 44; or

(iii) comprises or consists of an amino acid sequence having one or more (preferably no more than 10, more preferably no more than 5, 4, 3, 2, or 1) amino acid changes (preferably substitutions, more preferably conservative substitutions) compared to SEQ ID NO: 44, preferably the amino acid change(s) occur outside the CDR region, preferably, the amino acid change(s) occurs outside the heavy chain variable regions.

In some specific embodiments, the light chain of the anti-C5 antibody

(i) comprises or consists of an amino acid sequence having at least 90%, 91%, 92%, 93%, 94%, 95%, 96%, 97%, 98%, or 99% identity with the amino acid sequence of SEQ ID NO: 50; or

(ii) comprises or consists of the amino acid sequence of SEQ ID NO: 50; or

(iii) comprises or consists of an amino acid sequence having one or more (preferably no more than 10, more preferably no more than 5, 4, 3, 2, or 1) amino acid changes (preferably substitutions, more preferably conservative substitutions) compared to SEQ ID NO: 50, preferably the amino acid change(s) occurs outside the CDR region, preferably, the amino acid change(s) occurs outside the heavy chain variable regions.

In some specific embodiments, the anti-C5 antibody comprises a heavy chain and a light chain, wherein the heavy chain comprises or consists of the amino acid sequence as set forth in SEQ ID NO: 44, or an amino acid sequence having at least 85%, 90%, 91%, 92%, 93%, 94%, 95%, 96%, 97%, 98% or 99% identity thereto, and the light chain comprises or consists of the amino acid sequence as set forth in SEQ ID NO: 50, or an amino acid sequence having at least 85%, 90%, 91%, 92%, 93%, 94%, 95%, 96%, 97%, 98% or 99% identity thereto.

In some specific embodiments, the anti-C5 antibody comprises a heavy chain and a light chain, wherein the heavy chain comprises or consists of the amino acid sequence as set forth in SEQ ID NO: 44, and the light chain comprises or consists of the amino acid sequence as set forth in SEQ ID NO: 50.

In one embodiment of the present invention, the amino acid changes of the anti-C5 antibody described herein include substitutions, insertions or deletions of amino acids. In a preferred embodiment, the amino acid changes described in the present invention occur in regions outside the CDR (e.g., in the FR). More preferably, the amino acid changes described in the present invention occur in regions outside the heavy chain variable region and/or outside the light chain variable region. Preferably, the amino acid changes described herein are amino acid substitutions, preferably conservative substitutions.

In some embodiments, the anti-C5 antibodies or antigen-binding fragments thereof applicable for the fusion protein of the present invention have one or more of the following properties:

(i) showing the same or similar binding affinity and/or specificity to C5 as the antibodies of the present invention;

(ii) inhibiting (e.g., competitively inhibiting) the binding of the antibodies of the present invention to C5;

(iii) binding to the same or overlapping epitopes as the antibodies of the present invention;

(iv) competing with the antibodies of the present invention for binding to C5;

(v) having one or more biological properties of the antibodies of the present invention.

In some embodiments, the anti-C5 antibodies applicable for the fusion protein of the present invention are antibodies in the form of IgG1 or antibodies in the form of IgG2 or antibodies in the form of IgG3 or antibodies in the form of IgG4, for example, antibodies in the form of IgG1. In some embodiments, the light chain constant region of the anti-C5 antibodies applicable for present invention is a lambda or kappa light chain constant region, such as a Kappa light chain constant region.

In some embodiments, the anti-C5 antibody is a monoclonal antibody.

In some embodiments, the anti-C5 antibody is humanized.

In some embodiments, the anti-C5 antibody is a chimeric antibody.

In one embodiment, the anti-C5 antibody of the present invention also encompasses antibody fragments thereof (e.g., antigen-binding fragments), preferably antibody fragments selected from the following: Fab, Fab', Fab'-SH, Fv, single-chain antibodies (e.g., scFv), (Fab')$_2$, single-domain antibodies such as VHH, dAb (domain antibody) or linear antibodies.

In one aspect, the present invention also provides an immunoconjugate comprising a fusion protein molecule of the present invention, wherein the molecule of the present invention is conjugated to another agent (e.g., an anti-hemolytic drug) or a label to form an immunoconjugate.

**V. Polynucleotides, Vectors, and Host Cells**

The present invention provides a nucleic acid encoding any of the above hybrid protein mutants of the invention or fusion proteins of the invention. A vector comprising the nucleic acid is also provided. In one embodiment, the vector is an expression vector (e.g., a pCDNA vector, e.g., pCDNA3.1). A host cell comprising the nucleic acid or the vector is also provided. In one embodiment, the host cell is eukaryotic. In another embodiment, the host cell is selected from yeast cells, mammalian cells (e.g., CHO cells or 293 cells, e.g., 293FT or Expi293 cells). In another embodiment, the host cell is prokaryotic.

In one aspect, the present invention provides a nucleic acid encoding any of the above hybrid proteins or mutants thereof. The polypeptide encoded by the nucleic acid is capable of having DAF and/or CFH function when expressed from a suitable expression vector, e.g., dissociating C3 and/or C5 convertase, accelerating decay-accelerating activity against the classical pathway C3 convertase and/or the alternative pathway C3 convertase, dissociating the alternative pathway C3 convertase C3bBb, or binding to C3b, and having complement-inhibiting activity, including activity inhibiting the Alternative Pathway (AP), the Classical Pathway (CP), and/or C3b deposition.

The present invention also provides a nucleic acid encoding any of the above fusion proteins.

For ease of production and purification, the hybrid protein mutants or fusion proteins of the present invention may be fused at the N-terminus to a secretory signal peptide, and/or a tag peptide, such as a hexahistidine tag or biotin marker, to facilitate the purification.

As will be apparent to those skilled in the art, due to codon degeneracy, the polypeptide amino acid sequence of each strand of each fusion protein can be encoded by multiple nucleic acid sequences.

In some embodiments, the nucleic acid of the present invention comprises a nucleic acid encoding an amino acid sequence as set forth in anyone selected from SEQ ID NOs: 39, 44, 45, 50, 53-58, 60-96, or a nucleic acid encoding an amino acid sequence having at least 85%, 90%, 91%, 92%, 93%, 94%, 95%, 96%, 97%, 98% or 99% identity with an amino acid sequence as set forth in anyone selected from SEQ ID NOs: 39, 44, 45, 50, 53-58, 60-96.

The nucleic acid sequence encoding the hybrid protein mutants of the present invention or the fusion protein molecules of the present invention can be produced by methods well known in the art, such as by de novo solid phase DNA synthesis, or by PCR amplification.

In one embodiment, one or more vectors comprising the nucleic acid of the present invention are provided. In one embodiment, the vector is an expression vector, such as a prokaryotic expression vector or a eukaryotic expression vector. The vector includes, but is not limited to, a virus, a plasmid, a cosmid, a phage lambda or a yeast artificial chromosome (YAC). In a preferred embodiment, the expression vector is pCDNA, such as pCDNA3.1.

In one embodiment, a host cell comprising one or more polynucleotides of the present invention is provided. In some embodiments, a host cell comprising an expression vector of the present invention is provided. As used herein, the term "host cell" refers to any type of cell system that can be engineered to produce an antibody molecule of the present invention. Host cells suitable for replication and support for expression of antibody molecules of the present invention are well known in the art. As required, such cells can be transfected or transduced with specific expression vectors, and a large number of cells containing vectors can be cultivated for inoculating large-scale fermenters to obtain sufficient amounts of the present molecule for clinical use.

Suitable host cells include prokaryotic microorganisms such as E.coli, eukaryotic microorganisms such as filamentous fungi or yeast, or various eukaryotic cells such as Chinese hamster ovary Cells (CHO), insect cells, and the like. Mammalian cell lines suitable for suspension culture may be used. Examples of useful mammalian host cell lines include SV40-transformed monkey kidney CV1 line (COS-7); human embryonic kidney line (HEK 293 or 293F cells or 293FT cells or Expi293 cells), baby hamster kidney cells (BHK), monkey kidney cells (CV1), African green monkey kidney cells (VERO-76), human cervical cancer cells (HELA), canine kidney cells (MDCK), Buffalo rat liver cells (BRL 3A), human lung cells (W138), human liver cells (Hep G2), CHO cells, NSO cells, myeloma cell lines such as YO, NS0, P3X63 and Sp2/0, etc. Mammalian host cell lines suitable for producing antibodies are known in the art. In a preferred embodiment, the host cell is a CHO or HEK293 cell or a 293FT cell or an Expi293 cell.

**V. Production and Purification of the Molecules of the Invention**

In yet another aspect, the present invention provides a method for producing a hybrid protein mutant of the present invention, the method comprising: culturing a host cell comprising a nucleic acid molecule encoding the hybrid protein mutant under conditions suitable for expression of the hybrid protein mutant, optionally further comprising isolating the hybrid protein mutant from the host cell or the host cell culture medium, and/or purifying the hybrid protein mutant.

In another aspect, the present invention provides a method for producing the fusion protein of the present invention, the method comprising: culturing a host cell containing a polynucleotide encoding the polypeptide chain under conditions suitable for expressing the polypeptide chain of the fusion protein molecule; optionally further comprising assembling the polypeptide chain to produce the molecule under conditions suitable for assembling the polypeptide chain into the fusion protein molecule.

For recombinant production, the polynucleotide encoding the polypeptide chain of the hybrid protein mutants of the invention or the fusion protein molecules of the invention can be inserted into one or more vectors for further cloning and/or expression in a host cell. Methods well known to those skilled in the art can be used to construct expression vectors. The expression vector includes, but is not limited to, a virus, a plasmid, a cosmid, a phage lambda or a yeast artificial chromosome (YAC). Once an expression vector containing one or more polynucleotides of the present invention for expression has been prepared, the expression vector can be transfected or introduced into a suitable host cell. A variety of techniques can be used to achieve this purpose, for example, protoplast fusion, calcium phosphate precipitation, electroporation, retroviral transduction, viral transfection, gene guns, liposome-based transfection or other conventional techniques.

The molecules prepared as described herein can be purified by known prior art techniques such as high performance liquid chromatography, ion exchange chromatography, gel electrophoresis, affinity chromatography (e.g., Protein A affinity chromatography), size exclusion chromatography, etc. The actual conditions used to purify a particular protein also depend on factors such as net charge, hydrophobicity, hydrophilicity, etc.

The purity of the molecules of the present invention can be determined by any of a variety of well-known analytical methods, including size exclusion chromatography, gel electrophoresis, high performance liquid chromatography, etc. The physical/chemical properties and/or biological activities of the antibody molecules provided herein can be identified, screened or characterized by a variety of assays known in the art.

## VI. Assay method

The hybrid protein mutants or fusion proteins provided herein can be identified, screened, or characterized for their physical/chemical properties and/or biological activity by a variety of assays known in the art.

The hemolytic inhibitory effect of the hybrid protein or fusion protein of the present invention can be measured by methods known in the art, such as in vitro assays and/or in vivo animal experiments. For example, a hemolytic assay, such as the method described in Examples 4.1 and 4.2 and Example 10, can be used to test for hemolytic inhibitory effects of the molecule on the classical and/or alternative and/or the lectin pathways of complement immunity.

The inhibitory activity of the hybrid protein of the present invention on the complement C3b deposition can be measured by methods known in the art, such as in vitro assays and/or in vivo animal experiments. For example, a hemolysis assay, such as the method described in Example 4.3, can be used to test the inhibitory effect of the molecule on the C3b deposition on the surface of red blood cells.

The inhibitory activity of the fusion protein of the present invention on the complement C3b deposition can be measured by methods known in the art, such as in vitro assays and/or in vivo animal experiments. For example, a hemolysis assay or a Wieslab, such as the method described in Example 9, can be used to test the inhibitory effect of the molecule on the C3b deposition on the surface of red blood cells.

The binding dissociation properties of the fusion protein molecules of the present invention with human complement C5 protein can be determined by methods known in the art, such as ELISA, Western blot, ForteBio, etc.

## VII. Pharmaceutical Composition, Pharmaceutical Combination, and Kit

In one aspect, the present invention provides a composition, e.g., a pharmaceutical composition, a medicine or a formulation, comprising a molecule of the present invention (e.g., a hybrid protein mutant or a fusion protein, or an immunoconjugate, etc.).

In one embodiment, the composition further comprises a pharmaceutical excipient, such as a pharmaceutical carrier, a pharmaceutical excipient, including a buffer, as known in the art.

As used herein, "pharmaceutical carrier" includes any and all solvents, dispersion media, isotonic agents, and absorption delaying agents that are physiologically compatible. For the use of pharmaceutical excipients and their uses, see also "Handbook of Pharmaceutical Excipients", 8th edition, R.C. Rowe, P.J. Seskey and S.C. Owen, Pharmaceutical Press, London, Chicago.

The composition or drug or formulation of the present invention may be in a variety of forms. These forms include, for example, liquid, semi-solid and solid dosage forms, such as liquid solutions (e.g., injections), powders or suspensions, liposomes and suppositories. The preferred form depends on the intended mode of administration and therapeutic use.

The composition or drug or formulation of the present invention may also contain other therapeutic agents in addition to one or more molecules of the present invention, which are required for the specific indication being treated and preferably do not adversely affect the activity of each other. Therefore, in one embodiment, the composition or formulation or drug, for example, a pharmaceutical composition, contains a combination of one or more molecules of the present invention, and one or more other therapeutic agents.

The composition or drug or formulation of the present invention may be in a variety of forms. These forms include, for example, liquid, semi-solid and solid dosage forms, such as liquid solutions (e.g., injections), powders or suspensions, liposomes and suppositories. The composition or drug or formulation of the present invention is suitable for intravenous, intramuscular, subcutaneous, parenteral, rectal, spinal or epidermal administration (e.g., by injection or infusion). The preferred form depends on the intended mode of administration and therapeutic use.

The present invention also provides a pharmaceutical combination or a pharmaceutical combination product, which comprises a molecule of the present invention (the fusion protein or the immunoconjugate thereof). Optionally, the pharmaceutical combination or pharmaceutical combination product also comprises one or more other therapeutic agents (e.g., anti-hemolytic agents).

The term "pharmaceutical combination" or "combination product" refers to a non-fixed combination product or a fixed combination product, including but not limited to a kit. The term "non-fixed combination" means that the active ingredients are administered to a patient as separate entities simultaneously, without specific time limits or at equal or different time intervals, sequentially, wherein such administration provides prophylactically or therapeutically effective levels of two or more active agents in the patient. In some embodiments, the hybrid protein of the present invention and the other therapeutic agent used in the pharmaceutical combination are administered at levels no greater than that when they are administered alone. The term "fixed combination" means that two or more active agents are administered to a patient simultaneously in the form of a single entity. The dosage amounts and/or time intervals of the two or more active agents are preferably selected such that the combined use of the parts results in a greater effect in the treatment of the disease or condition than that would be achieved by use of either component alone. The ingredients may each be in separate formulations, which may be the same or different.

The present invention also provides a kit containing the pharmaceutical combination, for example, the kit contains in the same package:

- a first container containing the pharmaceutical composition containing the molecule of the present invention (the fusion protein or the immunoconjugate thereof);
- optionally, a second container containing a pharmaceutical composition containing one or more other therapeutic agents (e.g., an anti-hemolytic agent) (in some embodiments, two or more other therapeutic agents are in the same container, or in different containers, respectively).

## VIII. Uses and Methods

In one aspect, the present invention provides a method for preventing or treating a complement system-related disease or condition in a subject, comprising administering to the subject an effective amount of a molecule of the present invention (e.g., the hybrid protein mutant or the fusion protein or the immunoconjugate thereof, etc.), or a composition or a medicine or a formulation comprising the same.

In some embodiments, the complement system-related disease is caused by abnormal activation of the complement system or dysregulation of the complement system. In some embodiments, the abnormal activation of the complement system or dysregulation of the complement system is due to, for example, an increase in microbial infection or autoimmune antibodies, or due to a reduction, deletion, disability, or functional interference or blockage of complement regulatory proteins. In some embodiments, the treatment of the disease will benefit from inhibiting the activity of the complement system.

In some embodiments, the complement system-related disease or condition is a complement C5-related disease or condition. In some embodiments, the subject has complement C5 protein (e.g., of nucleic acid or protein level, e.g., of an elevated level) (e.g., as compared to a sample from a healthy subject). In some embodiments, the subject has complement C5 protein (e.g., of nucleic acid or protein level, e.g., of an elevated level) in the blood or blood cells (e.g., compared to the blood or blood cells of a healthy subject). In some embodiments, the disease treatment will benefit from the inhibition of complement C5 protein at the nucleic acid or protein level.

In some embodiments, the complement C5-related disease or disorder may be a disease requiring hemolysis inhibition, such as a disease requiring inhibition of hemolysis of the classical pathway of complement immunity and/or the alternative pathway of complement immunity.

In some embodiments, the complement system-associated disease or disorder is a disease in which inhibition of C3b deposition activity is desired, such as Dense Deposit Disease (DDD).

In some embodiments, the hybrid protein mutants or fusion protein molecules or immunoconjugates or compositions or medicines or formulations comprising the same of the present invention will delay the onset of the condition and/or symptoms associated with the condition.

In some embodiments, the hybrid protein mutants or fusion protein molecules or immunoconjugates or the nucleic acid encoding the same or the composition or medicament or formulation comprising the proteins or nucleic acids of the present invention can also be administered in combination with one or more other therapies, e.g., therapeutic modalities and/or other therapeutic agents, for use as described herein, e.g., for the prevention and/or treatment of the related diseases or disorders as mentioned herein.

The routes of administration of the hybrid protein mutants or fusion protein molecules or immunoconjugates or the nucleic acid encoding the same or the composition or medicament or formulation comprising the proteins or nucleic acids of the present invention depend on known methods, e.g., injection or infusion.

In other aspects, the invention provides the use of the hybrid protein mutants or fusion protein molecules or immunoconjugates or the nucleic acid encoding the same or the composition or medicament or formulation comprising the proteins or nucleic acids of the present invention in the production or preparation of a medicament for use as described herein, e.g., for preventing or treating a complement C5-related disease or condition mentioned herein.

In other aspects, the invention also relates to the hybrid protein mutants or fusion protein molecules or immunoconjugates or the nucleic acid encoding the same or the composition or medicament or formulation comprising the proteins or nucleic acids of the present invention for use in therapy, for example for preventing or treating complement C5-related disease or condition mentioned herein.

## IV. Diagnosis and Detection

In certain embodiments, the fusion proteins or immunoconjugates thereof provided herein can be used to detect the presence of complement C5 in a biological sample.

The term "detection" as used herein includes quantitative or qualitative detection, and exemplary detection methods may involve immunohistochemistry, immunocytochemistry, flow cytometry (e.g., FACS), magnetic beads complexed with antibody molecules, ELISA assays, PCR-techniques (e.g., RT-PCR). In certain embodiments, the biological sample is a body fluid.

In certain embodiments, the method comprises contacting a biological sample with the fusion protein molecule as described herein under conditions that allow it to bind to complement C5, and detecting whether a complex is formed between the molecule and complement C5, wherein the formation of a complex indicates the presence of complement C5. The method may be an in vitro or in vivo method. In one embodiment, the fusion protein molecules of the present invention are used to select subjects suitable for treatment with an inhibitor against complement C5 (e.g., an antibody against complement C5, such as the anti-C5 antibody or antigen-binding fragment thereof or the fusion protein thereof or the immunoconjugate thereof of the present invention, etc.), for example, wherein complement C5 is a biomarker for selecting the subject.

In certain embodiments, a labeled fusion protein or the immunoconjugate thereof of the invention is provided. Labels include, but are not limited to, directly detected labels or moieties (e.g., fluorescent labels, chromophore labels, electron-dense labels, chemiluminescent labels, and radioactive labels), and indirectly detected moieties, such as enzymes or ligands, for example, by enzymatic reactions or molecular interactions.

In some embodiments, the label is a lable such as biotin or a His tag.

In some embodiments provided herein, the sample is obtained prior to treatment with a molecule of the invention or a composition, drug, or formulation comprising thereof. In some embodiments, the sample is obtained prior to treatment with another therapy. In some embodiments, the sample is obtained during treatment with another therapy, or after treatment with another therapy.

In some embodiments, complement C5 is detected prior to treatment, for example, prior to initiation of treatment or prior to a treatment after a treatment interval.

In some embodiments, a method of treating a disease of the present invention is provided, the method comprising: testing a subject (e.g., a sample) (e.g., a subject sample) for the presence of complement C5, thereby determining a complement C5 value, comparing the complement C5 value with a control value (e.g., a value in a normal individual), and if the complement C5 value is greater than the control value, administering to the subject a therapeutically effective amount of a molecule of the present invention, or a composition, drug or formulation comprising thereof optionally in combination with one or more other therapies, thereby treating the disease.

## Examples

### Example1. Molecular construction of hybrid protein

In order to construct the protein expression vector of the hybrid molecule involved in the present invention and the complement regulatory molecule of the prior art, the DNA encoding each protein was subcloned into the pcDNA3.1 expression vector (purchased from Biofeng) with the synthesized gene DNA (GENEWIZ Inc., Suzhou/Sangon Biotech (Shanghai) Co., Ltd.) as a template by using a method of conventional molecular cloning technology. The plasmid was verified by sequencing and then was used for transient protein expression. Each encoded protein was added with a signal peptide MGWSCIILFLVATATGVHS to the N-terminus and a GHHHHHH 6×His tag to the C-terminus to facilitate subsequent secretion expression with mammalian cells and nickel column purification.

The amino acid sequence of each protein is as follows:

>CR13m (human CR1 CCP1-3 or SCR1-3, comprising N29K, S37Y, G79D and D109N mutations, the sequence is derived from patent US9988611_B2): SEQ ID NO: 34

QCNAPEWLPFARPTNLTDEFEFPIGTYL**K**YECRPGY**Y**GRPFSIICLKNSVWTGAKDRCRRKSCRNPPDPVN
GMVHVIK**D**IQFGSQIKYSCTKGYRLIGSSSATCIISG**N**TVIWDNETPICDRIPCGLPPTITNGDFISTNRENFH
YGSVVTYRCNPGSGGRKVFELVGEPSIYCTSNDDQVGIWSGPAPQCI*GHHHHHH*

>D24 (human DAF/CD55 CCP2-4 or SCR2-4, the sequence can take reference from Hui-fen Zhang et al., THE JOURNAL OF BIOLOGICAL CHEMISTRY, Vol. 276, No. 29, Issue of July 20, pp. 27290-27295, 2001): SEQ ID NO: 35

SCEVPTRLNSASLKQPYITQNYFPVGTVVEYECRPGYRREPSLSPKLTCLQNLKWSTAV
EFCKK**KSCPNPGEIRNGQIDVPGGILFGATISFSCNTGYKLFGSTSSFCLISGSSVQWSDP**
**LPECREIYCPAPPQIDNGIIQGERDHYGYRQSVTYACNKGFTMIGEHSIYCTVNNDEGE**
**WSGPPPECRG***GHHHHHH*

>FH15 (human CFH CCP1-5 or SCR1-5, comprising V62I mutation, the sequence can take reference from Masha Fridkis-Hareli et al., Blood. 2011 Oct 27; 118(17): 4705-4713 and Agustín Tortajada et al., Hum Mol Genet. 2009 September 15; 18(18): 3452-3461): SEQ ID NO:36

EDCNELPPRRNTEILTGSWSDQTYPEGTQAIYKCRPGYRSLGN**I**IMVCRKGEWVALNP
LRKCQKRPCGHPGDTPFGTFTLTGGNVFEYGVKAVYTCNEGYQLLGEINYRECDTDGWTN
DIPICEVVKCLPVTAPENGKIVSSAMEPDREYHFGQAVRFVCNSGYKIEGDEEMHCSDDGF

WSKEKPKCVEISCKSPDVINGSPISQKIIYKENERFQYKCNMGYEYSERGDAVCTESGWRPL
PSCEEKSCDNPYIPNGDYSPLRIKHRTGDEITYQCRNGFYPATRGNTAKCTSTGWIPAPRCTL
KPGHHHHHH

>F1D34-1 (human CFH CCP1 or SCR1 - human DAF/CD55 CCP3-4 or SCR3-4): SEQ ID NO: 22
>F1D34-2i (human CFH CCP1 or SCR1 - human DAF/CD55 CCP3-4 or SCR3-4, comprising a V62I mutation): SEQ ID NO: 24
>CR15D34 (human CR1 CCP15 or SCR15 - human DAF/CD55 CCP3-4 or SCR3-4): SEQ ID NO: 37

GHCQAPDHFLFAKLKTQTNASDFPIGTSLKYECRPEYYGRPFSITCLDNLVWSSPKDVC
KRK**SCPNPGEIRNGQIDVPGGILFGATISFSCNTGYKLFGSTSSFCLISGSSVQWSDPLPE**
**CREIYCPAPPQIDNGIIQGERDHYGYRQSVTYACNKGFTMIGEHSIYCTVNNDEGEWS**
**GPPPECRG***GHHHHHH*

>FH13 (human CFH CCP1-3 or SCR1-3, comprising a V62I mutation, there are two more amino acids at the C-terminus for connecting His purification tag): SEQ ID NO: 51

EDCNELPPRRNTEILTGSWSDQTYPEGTQAIYKCRPGYRSLGN**I**IMVCRKGEWVALNP
LRKCQKRPCGHPGDTPFGTFTLTGGNVFEYGVKAVYTCNEGYQLLGEINYRECDTDGWTN
DIPICEVVKCLPVTAPENGKIVSSAMEPDREYHFGQAVRFVCNSGYKIEGDEEMHCSDDGF
WSKEKPKCVEIS*GHHHHHH*

>D14 (human DAF/CD55 CCP1-4 or SCR1-4, no leader sequence, there are two more amino acids at the C-terminus for connecting His purification tag): SEQ ID NO: 52

DCGLPPDVPNAQPALEGRTSFPEDTVITYKCEESFVKIPGEKDSVICLKGSQWSDIEEFC
NRSCEVPTRLNSASLKQPYITQNYFPVGTVVEYECRPGYRREPSLSPKLTCLQNLKWSTAVE
FCKKKSCPNPGEIRNGQIDVPGGILFGATISFSCNTGYKLFGSTSSFCLISGSSVQWSDPLPEC
REIYCPAPPQIDNGIIQGERDHYGYRQSVTYACNKGFTMIGEHSIYCTVNNDEGEWSGPPPE
CRGKS*GHHHHHH*

**Example 2. Hybrid Protein expression and purification**

After amplification of the plasmid with strain DH5 alpha (Yeasen Biotechnology), the plasmid was prepared using the NucleoBond Xtra Midi Plus (MACHEREY-NAGEL, see the product Manual for details). Expi293 cells (purchased from Thermo fisher) were transfected with the prepared plasmids using PEI (Polyscience) for transient expression of proteins (see Jäger, V., et al., BMC Biotechnol 13, 52, 2013. for the transfection method). The harvested supernatant was purified by affinity chromatography in a nickel ion pre-packed column (GE life sciences, HisTrap HP, see Product Manual for details). The protein sample was first washed with 20 mM imidazole buffer to remove impurities, and then eluted with 250mM or 500 mM imidazole buffer. The protein sample was then buffer-exchanged to PBS buffer using an ultrafiltration concentrator (Amicon® Ultra, Merck Millipore).The protein samples after fluid change were sterilized by filtration through 0.2 μm filters, and protein quantification was performed according to the theoretical extinction coefficient using the NanoDrop (Thermofisiher) A280 method.

**Example 3. Detection of protein purity**

**SDS-PAGE**

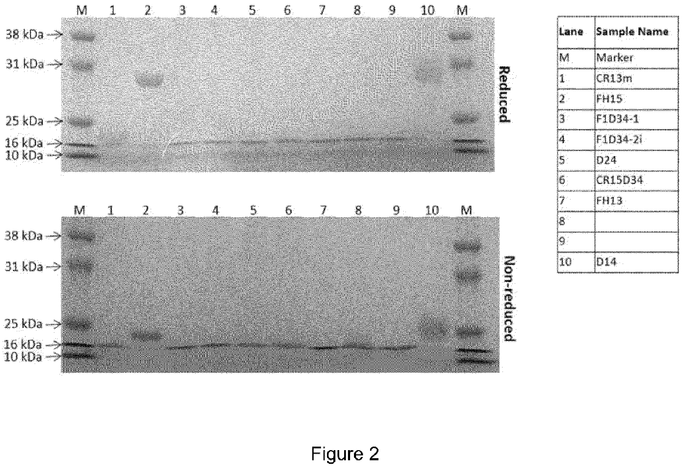

2.5 μg of protein was each taken and added proportionally to the loading buffer (Sangon Biotech) with reducing agent and without reducing agent, respectively. The samples with loading buffer containing reducing agent were boiled at 95°C for 5 minutes to fully denature the proteins. For the samples with loading buffer without reducing agent, no heat treatment was performed. Electrophoresis was performed with a 10% Precast-GLgel Tris-Glycine precast gel (Sangon Biotech) in a Mini-PROTEAN® Tetra electrophoresis system (BioRad). After completing the electrophoresis, the gel was stained with 0.1% Coomassie Brilliant Blue staining solution and de-stained with an ethanol-glacial acetic acid solution.
The SDS-PAGE electrophoresis results showed that except for a slight diffusion of the D14 protein band, the other protein bands were relatively clear, indicating that the purity of the proteins under both reducing and non-reducing conditions was good (as set forth in Figure 2). Additionally, due to differences in protein reduction, denaturation, modification status, and electrophoresis conditions, there was a certain discrepancy between the electrophoretic molecular weight and the theoretical molecular weight.

**SEC-HPLC**

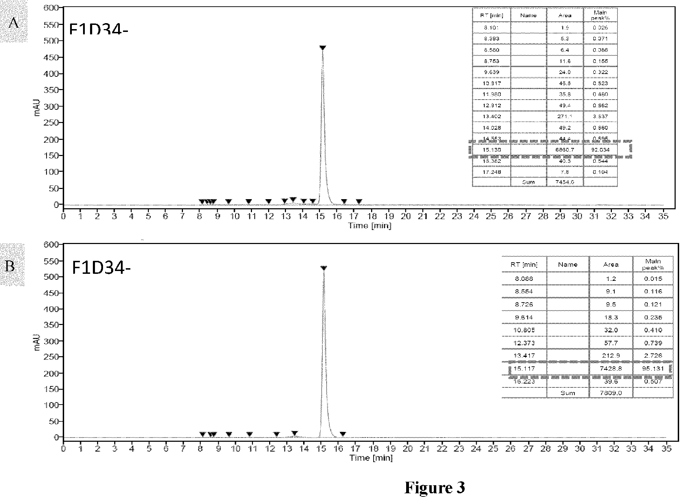

Protein purity analysis was performed using Size Exclusion Chromatography (SEC). The purified protein sample in PBS was applied to a TSKgel Super SW3000 column (300×4.6 mm, 5 μm; TOSOH). The SEC was performed using a U3000 HPLC instrument (DIONEX). The elution was carried out isocratically at a flow rate of 0.25 mL/min. All proteins were detected using UV detection at wavelengths of 280 nm and 214 nm. Component analysis was performed using the instrument's built-in software.
The analysis results showed that the monomer purity of the hybrid proteins FID34-1 and FID34-2i in solution reached 92% and 95%, respectively (Figure 3 A/B), indicating good monomer purity.

**Example 4. Detection of complement inhibitory activity of hybrid protein**

**4.1 Detection of inhibitory activity on Classical Pathway of complement (CP)**

In this experiment, the degree of inhibition of hemolysin-mediated hemolysis of sheep red blood cells caused by normal human serum complement activity was measured by testing the protein to be tested at different concentrations, and the inhibitory activity of each protein on complement CP was analyzed and compared.
The experimental process was briefly described as follows:

(1) Gradient dilution of proteins: the protein was formulated into a 12000 nM solution with GVB++ buffer (Gelatin Flora Buffer comprising Ca2+ and Mg2+, Cat. No.: 25-02080, TIANDZ), followed by 4-fold gradient dilution, with a total of 7 gradients;
(2) Dilution of serum: The normal human serum NHS (Shanghai Schbio Co., Ltd.) was taken out of the -70°C refrigerator, followed by naturally thawing at 4°C and then the serum was diluted to 4% with GVB++ buffer;
(3) Activation of sheep red blood cells: the sheep red blood cells (Nanjing SenBeiJia Biological Technology Co., Ltd.) were washed with GVB++ buffer until the supernatant was transparent, then resuspended with GVB++ buffer, and hemolysin (BM351Y, Beijing Bersee Technology Co., Ltd.) was added to the red blood cell suspension at a ratio of 1:4000, followed by incubation at 4°C for 15 minutes to activate the sheep red blood cells. The activated sheep red blood cells were washed twice with GVB++ and resuspended in GVB++ buffer;

(4) Incubation: 25 μL each of the diluted protein and serum were mixed in a 96-well plate, then 50 μL of the red blood cell suspension obtained in step (3) was added, resulting in an initial final concentration of the protein of 3000 nM and a final concentration of the serum of 1%. The mixture was blended thoroughly and placed in a 37°C constant temperature incubator for 1 hour, with a negative control (comprising only serum) and a positive control (comprising only serum and red blood cells);

(5) Termination: After the incubation, 100 μL of 20 mM EDTA-GVB buffer (GVBE) was added to each reaction well to terminate the reaction;

(6) Reading value: The 96-well plate was centrifuged at 3000 rpm for 5 minutes. 100 μL of the supernatant was added into a new 96-well flat-bottom plate. The absorbance value at OD405nm was measured in a multi-function plate reader, and the data were saved;

(7) Data processing: the obtained OD405nm reading value was put into the following formula to calculate the red blood cell hemolysis inhibition rate:

Hemolysis inhibition rate (%) = (positive control reading value - experimental group reading value) / (positive control reading value - negative control reading value) * 100%

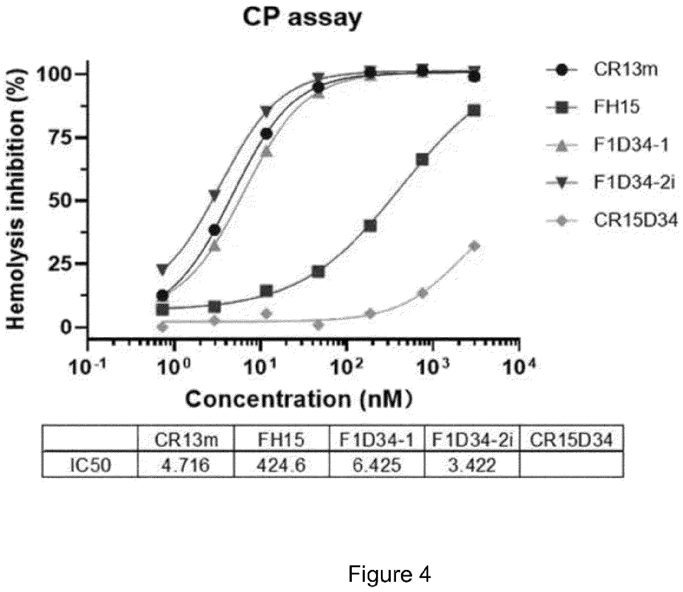

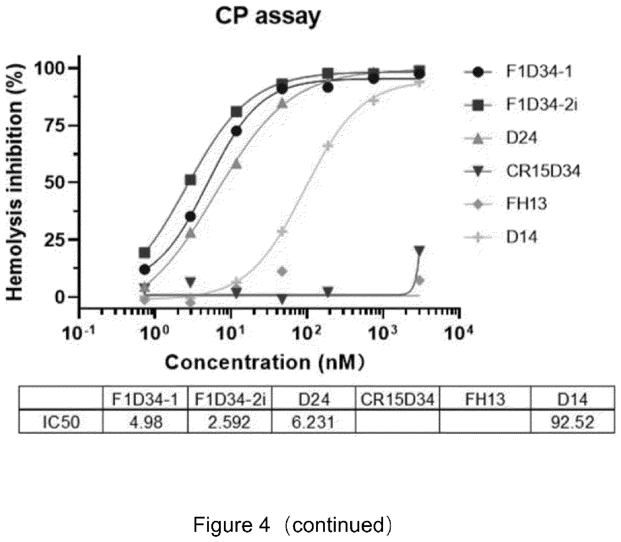

Then the IC50 value of the protein was calculated by plotting a four-parameter fit curve, with the final protein concentration as the abscissa and the hemolysis inhibition rate as the ordinate model. The results were shown in Figure 4.

The experimental test results of CP hemolytic inhibitory activity showed that the hybrid proteins of CFH and DAF F1D34-1 and F1D34-2i both had strong CP inhibitory activity, which was equivalent to that of CR1 activity-enhancing fragment CR13m and DAF fragment D24, significantly better than that of CFH fragment FH15 and DAF fragment D14, and also far superior to that of CR15D34, a hybrid protein of CR1 and DAF. Only weak CP inhibitory activity was detected for CR15D34, while CFH fragment FH13 had little activity (as set forth in Figure 4), indicating that the hybrid protein obtained through domain hybridization had excellent CP inhibitory activity.

**4.2 Detection of inhibitory activity on Alternative Pathway of complement (AP)**

In this experiment, the degree of inhibition of rabbit erythrocyte hemolysis caused by normal human serum complement activity was measured by testing the protein to be tested at different concentrations, and the inhibitory activity of each protein on complement AP was analyzed and compared.

The experimental process was briefly described as follows:

(1) Gradient dilution of proteins: The protein was formulated into a 28000 nM solution with GVBMG buffer (Gelatin Flora Buffer comprising Mg2+ and EGTA, Cat. No.: 25-02090, TIANDZ), followed by 4-fold gradient dilution, with a total of 7 gradients;

(2) Dilution of serum: The normal human serum NHS (Shanghai Schbio Co., Ltd.) was taken out of the -70°C refrigerator, followed by naturally thawing at 4°C and the serum was diluted to 60% with GVBMG;

(3) Preparation of rabbit red blood cells: rabbit red blood cells (Nanjing SenBeiJia Biological Technology Co., Ltd.) were washed with GVBMG until the supernatant was transparent, and then resuspended with GVBMG;

(4) Incubation: 25 μL each of the diluted protein and serum were mixed in a 96-well plate, then 50 μL of the red blood cell suspension obtained in step (3) was added, resulting in an initial final concentration of the protein of 7000 nM and a final concentration of the serum of 15%. The mixture was blended thoroughly and placed in a 37°C constant temperature incubator for 1 hour, with a negative control (comprising only serum) and a positive control (comprising only serum and red blood cells);

(5) Termination: After the incubation, 100 μL of 10 mM EDTA-GVB buffer (GVBE) was added to each reaction well to terminate the reaction;

(6) Reading value: The 96-well plate was centrifuged at 3000 rpm for 5 minutes. 100 μL of the supernatant was added into a new 96-well flat-bottom plate. The absorbance value at OD405nm was measured in a multi-function plate reader, and the data were saved;

(7) Data processing: the obtained OD405nm reading value was put into the following formula to calculate the red blood cell hemolysis inhibition rate:

Hemolysis inhibition rate (%) = (positive control reading value - experimental group reading value) / (positive control reading value - negative control reading value) * 100%

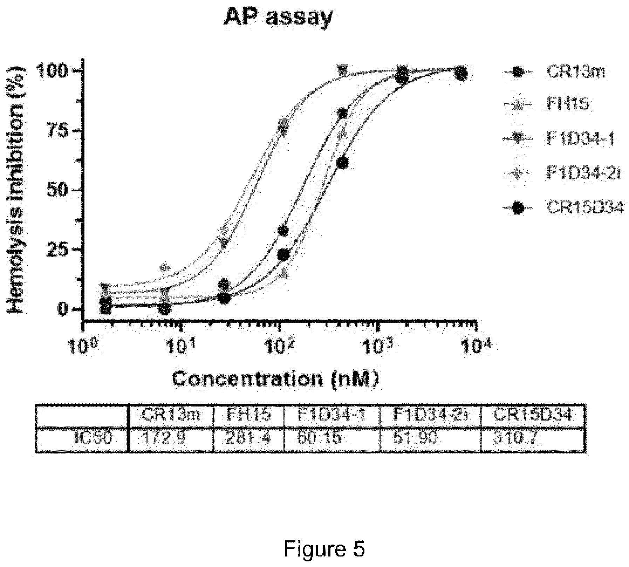

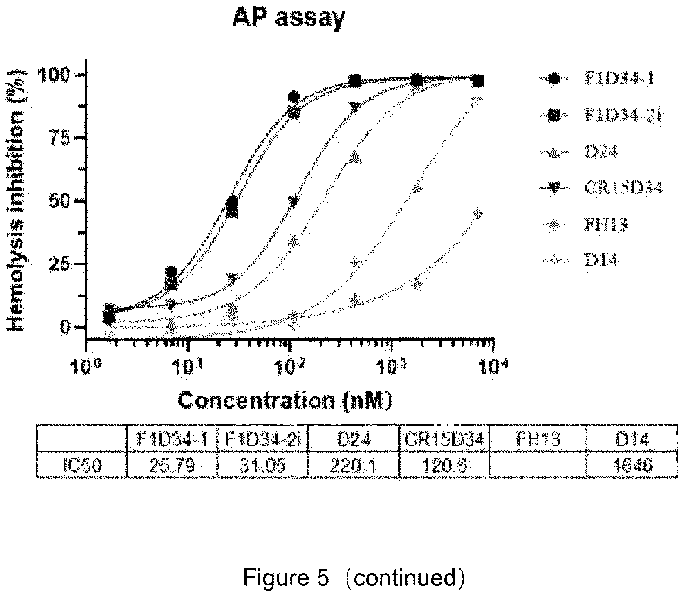

Then the IC50 value of the antibody was calculated by plotting a four-parameter fit curve, with the final protein concentration as the abscissa and the hemolysis inhibition rate as the ordinate. The results were shown in Figure 5.

The experimental test results of AP hemolytic inhibitory activity showed that the hybrid proteins of CFH and DAF FID34-1 and FID34-2i both had strong AP inhibitory activity, which was better than that of CFH fragments FH15 and FH13, and DAF fragments D24 and D14, and was also superior to that of the hybrid protein of CR1 and DAF CR15D34 and the CR1 activity-enhancing fragment CR13m (as set forth in Figure 5), indicating that the hybrid protein obtained through domain hybridization had more excellent AP inhibitory activity.

**4.3 Detection of inhibitory activity in complement C3b deposition**

In this experiment, the inhibition of C3b deposition on the surface of rabbit red blood cells by different complement regulatory proteins after human complement activation was detected, and the inhibitory activity of each protein on C3b deposition was analyzed and compared.

The experimental process was briefly described as follows:

(1) Gradient dilution of proteins: The protein was prepared into a 1400 nM solution with GVBMG buffer (Gelatin Flora Buffer comprising Mg2+ and EGTA, Cat. No.: 25-02090, TIANDZ), followed by 3-fold gradient dilution, with a total of 7 gradients;

(2) Dilution of serum: In order to prevent complement activation from causing hemolysis and rupture of rabbit red blood cells, resulting in the inability to accurately detect the deposition of C3b on the cell surface, the C5-depleted normal human serum was used for the experiment. C5-Dpl NHS (Complement Technology) was taken out of the -70°C refrigerator, followed by naturally thawing at 4°C and the serum was diluted to 40% with GVBMG;

(3) Preparation of rabbit red blood cells: 4% rabbit red blood cells (Nanjing SenBeiJia Biological Technology Co., Ltd.) were washed with GVBMG until the supernatant was transparent, and then resuspended with GVBMG to a cell density of $1 \times 10^7$ cells/mL;

(4) Incubation: 25 μL each of the diluted protein and serum were mixed in a 96-well plate, then 50 μL of the red blood cell suspension obtained in step (3) was added, resulting in an initial final concentration of the protein of 350 nM and a final concentration of the serum of 10%, the mixture was blended thoroughly and placed in a 37°C constant-temperature incubator for 1 hour, with a negative control (comprising only complement-inactivated serum and red blood cells) and a positive control (comprising only normal serum and red blood cells);

(5) Termination: After the incubation, 100 μL of 10 mM EDTA-GVB buffer (GVBE) was added to each reaction well to terminate the reaction;

(6) Detection: the incubated cells were washed three times with PBS, fluorescein-labeled anti-human C3b/iC3b antibody was added for staining (APC anti-complement C3b/iC3b Antibody, Biolegend), followed by incubation at 4°C for 30 minutes and washing again 3 times. The APC fluorescence MFI (mean fluorescence intensity) of each sample was measured with a flow cytometer (Cytoflex, Beckman), and the data were saved;

(7) Data processing: the deposition rate of C3b on the surface of red blood cells was calculated with the following formula:

$$\text{Deposition rate (\%)} = \text{experimental group MFI value} / \text{positive control MFI value} * 100\%$$

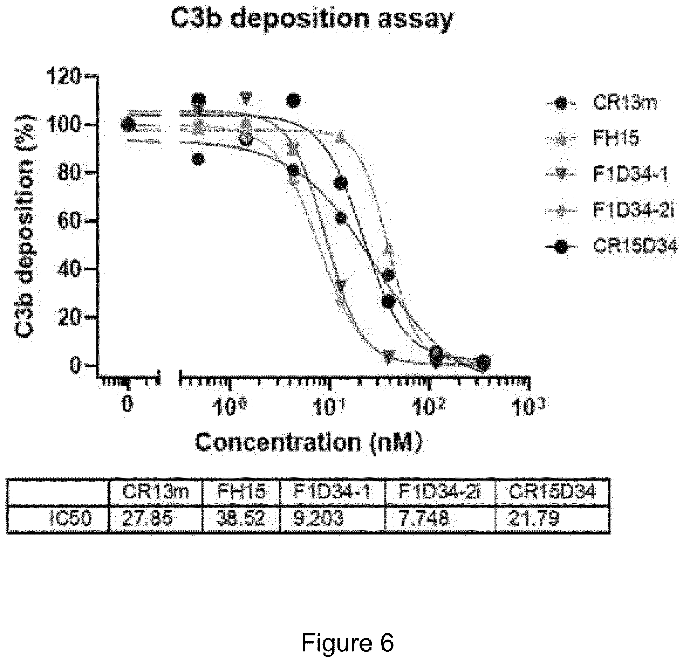

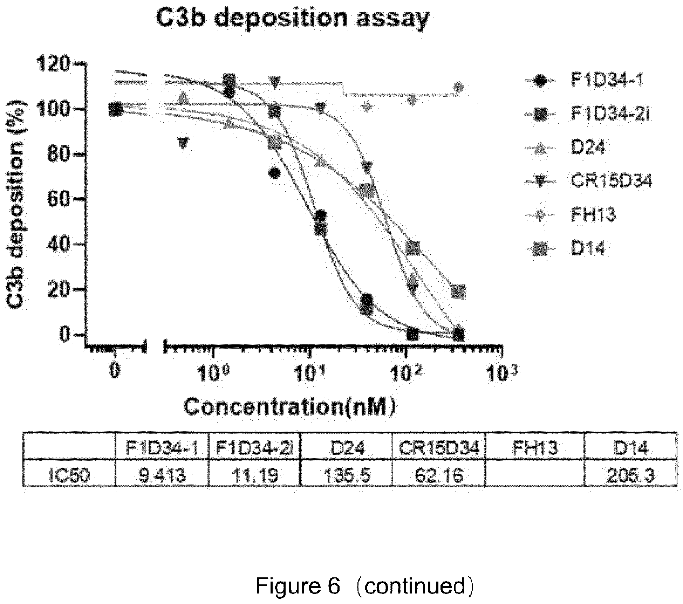

Then the IC50 value of the antibody was calculated by plotting a four-parameter fit curve, with the final protein concentration as the abscissa and the deposition rate as the ordinate. The results were shown in Figure 6.

The experimental test results of C3b deposition showed that the hybrid proteins of CFH and DAF F1D34-1 and F1D34-2i both had strong C3b deposition inhibitory activity, which was better than that of the CFH fragments FH15 and FH13 and the DAF fragments D24 and D14, and was also better than that of the hybrid protein of CR1 and DAF CR15D34 and the CR1 activity-enhancing fragment CR13m (as set forth in Figure 6), indicating that the hybrid protein obtained through domain hybridization had more excellent C3b deposition inhibitory activity.

**Example 5: Preparation and activity of anti-C5 antibodies**

**5.1 Anti-C5 antibody preparation**

The anti-human C5 antibody was obtained through mouse immunization, hybridoma screening, and humanized transformation. For its sequence and preparation method, please refer to CN113754763A (16H46L39am).

**5.2 pH-dependent engineering of anti-C5 antibodies**

Existing reports have shown that molecules with pH-dependent dissociation properties can quickly dissociate from target molecules in endocytoses, thereby reducing target-mediated clearance (TMDD) and prolonging half-life in vivo, and said molecules with pH-dependent properties can be rapidly obtained through "histidine replacement" (Casim A. Sarkar et al., Nat Biotechnol. 2002 Sep; 20(9):908-13.doi:10.1038/nbt725; Tomoyuki Igawa et al., Nat Biotechnol. 2010 Nov; 28(11) :1203-7.doi:10.1038/nbt.1691.).

After screening for "histidine substitution" in the antibody CDR region, a C5 antibody phAb with pH-dependent dissociation properties was finally obtained. The sequence is as set forth in Table 1.

ForteBio Octet K2 was used to detect the pH-dependent dissociation properties of antibodies. The detection process is briefly described as follows:

ANTI-HUMAN IgG FC sensor (FORTEBIO, 18-5060) is used to load the antibody in a pH neutral buffer with an antibody concentration of 10 μg/mL. After baseline, bind the C5 antigen (Complement Technology, A120) with an antigen concentration of 10 μg/mL, and then dissociated in pH7.4 buffer and pH5.4 buffer respectively. The pH5.4 buffer can be obtained by adjusting the pH value using phosphate buffer or citrate buffer. The parameter settings are: loading 180s, baseline 60s, association 180s, and dissociation 500s. For detailed methods, please refer to ForteBio Octet Instructions for Use.

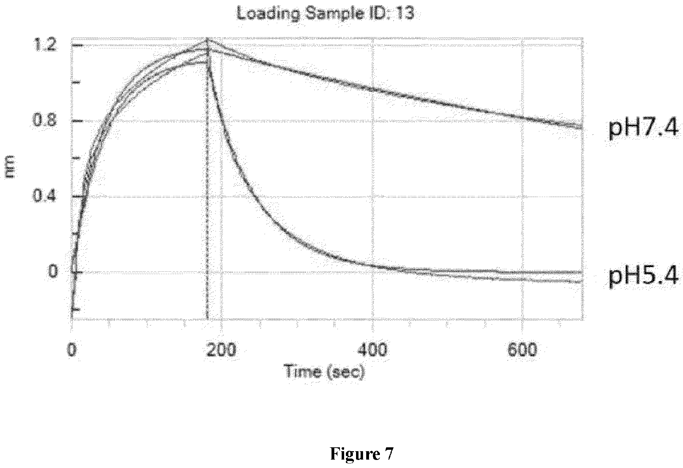

The detection results were shown in Figure 7, and the modified C5 antibody phAb dissociates from C5 faster in pH5.4 buffer than in pH7.4 buffer.

### 5.3 pH-dependent C5 antibody in vivo activity detection

In order to verify the complement inhibitory activity of pH-dependent C5 antibodies in animals, human C5 transgenic mice were used for testing. Specifically, the serum of human C5 transgenic mice (Southern Model Animal Center) was collected separately, and the human C5 content in the serum was detected using ELISA. According to the human C5 content in the serum, 1 mg of C5 antibody was administered per 105 μg/mL C5 for tail vein injection. The phAb is the C5 antibody with pH-dependent properties of the present application, and the Ab is 16H46L39am disclosed in CN113754763A. Both use the same human IgG4 constant region sequence (with S228P and LA mutations (M428L/N434A), SEQ ID NO:59). Serum was collected before and after injection (1 hour, 3 days, 6 days, 9 days, 12 days, 15 days, 20 days and 23 days), and serum complement activity was tested according to the following process:

(1) 1 mL of chicken red blood cells (SBJ-RBC-C003, Nanjing SenBeiJia Biological Technology Co., Ltd.) was taken and washed 3 times with GVB++ (gelatin Flora buffer containing Ca2+ and Mg2+, Cat. No.: 25-02080, Tianenze). After resuspension, anti-chicken red blood cell antibody (1ug/mL, anti-cRBCs, Cat. No.: 203-4139, Rockland) was added and the mixture was incubated at 4°C for 15 minutes to activate chicken red blood cells. The it is centrifuged, washed 3 times with GVB++, and the red blood cells were resuspended in 1mL GVB++.

(2) C5-depleted human serum (C5-deplet NHS, CompTech) at a final concentration of 10% was used and mixed with a transgenic mouse serum sample at final concentration of 6.25%. The diluents are both GVB++. Control 1 was set to contain only serum (ODctrl1) and GVB++, Control 2 was the serum sample before antibody injection (ODctrl2), and its hemolysis level was used as a control for 100% hemolysis.

(3) 30ul of activated chicken red blood cells was pippeted into the serum and incubateed at 37°C for 1 hour. After centrifugation, 80 ul of the supernatant was taken and the OD405 was read on a multifunctional plate reader.

$$\text{Hemolysis rate (\%)} = (\text{OD sample} - \text{ODctrl1})/(\text{ODctrl2} - \text{ODctrl1}) \times 100$$

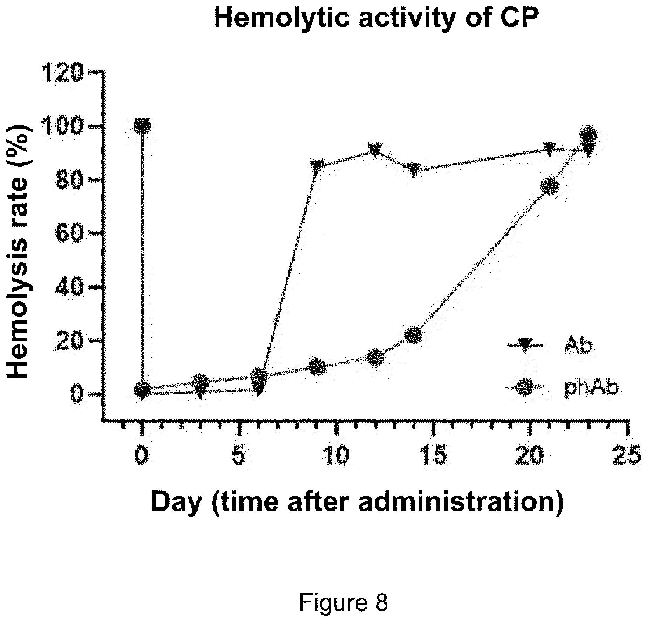

The test results are as set forth in Figure 8. According to the test results, the pH-dependent C5 antibody phAb has more sustained complement inhibitory activity.

Table 3. Anti-C5 antibody sequences

| SEQ ID NO | | Sequence |
|---|---|---|
| 39 | VH | QVQLVQSGAEVKKPGASVKVSCKASGYTFTNYGMNWVRQAPGQGLE WMGWINTYTGEPTYADDFKGRFTFTLDTSTSTAYLELSSLRSEDTAVYY CARTKVP**H**YFAYAMDYWGQGTLVTVSS |
| 40 | HCDR 1 | NYGMN |
| 41 | HCDR 2 | WINTYTGEPTYADDFKG |
| 42 | HCDR 3 | TKVPHYFAYAMDY |

(continued)

| SEQ ID NO | | Sequence |
|---|---|---|
| 43 | Heavy chain | ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES |
| | consta nt region | KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRW QEGNVFSCSVLHEALHAHYTQKSLSLSLGK |
| 44 | HC | QVQLVQSGAEVKKPGASVKVSCKASGYTFTNYGMNWVRQAPGQGLE WMGWINTYTGEPTYADDFKGRFTFTLDTSTSTAYLELSSLRSEDTAVYY CARTKVPHYFAYAMDYWGQGTLVTVSSASTKGPSVFPLAPCSRSTSEST AALGCLVKDYFPEPVTVSWNSGALTSGVHTFPAVLQSSGLYSLSSVVTVP SSSLGTKTYTCNVDHKPSNTKVDKRVESKYGPPCPPCPAPEFLGGPSVFL FPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKA KGQPREPQVYTLPPSQEEMTKNQVSLTCLVKGFYPSDIAVEWESNGQPE NNYKTTPPVLDSDGSFFLYSRLTVDKSRWQEGNVFSCSVLHEALHAHYT QKSLSLSLGK |
| 45 | VL | DIVLTQSPSSLSASVGDRVTITCKASQSVDY**H**GDSYLNWYQQKPGKPPK LLIYAASNLESGIPSRFSGSGSGTDFTLTISSLQPEDFATYYCQQSNEDPST FGGGTKLEIK |
| 46 | LCDR 1 | KASQSVDYHGDSYLN |
| 47 | LCDR 2 | AASNLES |
| 48 | LCDR 3 | QQSNEDPST |
| 49 | Light chain con- sta nt region | RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC |
| 50 | LC | DIVLTQSPSSLSASVGDRVTITCKASQSVDYHGDSYLNWYQQKPGKPPK LLIYAASNLESGIPSRFSGSGSGTDFTLTISSLQPEDFATYYCQQSNEDPST FGGGTKLEIKRTVAAPSVFIFPPSDEQLKSGTASVVCLLNNFYPREAKVQ WKVDNALQSGNSQESVTEQDSKDSTYSLSSTLTLSKADYEKHKVYACE VTHQGLSSPVTKSFNRGEC |
| 59 | IgG4 Fc region (includ ing LA mutati on) | APEFLGGPSVFLFPPKPKDTLMISRTPEVTCVVVDVSQEDPEVQFNWYV DGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VLHEALHAHYTQKSLSLSLGK |

**Example 6 Construction of hybrid protein mutants and fusion protein molecules comprising the hybrid protein mutants**

6.1 Molecular construction and mutant design

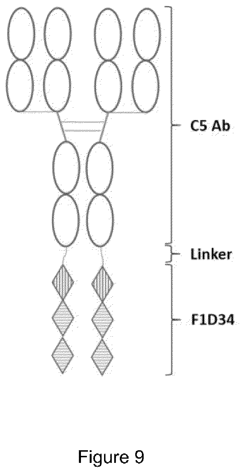

To obtain candidate molecules with better complement inhibitory activity, we fused the obtained hybrid protein molecules with C5 antibody (the fusion protein structure is shown in Figure 9), resulting in hybrid protein-antibody fusion proteins (Ab-F1D34: heavy chain: SEQ ID NO:60, light chain: antibody light chain: SEQ ID NO: 50). The transient expression results showed that the Ab-F1D34 protein was well expressed and the monomer purity was very high in the solution state. However, turbidity of the protein solution was observed when stored at 4°C, suggesting that there may be certain defects in the protein solubility, which is not conducive to the development of high-concentration pharmaceutical formulations. Ab-F15 was also prepared as a control fusion protein (Ab-F15: heavy chain: SEQ ID NO: 97; light chain: antibody light chain: SEQ ID NO: 50).

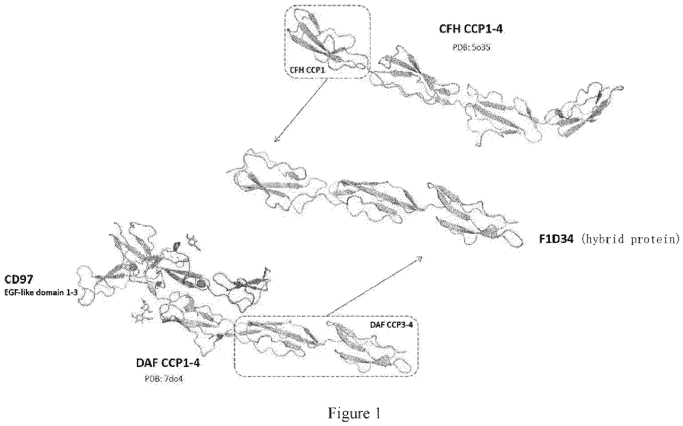

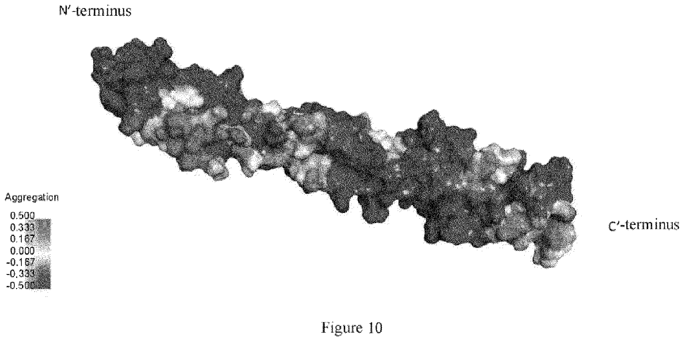

Since the antibody itself has good solubility, the solubility of the hybrid protein was firstly optimized. First, the structural model of the hybrid protein F1D34 was constructed based on the reported crystal structures of CFH (PDB: 5o35) and DAF (PDB: 7do4) (as shown in Figure 1). Then, the computer-aided molecular design software Discovery Studio 2020 (BIOVIA) was used to predict the aggregation sites (as shown in Figure 10, the red region is the hydrophobic amino acid site that potentially mediates aggregation), and the selected amino acid sites that potentially mediate protein aggregation were mutated. Finally, the mutated protein model was predicted again to obtain beneficial mutations that reduce protein aggregation, that is, the amino acids were mutated into more hydrophilic amino acids that is structurally similar. The mutation list is as follows (Tables 4 and 5):

Table 4. Hybrid protein mutations

| Mutation site | Note |
| --- | --- |
| C' -KS | Addition of KS amino acids at C'-terminus |
| L56E | Amino acid positions corresponding to the F1D34 sequence |
| L59D | Same as above |
| L59S | Same as above |
| G82D | Same as above |
| F86Y | Same as above |
| F107E | Same as above |
| L109D | Same as above |
| L109E | Same as above |
| I110T | Same as above |

Wherein, C'-KS indicates that KS amino acids are added at the C' -terminus of the fusion protein F1D34, namely, the fusion protein comprises an extended DAF sequence, and the rest mutant amino acid positions correspond to the F1D34 sequence.

Table 5: Hybrid protein optimization mutation sites

| Mutation site | Note |
| --- | --- |
| KS | Addition of amino acids KS at the C' -terminus |
| L56E | The amino acid at position 56 changes from L to E |
| L59D | The amino acid at position 59 changes from L to D |
| L59S | The amino acid at position 59 changes from L to S |
| G82D | The amino acid at position 82 changes from G to D |
| I110T | The amino acid at position 110 changes from I to T |
| Note: The amino acids KS added at the C'-terminus is derived from the amino acids at positions 286-287 of DAF (the extended sequence of CCP3-4, see Table 1 ), and the rest mutant amino acid positions correspond to the F1D34 sequence (as set forth in SEQ ID NO: 20). | |

The amino sequence of each mutant is as follows:

>F1D34-KS(SEQ ID NO: 60)
> F1D34-KS-6×His (italic): SEQ ID NO:56

EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRG**KS***GHHHHHH*

>F1D34-KS-L56E(SEQ ID NO: 62)
> F1D34-KS-L56E-6×His (italic): SEQ ID NO:57


EDCNELPPRRNTEILTGSWSDQTYPEGTQAIYKCRPGYRSLGNIIMVCRKGEWVA**E**NPLRKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRG**KS***GHHHHHH*

>F1D34-KS-L59D(SEQ ID NO: 64)
>F1D34-KS-L59D -6×His (italic): SEQ ID NO:58


EDCNELPPRRNTEILTGSWSDQTYPEGTQAIYKCRPGYRSLGNIIMVCRKGEWVALNP**D**RKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRG**KS***GHHHHHH*

>F1D34-KS-L59S(SEQ ID NO: 66)
>F1D34-KS-L59S -6×His (italic): SEQ ID NO:88


EDCNELPPRRNTEILTGSWSDQTYPEGTQAIYKCRPGYRSLGNIIMVCRKGEWVALNP**S**RKCQKKSCPNP
GEIRNGQIDVPGGILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRG**KS***GHHHHHH*

>F1D34-KS-G82D(SEQ ID NO: 68)
>F1D34-KS-G82D -6×His (italic): SEQ ID NO:89


EDCNELPPRRNTEILTGSWSDQTYPEGTQAIYKCRPGYRSLGNIIMVCRKGEWVALNPLRKCQKKSCPNP
GEIRNGQIDVP**D**GILFGATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRG**KS***GHHHHHH*

> F1D34-KSF86Y(SEQ ID NO:70)
> F1D34-KSF86Y-6×His (italic): SEQ ID NO:91


EDCNELPPRRNTEILTGSWSDQTYPEGTQAIYKCRPGYRSLGNIIMVCRKGEWVALNPLRKCQKKSCPNP
GEIRNGQIDVPGGIL**Y**GATISFSCNTGYKLFGSTSSFCLISGSSVQWSDPLPECREIYCPAPPQIDNGIIQGER
DHYGYRQSVTYACNKGFTMIGEHSIYCTVNNDEGEWSGPPPECRGKS*GHHHHHH*

> **F1D34-KS-F107E(SEQ** ID NO:72)
> F1D34-KS-F107E×His (italic): SEQ ID NO:92


EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSS**E**CLISGSSVQWSDPLPECREIYCPAPPQIDNGIIQGER

DHYGYRQSVTYACNKGFTMIGEHSIYCTVNNDEGEWSGPPPECRGKS*GHHHHHH*

> F1D34-KS-L109D:SEQ ID NO:74
> F1D34-KS- L109D×His (italic): SEQ ID NO:93

EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFC**D**ISGSSVQWSDPLPECREIYCPAPPQIDNGIIQGER
DHYGYRQSVTYACNKGFTMIGEHSIYCTVNNDEGEWSGPPPECRGKS*GHHHHHH*

> F1D34-KS-L109E(SEQ ID NO:76)
> F1D34-KS-L109E -6×His (italic): SEQ ID NO:94


EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFC**E**ISGSSVQWSDPLPECREIYCPAPPQIDNGIIQGER
DHYGYRQSVTYACNKGFTMIGEHSIYCTVNNDEGEWSGPPPECRGKS*GHHHHHH*

>F1D34-KS-I110T(SEQ ID NO: 78)
>F1D34-KS-I110T -6×His (italic): SEQ ID NO:90


EDCNELPPRRNTEILTGSWSDQTYPEGTQAIYKCRPGYRSLGNIIMVCRKGEWVALNPLRKCQKKSCPNP
GEIRNGQIDVPGGILFGATISFSCNTGYKLFGSTSSFCL**T**SGSSVQWSDPLPECREIYCPAPPQIDNGIIQGER
DHYGYRQSVTYACNKGFTMIGEHSIYCTVNNDEGEWSGPPPECRG**KS***GHHHHHH*

The 59D, 59S, 82D, 110T and 56E mutations were selected for combination to construct hybrid protein mutants with combination mutations, the sequence of which is as follows:

> F1D34-KS-59D/82D/110T(SEQ ID NO:80)
>F1D KS-59D/82D/110T -659D/8 (italic): SEQ ID NO:95


EDCNELPPRRNTEILTGSWSDQTYPEGTQAIYKCRPGYRSLGNIIMVCRKGEWVALNP**D**RKCQKKSCPNP
GEIRNGQIDVP**D**GILFGATISFSCNTGYKLFGSTSSFCL**T**SGSSVQWSDPLPECREIYCPAPPQIDNGIIQGER
DHYGYRQSVTYACNKGFTMIGEHSIYCTVNNDEGEWSGPPPECRGKS*GHHHHHH*

> F1D34-KS-59S/82D/110T: SEQ ID NO:82
> F1D34-KS-59S/82D/110T -6×His (italic): SEQ ID NO:96


EDCNELPPRRNTEILTGSWSDQTYPEGTQAIYKCRPGYRSLGNIIMVCRKGEWVALNPSRKCQKKSCPNP
GEIRNGQIDVP**D**GILFGATISFSCNTGYKLFGSTSSFCL**T**SGSSVQWSDPLPECREIYCPAPPQIDNGIIQGER
DHYGYRQSVTYACNKGFTMIGEHSIYCTVNNDEGEWSGPPPECRGKS*GHHHHHH*

> F1D34-KS-59D/82D/110T/56E(SEQ ID NO:84)
> F1D34-KS-59D/82D/110T/56E -6×His (italic): SEQ ID NO:54


EDCNELPPRRNTEILTGSWSDQTYPEGTQAIYKCRPGYRSLGNIIMVCRKGEWVA**E**NP**D**RKCQKKSCPNP
GEIRNGQIDVP**D**GILFGATISFSCNTGYKLFGSTSSFCL**T**SGSSVQWSDPLPECREIYCPAPPQIDNGIIQGER
DHYGYRQSVTYACNKGFTMIGEHSIYCTVNNDEGEWSGPPPECRGKS*GHHHHHH*

> F1D34-KS-59S/82D/110T/56E(SEQ ID NO:86)
> F1D34-KS-59S/82D/110T/56E -6×His (italic): SEQ ID NO:53


EDCNELPPRRNTEILTGSWSDQTYPEGTQAIYKCRPGYRSLGNIIMVCRKGEWVA**E**NP**S**RKCQKKSCPNP

GEIRNGQIDVP**D**GILFGATISFSCNTGYKLFGSTSSFCL**T**SGSSVQWSDPLPECREIYCPAPPQIDNGIIQGER
DHYGYRQSVTYACNKGFTMIGEHSIYCTVNNDEGEWSGPPPECRGKS*GHHHHHH*

The hybrid protein mutant molecule is fused with a C5 antibody (the structure of the fusion protein is shown in Figure 9), so as to obtain the hybrid protein-antibody fusion protein, and the heavy chain amino acid sequence of each fusion protein is as follows:

>Ab-F1D34-HC
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK SLSLSLGK***GSG***EDCNELPPRRNTEILTGSWSDOTYPEGTOAIYKCRP GYRSLGNIIMVCRKGEWVALNPLRKCO KKSCPNPGEIRNGOIDVPGGILFGATISFSCNTGYKLFGSTSSFC LISGSSVOWSDPLPECREIYCPAPPOIDNGII OGERDHYGYROSVTYACNKGFTMIGEHSIYCTVNNDEGE WSGPPPECRG (SEQ ID NO:55, wherein the underlined part is the optimized hybrid protein, and its amino acid sequence is SEQ ID NO:60)

**>Ab-F1D34 C'-KS-HC**
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK SLSLSLGK***GSG***EDCNELPPRRNTEILTGSWSDOTYPEGTOAIYKCRP GYRSLGNIIMVCRKGEWVALNPLRKCO KKSCPNPGEIRNGOIDVPGGILFGATISFSCNTGYKLFGSTSSFC LISGSSVOWSDPLPECREIYCPAPPOIDNGII OGERDHYGYROSVTYACNKGFTMIGEHSIYCTVNNDEGE WSGPPPECRG**KS** (SEQ ID NO:61, wherein the underlined part is an optimized hybrid protein, and its amino acid sequence is SEQ ID NO:60)

Then, each fusion protein mutant was constructed based on Ab-F1D34 C'-KS-HC, and the sequence is as follows:

**>Ab-F1D34 L56E-HC**
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK SLSLSLGK***GSG***EDCNELPPRRNTEILTGSWSDOTYPEGTOAIYKCRP GYRSLGNIIMVCRKGEWVA**E**NPLRKCO KKSCPNPGEIRNGOIDVPGGILFGATISFSCNTGYKLFGSTSSFC LISGSSVOWSDPLPECREIYCPAPPOIDNGII OGERDHYGYROSVTYACNKGFTMIGEHSIYCTVNNDEGE WSGPPPECRGKS (SEQ ID NO:63, wherein the underlined part is an optimized hybrid protein, and its amino acid sequence is SEQ ID NO:62)

**>Ab-F1D34 L59D-HC**
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK SLSLSLGK***GSG***EDCNELPPRRNTEILTGSWSDOTYPEGTOAIYKCRP GYRSLGNIIMVCRKGEWVALNP**D**RKCO KKSCPNPGEIRNGOIDVPGGILFGATISFSCNTGYKLFGSTSSFC LISGSSVOWSDPLPECREIYCPAPPOIDNGII OGERDHYGYROSVTYACNKGFTMIGEHSIYCTVNNDEGE WSGPPPECRGKS (SEQ ID NO:65, wherein the underlined part is an optimized hybrid protein, and its amino acid sequence is SEQ ID NO:64)

**>Ab-F1D34 L59S-HC**
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK

SLSLSLGK*GSG*EDCNELPPRRNTEILTGSWSDOTYPEGTOAIYKCRP GYRSLGNIIMVCRKGEWVALNPSRKCO KKSCPNPGEIRNGOIDVPGGILFGATISFSCNTGYKLFGSTSSFC LISGSSVOWSDPLPECREIYCPAPPOIDNGII OGERDHYGYROSVTYACNKGFTMIGEHSIYCTVNNDEGE WSGPPPECRGKS (SEQ ID NO:67, wherein the underlined part is an optimized hybrid protein, and its amino acid sequence is SEQ ID NO:66)

>Ab-F1D34 G82D-HC
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK SLSLSLGK*GSG*EDCNELPPRRNTEILTGSWSDOTYPEGTOAIYKCRP GYRSLGNIIMVCRKGEWVALNPLRKCO KKSCPNPGEIRNGOIDVP**D**GILFGATISFSCNTGYKLFGSTSSFC LISGSSVOWSDPLPECREIYCPAPPOIDNGII OGERDHYGYROSVTYACNKGFTMIGEHSIYCTVNNDEGE WSGPPPECRGKS (SEQ ID NO:69, wherein the underlined part is an optimized hybrid protein, and its amino acid sequence is SEQ ID NO:68)

>Ab-F1D34 F86Y-HC
QVQLVQSGAEVKKPGASVKVSCKASGYTFTNYGMNWVRQAPGQGLEWMGWINTYTGEPTYADDFKG RFTFTL DTSTSTAYLELSSLRSEDTAVYYCARTKVPHYFAYAMDYWGQGTLVTVSSASTKGPSVFPLAPCS RSTSESTAAL GCLVKDYFPEPVTVSWNSGALTSGVHTFPAVLQSSGLYSLSSVVTVPSSSLGTKTYTCNVD HKPSNTKVDKRVE SKYGPPCPPCPAPEFLGGPSVFLFPPKPKDTLMISRTPEVTCVVVDVSQEDPEVQFNW YVDGVEVHNAKTKPR EEQFNSTYRVVSVLTVLHQDWLNGKEYKCKVSNKGLPSSIEKTISKAKGQPREP QVYTLPPSQEEMTKNQVSLT CLVKGFYPSDIAVEWESNGQPENNYKTTPPVLDSDGSFFLYSRLTVDKSR WQEGNVFSCSVLHEALHAHYTQKS LSLSLGK*GSG*EDCNELPPRRNTEILTGSWSDOTYPEGTOAIYKCRP GYRSLGNIIMVCRKGEWVALNPLRKCOK KSCPNPGEIRNGOIDVPGGIL**Y**GATISFSCNTGYKLFGSTSSFC LISGSSVOWSDPLPECREIYCPAPPOIDNGIIO GERDHYGYROSVTYACNKGFTMIGEHSIYCTVNNDEGE WSGPPPECRGKS (SEQ ID NO:71, wherein the underlined part is an optimized hybrid protein, and its amino acid sequence is SEQ ID NO:70)

>Ab-F1D34 F107E-HC
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK SLSLSLGK*GSG*EDCNELPPRRNTEILTGSWSDOTYPEGTOAIYKCRP GYRSLGNIIMVCRKGEWVALNPLRKCO KKSCPNPGEIRNGOIDVPGGILFGATISFSCNTGYKLFGSTSS**E**C LISGSSVOWSDPLPECREIYCPAPPOIDNGII OGERDHYGYROSVTYACNKGFTMIGEHSIYCTVNNDEGE WSGPPPECRGKS (SEQ ID NO:73, wherein the underlined part is an optimized hybrid protein, and its amino acid sequence is SEQ ID NO:72)

>Ab-F1D34 L109D-HC
QVQLUQSGAEVKKPGASVKVSCKASGYTFTNYGMNWVRQAPGQGLEWMGWINTYTGEPTYADDFKG RFTFT LDTSTSTAYLELSSLRSEDTAVYYCARTKVPHYFAYAMDYWGQGTLVTVSSASTKGPSVFPLAPCS RSTSESTAA LGCLVKDYFPEPVTVSWNSGALTSGVHTFPAVLQSSGLYSLSSVVTVPSSSLGTKTYTCNVD HKPSNTKVDKRV ESKYGPPCPPCPAPEFLGGPSVFLFPPKPKDTLMISRTPEVTCVVVDVSQEDPEVQFNW YVDGVEVHNAKTKP REEQFNSTYRVVSVLTVLHQDWLNGKEYKCKVSNKGLPSSIEKTISKAKGQPREP QVYTLPPSQEEMTKNQVSL TCLVKGFYPSDIAVEWESNGQPENNYKTTPPVLDSDGSFFLYSRLTVDKSR WQEGNVFSCSVLHEALHAHYTQK SLSLSLGK*GSG*EDCNELPPRRNTEILTGSWSDOTYPEGTOAIYKCRP GYRSLGNIIMVCRKGEWVALNPLRKCO KKSCPNPGEIRNGOIDVPGGILFGATISFSCNTGYKLFGSTSSFC **D**ISGSSVOWSDPLPECREIYCPAPPOIDNGII OGERDHYGYROSVTYACNKGFTMIGEHSIYCTVNNDEGE WSGPPPECRGKS (SEQ ID NO:75, wherein the underlined part is an optimized hybrid protein, and its amino acid sequence is SEQ ID NO:74)

>Ab-F1D34 L109E-HC
QVQLVQSGAEVKKPGASVKVSCKASGYTFTNYGMNWVRQAPGQGLEWMGWINTYTGEPTYADDFKG RFTFTL DTSTSTAYLELSSLRSEDTAVYYCARTKVPHYFAYAMDYWGQGTLVTVSSASTKGPSVFPLAPCS RSTSESTAAL GCLVKDYFPEPVTVSWNSGALTSGVHTFPAVLQSSGLYSLSSVVTVPSSSLGTKTYTCNVD HKPSNTKVDKRVE SKYGPPCPPCPAPEFLGGPSVFLFPPKPKDTLMISRTPEVTCVVVDVSQEDPEVQFNW YVDGVEVHNAKTKPR

EEQFNSTYRVVSVLTVLHQDWLNGKEYKCKVSNKGLPSSIEKTISKAKGQPREP QVYTLPPSQEEMTKNQVSLT
CLVKGFYPSDIAVEWESNGQPENNYKTTPPVLDSDGSFFLYSRLTVDKSR WQEGNVFSCSVLHEALHAHYTQKS
LSLSLGK*GSG*EDCNELPPRRNTEILTGSWSDOTYPEGTOAIYKCRP GYRSLGNIIMVCRKGEWVALNPLRKCOK
KSCPNPGEIRNGOIDVPGGILFGATISFSCNTGYKLFGSTSSFC EISGSSVOWSDPLPECREIYCPAPPOIDNGIIO
GERDHYGYROSVTYACNKGFTMIGEHSIYCTVNNDEGE WSGPPPECRGKS (SEQ ID NO:77, wherein the underlined part is an optimized hybrid protein, and its amino acid sequence is SEQ ID NO:76)

### >Ab-F1D34 I110T-HC

QVQLUQSGAEVKKPGASVKVSCKASGYTFTNYGMNWVRQAPGQGLEWMGWINTYTGEPTYADDFKG RFTFT
LDTSTSTAYLELSSLRSEDTAVYYCARTKVPHYFAYAMDYWGQGTLVTVSSASTKGPSVFPLAPCS RSTSESTAA
LGCLVKDYFPEPVTVSWNSGALTSGVHTFPAVLQSSGLYSLSSVVTVPSSSLGTKTYTCNVD HKPSNTKVDKRV
ESKYGPPCPPCPAPEFLGGPSVFLFPPKPKDTLMISRTPEVTCVVVDVSQEDPEVQFNW YVDGVEVHNAKTKP
REEQFNSTYRVVSVLTVLHQDWLNGKEYKCKVSNKGLPSSIEKTISKAKGQPREP QVYTLPPSQEEMTKNQVSL
TCLVKGFYPSDIAVEWESNGQPENNYKTTPPVLDSDGSFFLYSRLTVDKSR WQEGNVFSCSVLHEALHAHYTQK
SLSLSLGK*GSG*EDCNELPPRRNTEILTGSWSDOTYPEGTOAIYKCRP GYRSLGNIIMVCRKGEWVALNPLRKCO
KKSCPNPGEIRNGOIDVPGGILFGATISFSCNTGYKLFGSTSSFC LTSGSSVOWSDPLPECREIYCPAPPOIDNGII
OGERDHYGYROSVTYACNKGFTMIGEHSIYCTVNNDEGE WSGPPPECRGKS (SEQ ID NO:79, wherein the underlined part is an optimized hybrid protein, and its amino acid sequence is SEQ ID NO:78)

The combination mutant fusion protein was constructed, and the heavy chain amino acid sequence was as follows:

### >Ab-F1D34 59D/82D/110T-HC

QVQLUQSGAEVKKPGASVKVSCKASGYTFTNYGMNWVRQAPGQGLEWMGWINTYTGEPTYADDFKG RFTFT
LDTSTSTAYLELSSLRSEDTAVYYCARTKVPHYFAYAMDYWGQGTLVTVSSASTKGPSVFPLAPCS RSTSESTAA
LGCLVKDYFPEPVTVSWNSGALTSGVHTFPAVLQSSGLYSLSSVVTVPSSSLGTKTYTCNVD HKPSNTKVDKRV
ESKYGPPCPPCPAPEFLGGPSVFLFPPKPKDTLMISRTPEVTCVVVDVSQEDPEVQFNW YVDGVEVHNAKTKP
REEQFNSTYRVVSVLTVLHQDWLNGKEYKCKVSNKGLPSSIEKTISKAKGQPREP QVYTLPPSQEEMTKNQVSL
TCLVKGFYPSDIAVEWESNGQPENNYKTTPPVLDSDGSFFLYSRLTVDKSR WQEGNVFSCSVLHEALHAHYTQK
SLSLSLGK*GSG*EDCNELPPRRNTEILTGSWSDOTYPEGTOAIYKCRP GYRSLGNIIMVCRKGEWVALNP**D**RKCO
KKSCPNPGEIRNGOIDVP**D**GILFGATISFSCNTGYKLFGSTSSFC L**T**SGSSVOWSDPLPECREIYCPAPPOIDNGII
OGERDHYGYROSVTYACNKGFTMIGEHSIYCTVNNDEGE WSGPPPECRGKS (SEQ ID NO:81, wherein the underlined part is an optimized hybrid protein, and its amino acid sequence is SEQ ID NO:80)

### >Ab-F1D34 59S/82D/110T-HC

QVQLVQSGAEVKKPGASVKVSCKASGYTFTNYGMNWVRQAPGQGLEWMGWINTYTGEPTYADDFKG RFTFTL
DTSTSTAYLELSSLRSEDTAVYYCARTKVPHYFAYAMDYWGQGTLVTVSSASTKGPSVFPLAPCS RSTSESTAAL
GCLVKDYFPEPVTVSWNSGALTSGVHTFPAVLQSSGLYSLSSVVTVPSSSLGTKTYTCNVD HKPSNTKVDKRVE
SKYGPPCPPCPAPEFLGGPSVFLFPPKPKDTLMISRTPEVTCVVVDVSQEDPEVQFNW YVDGVEVHNAKTKPR
EEQFNSTYRVVSVLTVLHQDWLNGKEYKCKVSNKGLPSSIEKTISKAKGQPREP QVYTLPPSQEEMTKNQVSLT
CLVKGFYPSDIAVEWESNGQPENNYKTTPPVLDSDGSFFLYSRLTVDKSR WQEGNVFSCSVLHEALHAHYTQKS
LSLSLGK*GSG*EDCNELPPRRNTEILTGSWSDOTYPEGTOAIYKCRP GYRSLGNIIMVCRKGEWVALNPSRKCOK
KSCPNPGEIRNGOIDVP**D**GILFGATISFSCNTGYKLFGSTSSFC L**T**SGSSVOWSDPLPECREIYCPAPPOIDNGIIO
GERDHYGYROSVTYACNKGFTMIGEHSIYCTVNNDEGE WSGPPPECRGKS (SEQ ID NO:83, wherein the underlined part is an optimized hybrid protein, and its amino acid sequence is SEQ ID NO:82)

### >Ab-F1D34 59D/82D/110T/56E-HC

QVQLUQSGAEVKKPGASVKVSCKASGYTFTNYGMNWVRQAPGQGLEWMGWINTYTGEPTYADDFKG RFTFT
LDTSTSTAYLELSSLRSEDTAVYYCARTKVPHYFAYAMDYWGQGTLVTVSSASTKGPSVFPLAPCS RSTSESTAA
LGCLVKDYFPEPVTVSWNSGALTSGVHTFPAVLQSSGLYSLSSVVTVPSSSLGTKTYTCNVD HKPSNTKVDKRV
ESKYGPPCPPCPAPEFLGGPSVFLFPPKPKDTLMISRTPEVTCVVVDVSQEDPEVQFNW YVDGVEVHNAKTKP
REEQFNSTYRVVSVLTVLHQDWLNGKEYKCKVSNKGLPSSIEKTISKAKGQPREP QVYTLPPSQEEMTKNQVSL
TCLVKGFYPSDIAVEWESNGQPENNYKTTPPVLDSDGSFFLYSRLTVDKSR WQEGNVFSCSVLHEALHAHYTQK
SLSLSLGK*GSG*EDCNELPPRRNTEILTGSWSDOTYPEGTOAIYKCRP GYRSLGNIIMVCRKGEWVA**E**NP**D**RKCO
KKSCPNPGEIRNGOIDVP**D**GILFGATISFSCNTGYKLFGSTSSFC L**T**SGSSVOWSDPLPECREIYCPAPPOIDNGII
OGERDHYGYROSVTYACNKGFTMIGEHSIYCTVNNDEGE WSGPPPECRGKS (SEQ ID NO:85, wherein the underlined part is an optimized hybrid protein, and its amino acid sequence is SEQ ID NO:84)

>Ab-F1D34 59S/82D/110T/56E-HC
QVQLUQSGAEVKKPGASVKVSCKASGYTFTNYGMNWVRQAPGQGLEWMGWINTYTGEPTYADDFKG RFTFT
LDTSTSTAYLELSSLRSEDTAVYYCARTKVPHYFAYAMDYWGQGTLVTVSSASTKGPSVFPLAPCS RSTSESTAA
LGCLVKDYFPEPVTVSWNSGALTSGVHTFPAVLQSSGLYSLSSVVTVPSSSLGTKTYTCNVD HKPSNTKVDKRV
ESKYGPPCPPCPAPEFLGGPSVFLFPPKPKDTLMISRTPEVTCVVVDVSQEDPEVQFNW YVDGVEVHNAKTKP
REEQFNSTYRVVSVLTVLHQDWLNGKEYKCKVSNKGLPSSIEKTISKAKGQPREP QVYTLPPSQEEMTKNQVSL
TCLVKGFYPSDIAVEWESNGQPENNYKTTPPVLDSDGSFFLYSRLTVDKSR WQEGNVFSCSVLHEALHAHYTQK
SLSLSLGK*GSG*EDCNELPPRRNTEILTGSWSDOTYPEGTOAIYKCRP GYRSLGNIIMVCRKGEWVA**E**NPSRKCO
KKSCPNPGEIRNGOIDVP**D**GILFGATISFSCNTGYKLFGSTSSFC LTSGSSVOWSDPLPECREIYCPAPPOIDNGII
OGERDHYGYROSVTYACNKGFTMIGEHSIYCTVNNDEGE WSGPPPECRGKS (SEQ ID NO:87, wherein the underlined part is an optimized hybrid protein, and its amino acid sequence is SEQ ID NO:86)

The hybrid protein mutant was constructed in the manner of Examples 1 and 2 and the protein was expressed and purified. Wherein the construct is constructed as a signal peptide (SEQ ID NO: 17) + hybrid protein mutant +6xHis tag (SEQ ID NO: 16).

For the fusion protein, the gene encoding the fusion protein is synthesized by GENEWIZ and Nanjing Tsingke Biotechnology Co., Ltd. For secretory expression, a signal peptide was added to the N-terminus of the protein. The signal peptide sequence was: MGWSCIILFLVATATGVHS (SEQ ID NO: 17). The gene was cloned into a eukaryotic expression vector (pCDNA3.1, purchased from Miaoling Biology) using standard molecular biology techniques. The mutants were constructed using Overlap PCR, and the constructed plasmids were prepared using a plasmid midi kit (NucleoBond Xtra Midi Plus, MACHEREY-NAGEL) for protein expression after sequencing verification.

### Example 7. Fusion protein analysis

### 7.1 Fusion protein expression, purification and rough solubility assessment

Fusion protein expression used Expi293 (purchased from Thermo fisher) cells for transient transfection and expression. The prepared heavy chain and light chain plasmids at a ratio of 1:1.5 are co-transfected into Expi293 cells using PEI-MW40000 (PolySciences) to transiently express the required protein. The PEI preparation and usage methods can be referred to PolySciences PEI MAX Instructions for Use. For Expi293 cell culture and transfection procedures, please refer to the Thermofisher Expi293 Expression System Instructions for Use. The cell supernatant was harvested by centrifugation 5-7 days after transient transfection. The supernatant was filtered through a 0.45 $\mu$m filter for protein purification.

The transiently expressed protein was purified using a protein A prepacked column (GE Lifesciences). For the usage procedure, please refer to the manufacturer's instructions.

The harvested eluted protein sample is neutralized by Tris buffer solution and then placed in a refrigerator at 4 °C, the precipitation amount and the turbidity of the solution are visually observed after 24 hours, and the solubility was roughly assessed on a scale of 1 to 10, with 10 for the most precipitation or the most turbid, and 1 for the least precipitation or the clearest. Upon rough assessment of solubility, the scores for each fusion protein are given in the following table (Table 6), and based on the observed solubility, taking the C'-KS mutant fusion protein as the benchmark, each mutation improved the solubility of the fusion protein to varying degrees.

Table 6 Scores for rough solubility assessment of fusion protein

| Mutation | Turbidity/precipitation amount of fusion protein |
|----------|--------------------------------------------------|
| C'-KS | 10 |
| L56E | 5 |
| L59D | 3 |
| L59S | 4 |
| G82D | 5 |
| F86Y | 5 |
| F107E | 4 |
| L109D | 9 |
| L109E | 5 |

(continued)

| Mutation | Turbidity/precipitation amount of fusion protein |
|----------|--------------------------------------------------|
| I110T | 8 |

After being centrifuged or filtered to remove precipitations, the solution is dialyzed or ultrafiltrated and exchanged into the PBS buffer system, and finally filtered and sterilized through a 0.22 μm filter to obtain the protein sample to be tested. Protein samples were quantified using a NanoDrop™ spectrophotometer (Thermo Scientific) in combination with the theoretical extinction coefficient of the protein.

7.2 Analysis of fusion protein purity

Fusion protein purity was analyzed using size exclusion chromatography (SEC) by applying purified samples in PBS to a TSKgel SuperSW3000 300 x 4.6 mm, 5 μm column (TOSOH). SEC was performed using a model U3000 HPLC instrument (DIONEX). All proteins were detected using UV detection at wavelengths of 280 nm and 214 nm. Elution was isocratic at a flow rate of 0.25 mL/min. The analysis results show that the monomer purity of the fusion protein purified in one step using Protein A is good in the solution state (Table 7), indicating the physical homogenecity and stability of the fusion protein in the solution state as a monon protein.

Table 7. Fusion protein purity (SEC-HPLC)

| Fusion protein | Main peak% |
|----------------|------------|
| Ab-F1D34 | 90.2 |
| C'-KS | 98.2 |
| L56E | 97.8 |
| L59D | 97.5 |
| L59S | 98.2 |
| G82D | 97.5 |
| F86Y | 97.8 |
| F107E | 97.5 |
| L109D | 98.2 |
| L109E | 94.4 |
| I110T | 98.3 |
| 59D/82D/110T | 96.7 |
| 59S/82D/110T | 94.5 |
| 59D/82D/110T/56E | 97.1 |
| 59S/82D/110T/56E | 95 |

**7.3 Solubility assessment**

The clarified fusion protein solution sample that was exchanged into PBS was concentrated by centrifugation using an ultrafiltration tube (Amicon® Ultra 30 Kda) at 4000 g at 10°C. The sample was mixed after each 15-min centrifugation and then centrifuged again until precipitation appeared or the sample solution volume did not change significantly. The concentration of the protein samples was measured using a NanoDrop™ spectrophotometer (Thermo Scientific) in combination with the theoretical extinction coefficient of the protein. The concentration of each concentrated combination mutant fusion proteins is shown in Table 8 below. According to the measurement results, the concentration of the concentrated fusion protein containing the 59D mutation is higher than that of the concentrated fusion protein containing the 59S mutation, and the concentration of the concentrated fusion protein containing four mutations is higher than that of the concentrated fusion protein containing three mutations, wherein the concentrated fusion protein containing the 59D/82D/110T/56E mutations has the highest concentration, about 140 mg/Ml, indicating its better solubility.

Table 8. Concentration of concentrated fusion protein

| Fusion protein | Concentration after concentration mg/mL |
|---|---|
| 59D/82D/110T | 107.088 |
| 59S/82D/110T | 66.355 |
| 59D/82D/110T/56E | 139.578 |
| 59S/82D/110T/56E | 78.382 |

**Example 8 Hemolytic inhibitory Activity of fusion protein**

**8.1 Alternative pathway (AP) hemolytic inhibitory activity**

The detection method of fusion protein complement AP inhibitory activity is the same as 4.2.

**AP Activity of fusion protein with single-point mutation**

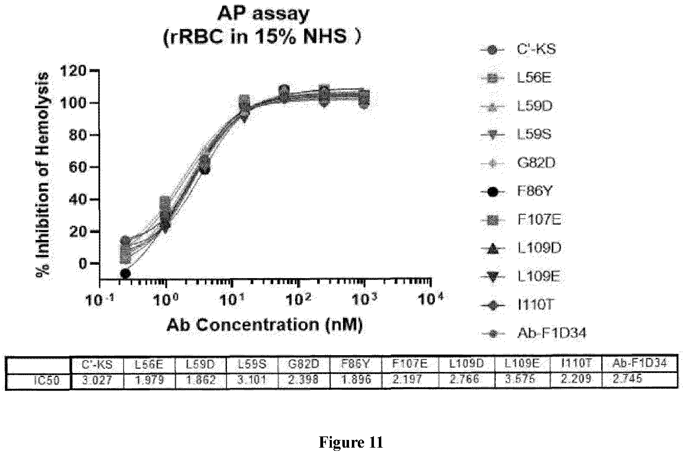

To test the AP inhibitory activity of the fusion protein with single-point mutation, 4-fold gradient dilution was performed from 1000 nM of final protein concentration, with a total of 7 concentration points, and the final serum concentration was 15%. The test results showed that the activity of each point mutant fusion protein in inhibiting AP hemolysis was substantially the same, and there was no significant difference with the fusion protein Ab-F1D34 before mutation, indicating that each mutation did not affect the AP inhibitory activity. The results are shown in Figure 11 (the hybrid protein mutations were used to listed in the figure to represent the fusion protein with the antibody fused with it).

**AP Activity of fusion protein with combination mutation**

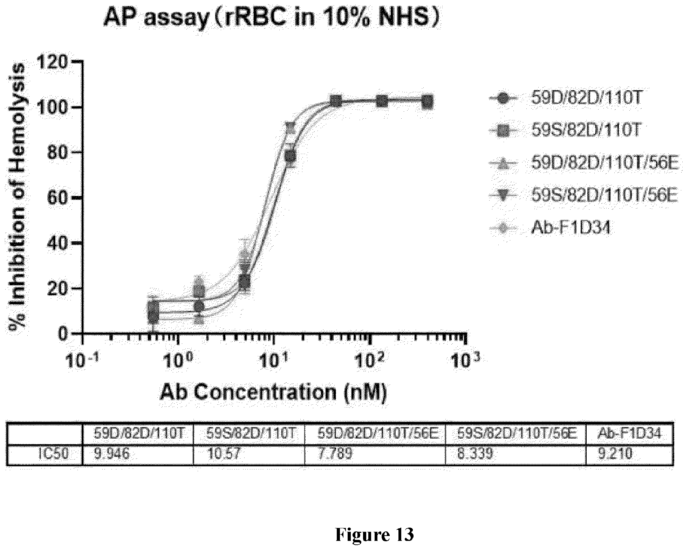

To test the AP inhibitory activity of the fusion protein with combination mutations, 3-fold gradient dilution was performed from 400 nM of final protein concentration or 3-fold gradient dilution was performed from 300 nM or 900 nM of final protein concentration, with a total of 7 concentration points, and the final serum concentration was 10%. The test results showed that the activity of each combination mutant fusion protein in inhibiting AP hemolysis was substantially the same, and there was no significant difference with the fusion protein Ab-F1D34 before mutation, indicating that each combination mutation did not affect the AP inhibitory activity. The results are shown in Figure 13A (the hybrid protein mutations were used to listed in the figure to represent the fusion protein with the antibody fused with it).

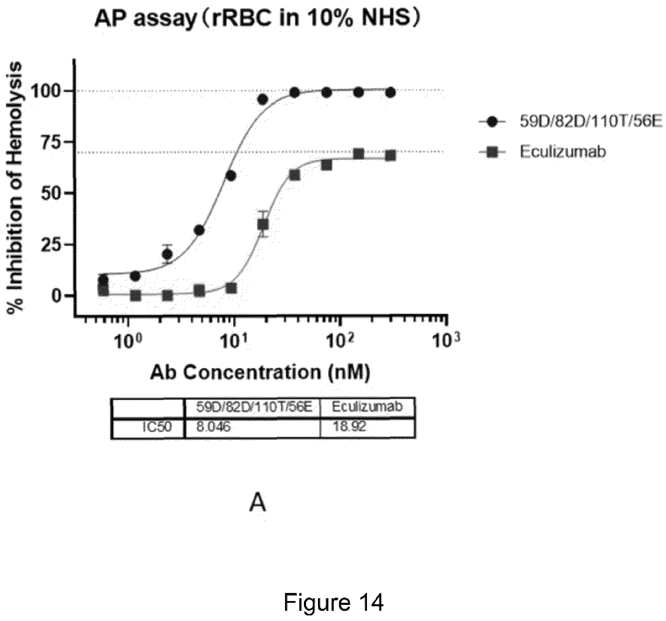

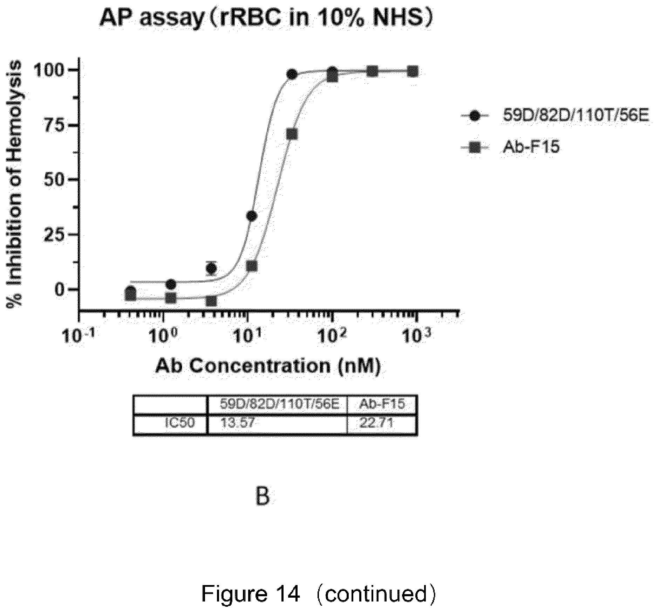

Compared with the Eculizumab monoclonal antibody in the prior art, the fusion protein with 59D/82D/110T/56E combination mutation has better AP inhibitory activity, and overcomes the problem that the Eculizumab monoclonal antibody cannot completely inhibit AP hemolysis (as shown in Figure 14A). In addition, compared with the C5 antibody-CFH CCP1-5 fusion protein Ab-F15, AP inhibitory activity of the fusion protein with the 59D/82D/110T/56E combination mutation was also slightly better (as shown in Figure 14B) (59D/82D/110T/56E in Figure 14 represents the fusion protein of the hybrid protein mutant having 59D/82D/110T/56E and the antibody).

**8.2 Classical pathway (CP) hemolytic inhibitory activity**

The detection method of fusion protein complement AP inhibitory activity is the same as 4.1.

**CP Activity of fusion protein with single-point mutation**

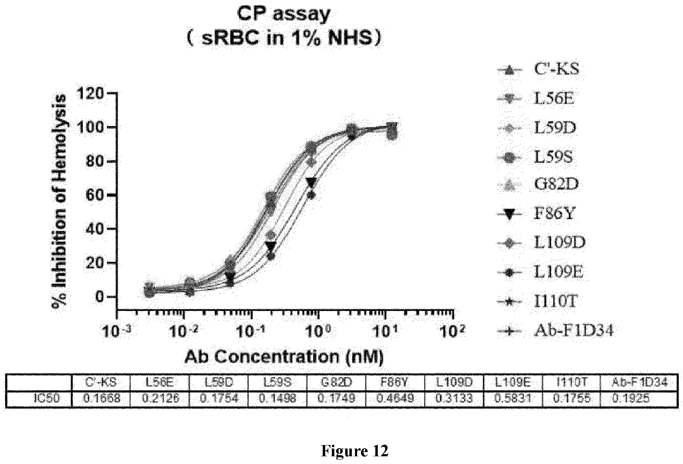

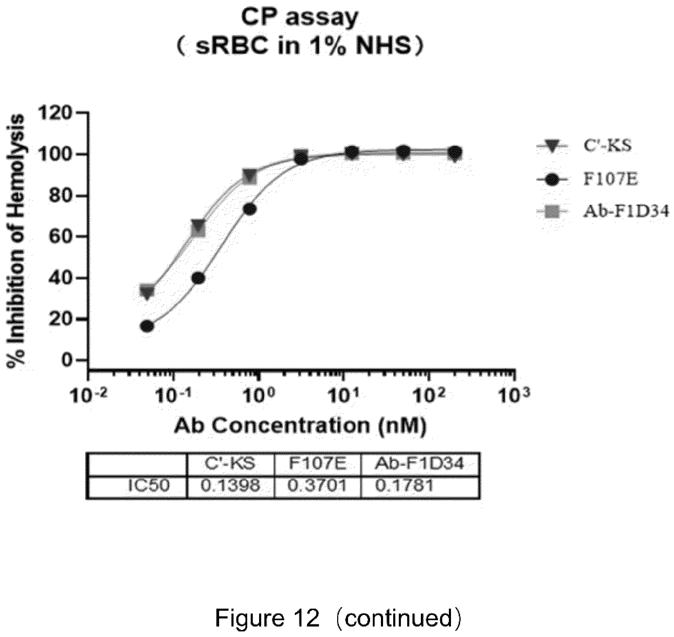

To test the CP inhibitory activity of the fusion protein with single-point mutation, 4-fold gradient dilution was performed from 12.5 or 200 nM of final protein concentration, with a total of 7 concentration points, and the final serum concentration was 1%. The test results showed that the activity of each point mutant fusion protein for inhibiting CP hemolysis was different, wherein fustion protein with C' -KS, L56E, L59D, L59S, G82D and I110T mutations have no significant difference from Ab-F1D34 fusion protein before mutation, but fustion protein with F86Y, L109D, L109E and F107E mutations were slightly decreased, indicating that different mutations had different effects on CP inhibition activity, and the results are shown in Figure 12 (the hybrid protein mutations were used to listed in the figure to represent the fusion protein with the antibody fused with it).

**CP Activity of fusion protein with combination mutation**

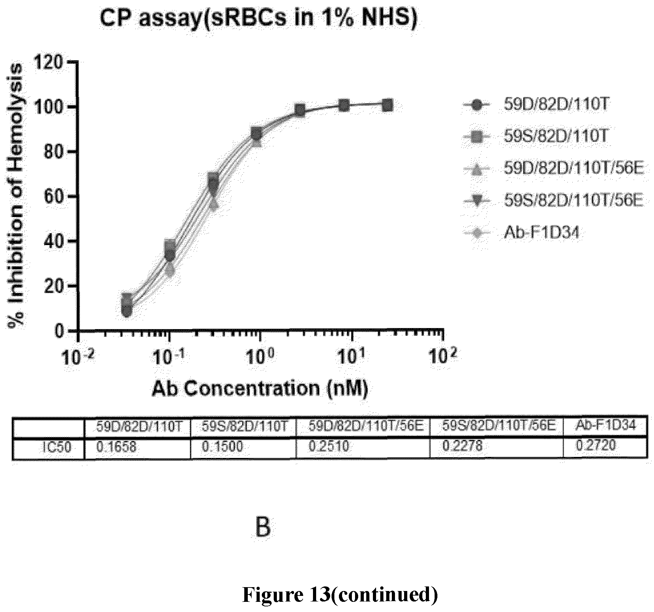

To test the CP inhibitory activity of the fusion protein with combination mutation, 3-fold gradient dilution was performed from 25 or 150 nM of final protein concentration, with a total of 7 or 8 concentration points, and the final serum concentration was 1% or 5%. The test results showed that the activity of each combination mutant fusion protein in inhibiting CP hemolysis was substantially the same, and there was no significant difference with the fusion protein Ab-F1D34 before mutation, indicating that each combination mutation did not affect the CP inhibitory activity. The results are shown in Figure 13B (the hybrid protein mutations were used to listed in the figure to represent the fusion protein with the antibody fused with it).

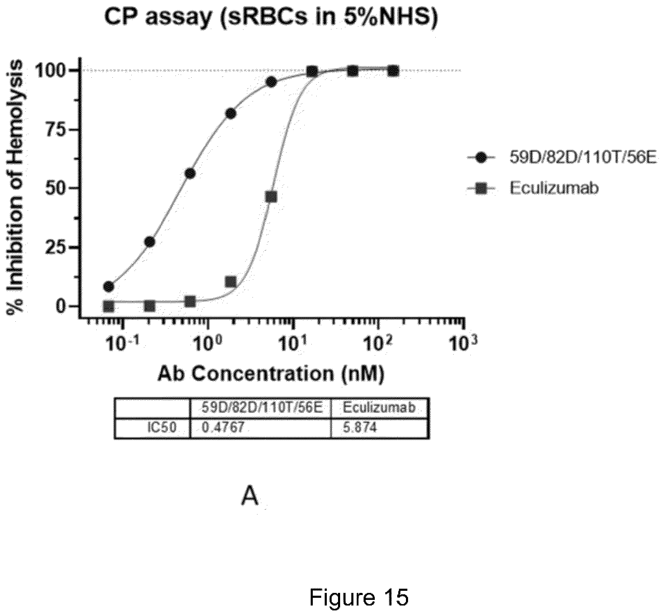

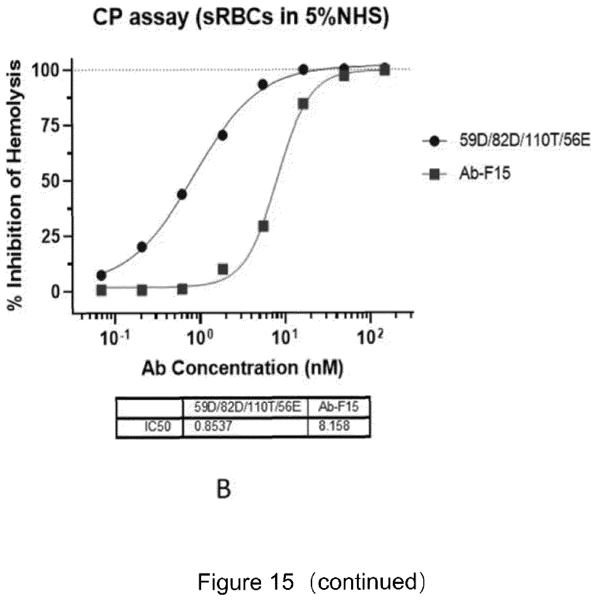

Compared with the Eculizumab monoclonal antibody in the prior art, the fusion protein with the 59D/82D/110T/56E combination mutation has better CP hemolysis inhibitory activity for sheep red blood cell (sRBC), and the IC50 of the monoclonal antibody and the fusion protein are 5.874 nM and 0.4767 nM respectively (Figure 15A). In addition, compared with the C5 antibody-CFH CCP1-5 fusion protein Ab-F15, CP hemolysis inhibitory activity for sheep red blood cell (sRBC) of the fusion protein with the 59D/82D/110T/56E combination mutation was also significantly stronger, and the IC50 of the two fusion proteins are 8.158 nM and 0.8537 nM respectively (Figure 15B).

**Example 9 C3b deposition inhibitory activity**

9.1 Rabbit erythrocyte method

The experimental procedure was the same as 4.3, except that 3-fold gradient dilution was performed from 900 nM of protein concentration, with a total of 7 gradients, C6 or C7-depleted human serum (C6 or C7-depleted NHS, CompTech) was used for incubation to prevent the erythrocytes with C3b deposition from rupturing due to lysis by MAC (membrane attack complex) and thus being unable to be accurately detected. The incubated erythrocytes were stained with Anti-human C3b APC (BioLegend) and detected using a flow cytometer (Beckman Cytoflex). The final data were calculated using the MFI (Median Fluorescence Intensity) value of the erythrocyte group without complement inhibitory protein as 100%, and the inhibitory activity of each complement inhibitory protein in inhibiting C3b deposition on the erythrocyte surface was compared.

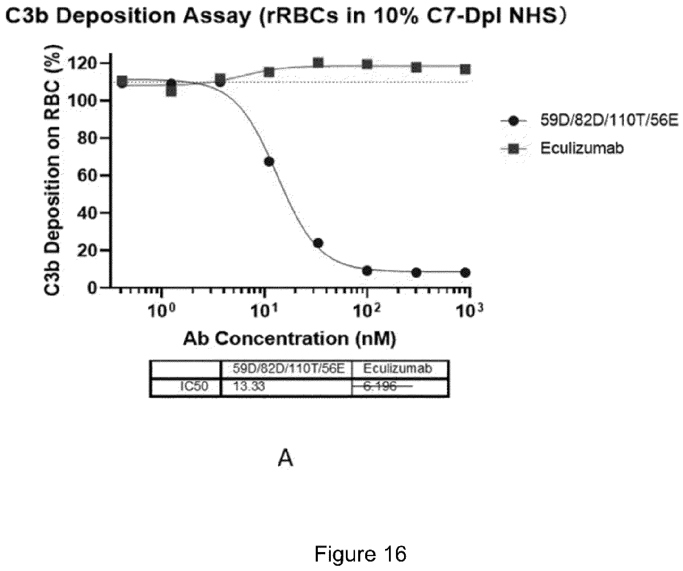

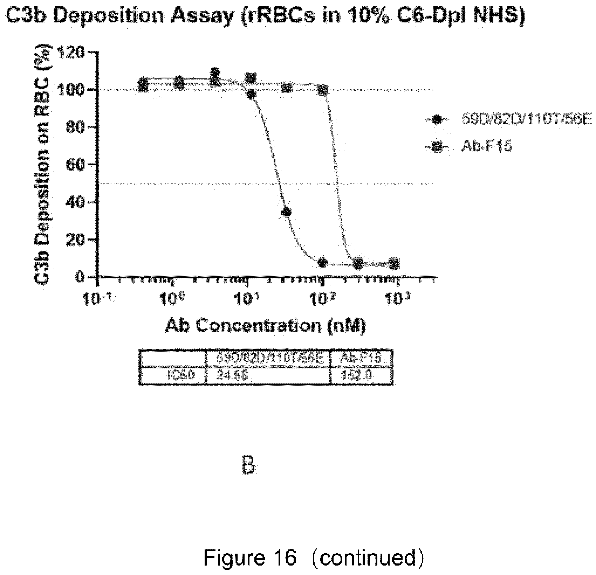

The detection result shows that the Eculizumab monoclonal antibody has no C3b deposition inhibitory activity at all, the fusion protein with the 59D/82D/110T/56E combination mutation has good C3b deposition inhibitory activity (Figure 16A), and the activity is also obviously better than that of C5 antibody-CFH CCP1-5 fusion protein Ab-F15, and IC50 of the two fusion proteins are 24.58 nM and 152 nM respectively (Figure 16B).

9.2 Wieslab method

WIESLAB® Complement System Alternative Pathway kit (Svar, COMPLAP330RUO) was used to replace the final C5b-9 (MAC) detection antibody with a C3b antibody (prepared in a similar manner to the C5 antibody, see SEQ ID NO: 1 and 5 in US 2012/0128674 A1 for the sequence) to detect the deposition of C3b generated after activation of the complement system at the bottom of the microplate, and the impact of adding antibodies or fusion proteins on the production of C3b or deposition of C3b on the bottom surface of the plate.

The detection process is briefly described as follows: the buffer provided by the kit was used to dilute normal human serum (NHS, Shanghai Schbio Co., Ltd.) and the protein sample to be tested. 50 mL of the diluted NHS and protein sample to be tested (59D/82D/110T/56E fusion protein, Eculizumab and Ab-F15) was taken and mixed well and added to the microplate provided in the kit. The final concentration of serum was 5%, and the final concentration of the protein sample to be tested was 900, 300, 100, 33.33, 11.11 and 3.7nM, while setting Blank, PC and NC as instructed. After incubating at 37°C for 1 hour, the mixture was washed with wash solution in the kit three times, added with mouse C3b antibody at a final concentration of 1 $\mu$g/Ml, incubated at 37°C for 1 hour, washed with wash solution three times, and then added with Goat Anti-Mouse IgG-Fc Secondary Antibody( HRP) (1:2000 dilution, SinoBiological, SSA006), washed with wash solution 3 times, and added with substrate to develop color and terminate. OD405nm absorbance value was detected with a microplate reader, and the inhibition rate was calculated:

$$\%\text{complement inhibition}:[1-(\text{Sample}-NC)/(PC-NC)]\times100$$

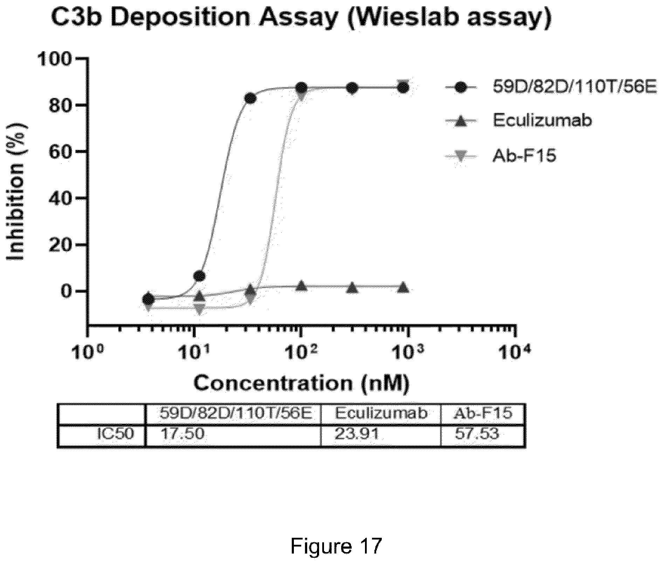

The experimental results show that the fusion protein 59D/82D/110T/56E containing the hybrid protein mutant has stronger activity, which is obviously better than the fusion protein Ab-F15 containing CFH CCP1-5, whereas Eculizumab has no C3b deposition inhibitory activity (as shown in Figure 17), which is substantially consistent with the results of the assay with rabbit erythrocyte method, and further verifies the advantage of the fusion protein containing the hybrid protein in the activity of inhibiting C3b deposition.

**Examples 10 Biotin pathway (AP) hemolytic inhibitory activity**

Wieslab method

The WIESLAB® Complement System Alternative Pathway Kit (Svar, COMPLAP330RUO) was used for detection, and the detailed method is shown in the product manual.

The detection process is briefly described as follows: the buffer provided by the kit was used to dilute normal human serum (PC, included in the kit) and the protein sample to be tested. 50 mL of the diluted NHS and protein sample to be tested (59D/82D/110T/56E, Eculizumab and Ab-F15) was taken and mixed well and added to the microplate provided in the kit. The final concentration of serum was 1%, and the final concentration of the protein sample to be tested was 40, 20, 10, 5, 2.5, 1.25, 0.625, 0.3125 and 0.15625 nM, while setting Blank, PC and NC as instructed. After incubating at 37°C for 1 hour, the mixture was washed with wash solution in the kit three times, added with the secondary antibody included in the kit, incubated at 37°C for 1 hour, washed with wash solution 3 times, and added with substrate to develop color and terminate. OD405nm absorbance value was detected with a microplate reader, and the inhibition rate was calculated:

$$\%\text{complement inhibition:}[1\text{-}(\text{Sample-NC})/(\text{PC-NC})]\text{x}100$$

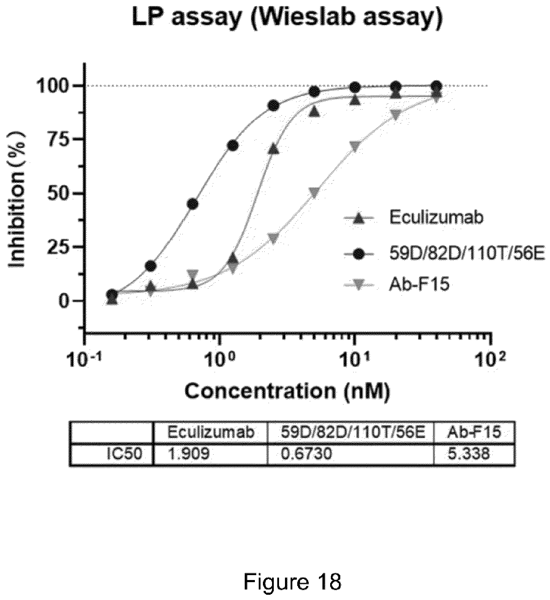

The experimental results show that the fusion protein 59D/82D/110T/56E containing the hybrid protein mutant has stronger LP inhibitory activity, which is obviously better than Eculizumab and the fusion protein Ab-F15 containing CFH CCP1-5 (as shown in Figure 18).

**Example 11. Purity analysis of hybrid protein mutant**

Protein purity analysis was performed using Size Exclusion Chromatography (SEC). The purified protein sample in PBS from the same batch expressed and purified using the same conditions was applied to a TSKgel Super SW3000 column (300×4.6 mm, 5 μm; TOSOH). The SEC was performed using a U3000 HPLC instrument (DIONEX). The elution was carried out isocratically at a flow rate of 0.25 mL/min. All proteins were detected using UV detection at wavelengths of 280 nm and 214 nm. Component analysis was performed using the instrument's built-in software.

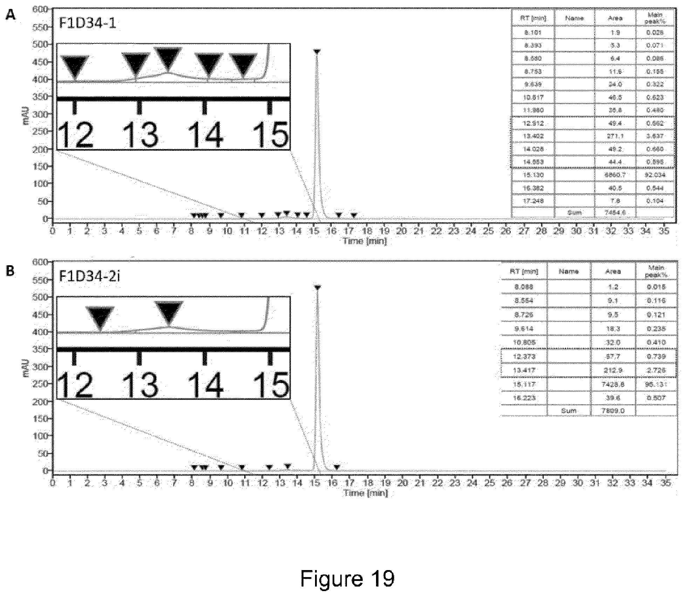

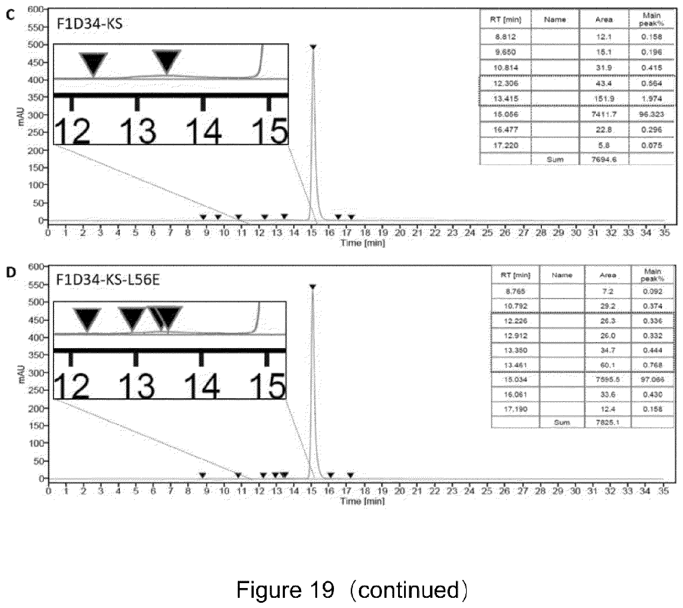

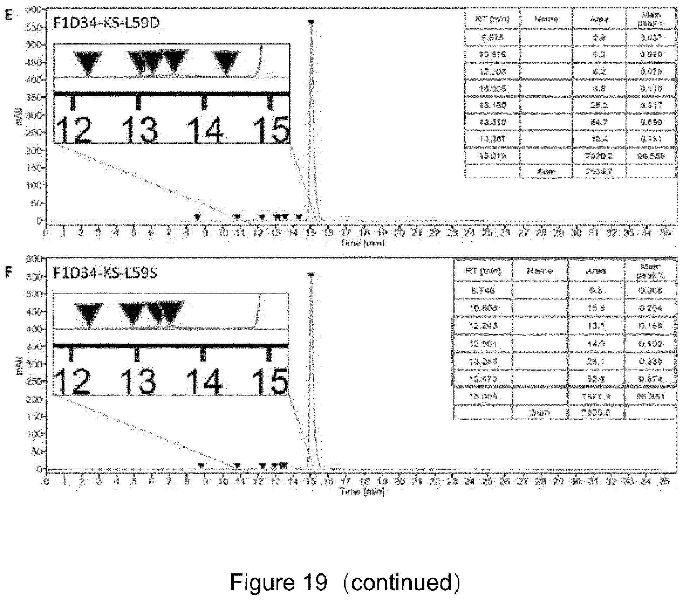

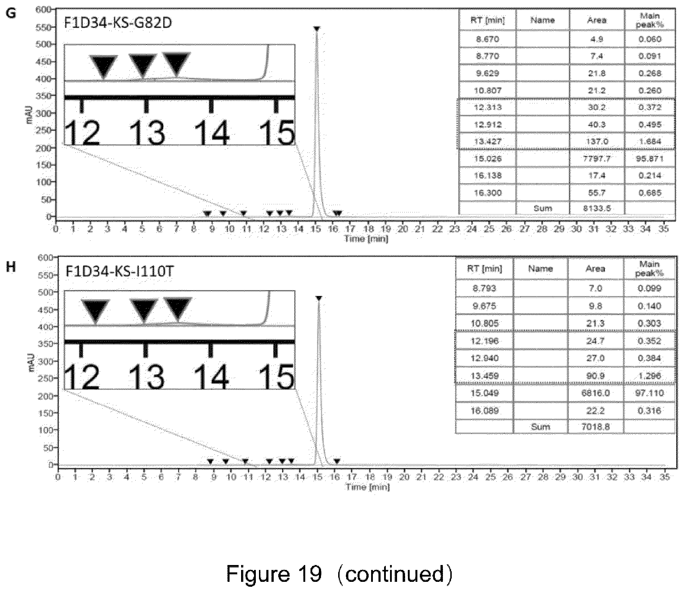

The analysis results showed that the monomer purity of the hybrid proteins F1D34-1 and F1D34-2i in solution state reached 92% and 95%, respectively (Figure 19 A/B), indicating good monomer purity. On the other hand, however, it was observed that both of them had distinct aggregates at a position around the time of peak appearance of 13.4 (RT/min), which was their main aggregate form. The proportion of FID34-1 aggregates exceeded 5% (Figure 19A), while the proportion of F1D34-2i aggregates also exceeded 3% (Figure 19B).

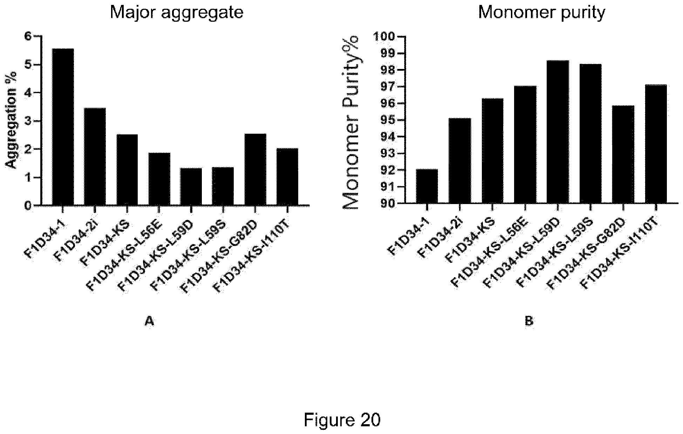

To compare the improvement in aggregate for each of the hybrid protein mutants, the aggregate proportion near the time of peak appearance of the main aggregate peak (12-15 RT/min) was statistically summarized (Figure 19 A-H). The results showed that adding KS to the C' terminus of the hybrid protein F1D34-2i resulted in F1D34-KS which significantly reduce the formation of major aggregates (reduced by 3 percentage points relative to F1D34-1, 2.5% vs 5.6%; reduced by about 1 percentage point relative to F1D34-2i, 2.5% vs 3.5%), while other mutations on the basis of KS addition also improved the formation of major aggregates to varying degrees, Among them, L59D and L59S mutations showed the most obvious improvement, reducing F1D34-KS-L59D and F1D34-KS 59S aggregates to about 1.3%. Further, the aggregates of F1D34-KS-L56E and F1D34-KS-I110T obtained by L56E and I110T mutation reduced by 0.7% and 0.5% respectively compared to the aggregates of F1D34-KS with KS mutation; the aggregates of F1D34-KS-G82D with G82D mutation were substantially consistent with the aggregates of F1D34-KS with KS mutation, 2.55% vs 2.54%, indicating a failure to further reduce formation of major aggregate on the basis of KS (Figure 20A). Accordingly, the monomer purity of each mutant was also improved to various degrees (Figure 20B), which is also consistent with the improved results of the aggregates.

**Example 12. Comparison of expression amount of mutant proteins**

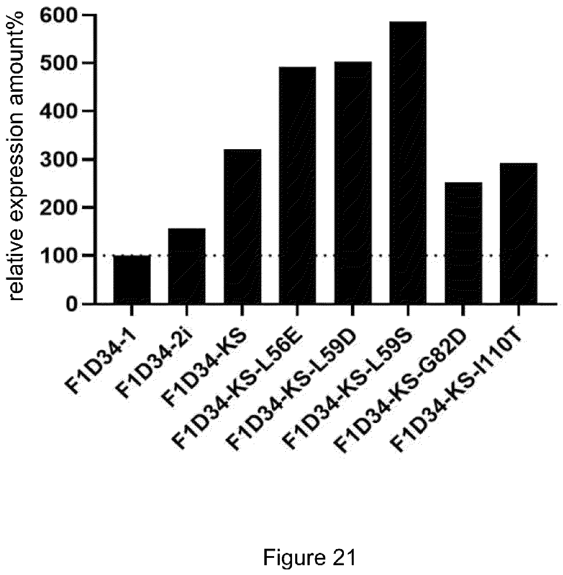

To evaluate the effect of mutation on the protein expression level, transient transfection expression amount of each mutant expressed and purified in the same batch under the same conditions were compared with each other with reference to the expression amount of F1D34-1 (100%), and the expression method and the quantitative method were referred to the method of Example 2. The result is shown in Figure 21, the expression level of F 1D34-2i is about 50% higher than that of F1D34-1, F1D34-KS obtained by adding amino acid KS to the C'-terminus of hybrid protein F1D34-2i improves the expression level by about 1-fold. F1D34-KS-L56E, F1D34-KS-L59D or F1D34-KS-L59S with His tag obtained by mutation of L56E, L59D or L59S respectively on the basis of addition of KS further improves the expression level, and F1D34-KS-G82D or F1D34-KS-1110T obtained by mutation of G82D or I110T fails to further improve the expression level on the basis of F1D34-KS obtained by KS mutation.

**Example 13. Comparison of complement inhibitory activity of mutant proteins**

13.1 Detection of inhibitory activity on Classical Pathway of complement (CP)

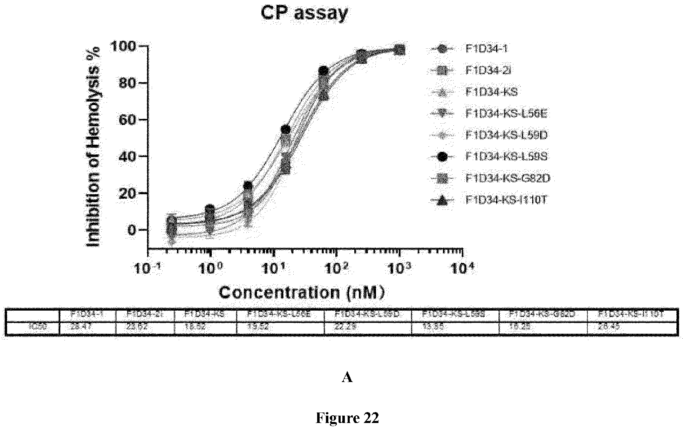

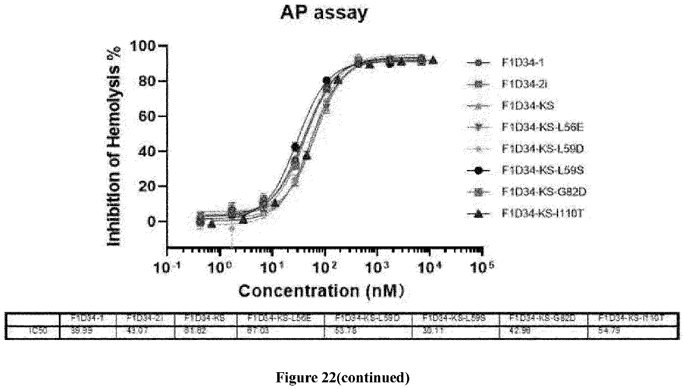

To evaluate the effect of the mutations on the protein CP inhibitory activity, each mutant was detected for its complement CP inhibitory activity in the same experimental procedure as 4.1, with a final serum concentration of 5% and 4-fold dilution was performed from 1000 nM of final hybrid protein concentration. The results of the activity detection are shown in Figure 22A. Compared with hybrid proteins F1D34-1 and F1D34-2i, each mutant did not significantly cause a decrease in CP inhibitory activity.

13.2 Detection of inhibitory activity on Alternative Pathway of complement (AP)

To evaluate the effect of the mutations on the protein AP inhibitory activity, each mutant was detected for its complement AP inhibitory activity in the same experimental procedure as 4.2, with a final serum concentration of 15% and 4-fold dilution was performed from 7000 nM of final hybrid protein concentration (from 11534 nM of final F1D34-KS-I110T concentration). The results of the activity detection are shown in Figure 22A. Compared with hybrid proteins FID34-1 and F1D34-2i, each mutant did not significantly cause a decrease in CP inhibitory activity.

Sequence list

| SE Q ID NO: 1 | MTVARPSVPAALPLLGELPRLLLLVLLCLPAVWGDCGLPPDVPNAQPALEGRTSFPEDTVITYKCEESFVKIPGEKDSV ICLKGSQWSDIEEFCNRSCEVPTRLNSASLKQPYITQNYFPVGTVVEYECRPGYRREPSLSPKLTCLQNLKWSTAVEFC KKKSCPNPGEIRNGQIDVPGGILFGATISFSCNTGYKLFGSTSSFCLISGSSVQWSDPLPECREIYCPAPPQIDNGIIQGER DHYGYRQSVTYACNKGFTMIGEHSIYCTVNNDEGEWSGPPPECRGKSLTSKVPPTVQKPTTVNVPTTEVSPTSQKTT TKTTTPNAQATRSTPVSRTTKHFHETTPNKGSGTTSGTTRLLSGHTCFTLTGLLGTLVTMGLLT |
|---|---|
| SE Q ID NO: 2 | ATGACCGTCGCGCGGCCGAGCGTGCCCGCGGCGCTGCCCCTCCTCGGGGAGCTGCCCCGGCTGCTGCTGCTGGT GCTGTTGTGCCTGCCGGCCGTGTGGGGTGACTGTGGCCTTCCCCCAGATGTACCTAATGCCCAGCCAGCTTTGGA AGGCCGTACAAGTTTTCCCGAGGATACTGTAATAACGTACAAATGTGAAGAAAGCTTTGTGAAAATTCCTGGCGA GAAGGACTCAGTGATCTGCCTTAAGGGCAGTCAATGGTCAGATATTGAAGAGTTCTGCAATCGTAGCTGCGAGGT GCCAACAAGGCTAAATTCTGCATCCCTCAAACAGCCTTATATCACTCAGAATTATTTTCCAGTCGGTACTGTTGTG GAATATGAGTGCCGTCCAGGTTACAGAAGAGAACCTTCTCTATCACCAAAACTAACTTGCCTTCAGAATTTAAAA TGGTCCACAGCAGTCGAATTTTGTAAAAAGAAATCATGCCCTAATCCGGGAGAAATACGAAATGGTCAGATTGAT GTACCAGGTGGCATATTATTTGGTGCAACCATCTCCTTCTCATGTAACACAGGGTACAAATTATTTGGCTCGACTT CTAGTTTTTGTCTTATTTCAGGCAGCTCTGTCCAGTGGAGTGACCCGTTGCCAGAGTGCAGAGAAATTTATTGTCC AGCACCACCACAAATTGACAATGGAATAATTCAAGGGGAACGTGACCATTATGGATATAGACAGTCTGTAACGTA TGCATGTAATAAAGGATTCACCATGATTGGAGAGCACTCTATTTATTGTACTGTGAATAATGATGAAGGAGAGTGG AGTGGCCCACCACCTGAATGCAGAGGAAAATCTCTAACTTCCAAGGTCCCACCAACAGTTCAGAAACCTACCAC AGTAAATGTTCCAACTACAGAAGTCTCACCAACTTCTCAGAAAACCACCACAAAAACCACCACACCAAATGCTC AAGCAACACGGAGTACACCTGTTTCCAGGACAACCAAGCATTTTCATGAAACAACCCCAAATAAAGGAAGTGG AACCACTTCAGGTACTACCCGTCTTCTATCTGGGCACACGTGTTTCACGTTGACAGGTTTGCTTGGGACGCTAGT AACCATGGGCTTGCTGACTTAG |
| SE Q ID NO: 3 | MRLLAKIICLMLWAICVAEDCNELPPRRNTEILTGSWSDQTYPEGTQAIYKCRPGYRSLGNVIMVCRKGEWVALNPL RKCQKRPCGHPGDTPFGTFTLTGGNVFEYGVKAVYTCNEGYQLLGEINYRECDTDGWTNDIPICEVVKCLPVTAPEN GKIVSSAMEPDREYHFGQAVRFVCNSGYKIEGDEEMHCSDDGFWSKEKPKCVEISCKSPDVINGSPISQKIIYKENERF QYKCNMGYEYSERGDAVCTESGWRPLPSCEEKSCDNPYIPNGDYSPLRIKHRTGDEITYQCRNGFYPATRGNTAKCT STGWIPAPRCTLKPCDYPDIKHGGLYHENMRRPYFPVAVGKYYSYYCDEHFETPSGSYWDHIHCTQDGWSPAVPCLR KCYFPYLENGYNQNYGRKFVQGKSIDVACHPGYALPKAQTTVTCMENGWSPTPRCIRVKTCSKSSIDIENGFISESQY TYALKEKAKYQCKLGYVTADGETSGSITCGKDGWSAQPTCIKSCDIPVFMNARTKNDFTWFKLNDTLDYECHDGYE SNTGSTTGSIVCGYNGWSDLPICYERECELPKIDVHLVPDRKKDQYKVGEVLKFSCKPGFTIVGPNSVQCYHFGLSPD LPICKEQVQSCGPPPELLNGNVKEKTKEEYGHSEVVEYYCNPRFLMKGPNKIQCVDGEWTTLPVCIVEESTCGDIPEL EHGWAQLSSPPYYYGDSVEFNCSESFTMIGHRSITCIHGVWTQLPQCVAIDKLKKCKSSNLIILEEHLKNKKEFDHNS NIRYRCRGKEGWIHTVCINGRWDPEVNCSMAQIQLCPPPPQIPNSHNMTTTLNYRDGEKVSVLCQENYLIQEGEEITC KDGRWQSIPLCVEKIPCSQPPQIEHGTINSSRSSQESYAHGTKLSYTCEGGFRISEENETTCYMGKWSSPPQCEGLPCK SPPEISHGVVAHMSDSYQYGEEVTYKCFEGFGIDGPAIAKCLGEKWSHPPSCIKTDCLSLPSFENAIPMGEKKDVYKA GEQVTYTCATYYKMDGASNVTCINSRWTGRPTCRDTSCVNPPTVQNAYIVSRQMSKYPSGERVRYQCRSPYEMFGD EEVMCLNGNWTEPPQCKDSTGKCGPPPPIDNGDITSFPLSVYAPASSVEYQCQNLYQLEGNKRITCRNGQWSEPPKCL HPCVISREIMENYNIALRWTAKQKLYSRTGESVEFVCKRGYRLSSRSHTLRTTCWDGKLEYPTCAKR |

| | |
|---|---|
| SEQ ID NO: 4 | ATGAGACTTCTAGCAAAGATTATTTGCCTTATGTTATGGGCTATTTGTGTAGCAGAAGATTGCAATGAACTTCCTCC AAGAAGAAATACAGAAATTCTGACAGGTTCCTGGTCTGACCAAACATATCCAGAAGGCACCCAGGCTATCTATAA ATGCCGCCCTGGATATAGATCTCTTGGAAATGTAATAATGGTATGCAGGAAGGGAGAATGGGTTGCTCTTAATCCA TTAAGGAAATGTCAGAAAAGGCCCTGTGGACATCCTGGAGATACTCCTTTTGGTACTTTTACCCTTACAGGAGGA AATGTGTTTGAATATGGTGTAAAAGCTGTGTATACATGTAATGAGGGGTATCAATTGCTAGGTGAGATTAATTACCG TGAATGTGACACAGATGGATGGACCAATGATATTCCTATATGTGAAGTTGTGAAGTGTTTACCAGTGACAGCACC AGAGAATGGAAAAATTGTCAGTAGTGCAATGGAACCAGATCGGGAATACCATTTTGGACAAGCAGTACGGTTTG TATGTAACTCAGGCTACAAGATTGAAGGAGATGAAGAAATGCATTGTTCAGACGATGGTTTTTGGAGTAAAGAG AAACCAAAGTGTGTGGAAATTTCATGCAAATCCCCAGATGTTATAAATGGATCTCCTATATCTCAGAAGATTATTTA TAAGGAGAATGAACGATTTCAATATAAATGTAACATGGGTTATGAATACAGTGAAAGAGGAGATGCTGTATGCAC TGAATCTGGATGGCGTCCGTTGCCTTCATGTGAAGAAAAATCATGTGATAATCCTTATATTCCAAATGGTGACTAC TCACCTTTAAGGATTAAACACAGAACTGGAGATGAAATCACGTACCAGTGTAGAAATGGTTTTTATCCTGCAACC CGGGGAAATACAGCAAAATGCACAAGTACTGGCTGGATACCTGCTCCGAGATGTACCTTGAAACCTTGTGATTAT CCAGACATTAAACATGGAGGTCTATATCATGAGAATATGCGTAGACCATACTTTCCAGTAGCTGTAGGAAAATATT ACTCCTATTACTGTGATGAACATTTTGAGACTCCGTCAGGAAGTTACTGGGATCACATTCATTGCACACAAGATGG ATGGTCGCCAGCAGTACCATGCCTCAGAAAATGTTATTTTCCTTATTTGGAAAATGGATATAATCAAAATCATGGA AGAAAGTTTGTACAGGGTAAATCTATAGACGTTGCCTGCCATCCTGGCTACGCTCTTCCAAAAGCGCAGACCACA GTTACATGTATGGAGAATGGCTGGTCTCCTACTCCCAGATGCATCCGTGTCAAAACATGTTCCAAATCAAGTATAG ATATTGAGAATGGGTTTATTTCTGAATCTCAGTATACATATGCCTTAAAAGAAAAAGCGAAATATCAATGCAAACT AGGATATGTAACAGCAGATGGTGAAACATCAGGTAATTACATGTGGGAAAGATGGATGGTCAGCTCAACCCAC GTGCATTAAATCTTGTGATATCCCAGTATTTATGAATGCCAGAACTAAAAATGACTTCACATGGTTTAAGCTGAAT GACACATTGGACTATGAATGCCATGATGGTTATGAAAGCAATACTGGAAGCACCACTGGTTCCATAGTGTGTGGTT ACAATGGTTGGTCTGATTTACCCATATGTTATGAAAGAGAATGCGAACTTCCTAAAATAGATGTACACTTAGTTCC TGATCGCAAGAAAGACCAGTATAAAGTTGGAGAGGTGTTGAAATTCTCCTGCAAACCAGGATTTACAATAGTTG GACCTAATTCCGTTCAGTGCTACCACTTTGGATTGTCTCCTGACCTCCCAATATGTAAAGAGCAAGTACAATCATG TGGTCCACCTCCTGAACTCCTCAATGGGAATGTTAAGGAAAAAACGAAAGAAGAATATGGACACAGTGAAGTG GTGGAATATTATTGCAATCCTAGATTTCTAATGAAGGGACCTAATAAAAATTCAATGTGTTGATGGAGAGTGGACAA CTTTACCAGTGTGTATTGTGGAGGAGAGTACCTGTGGAGATATACCTGAACTTGAACATGGCTGGGCCCAGCTTT CTTCCCCTCCTTATTACTATGGAGATTCAGTGGAATTCAATTGCTCAGAATCATTTACAATGATTGGACACAGATCA ATTACGTGTATTCATGGAGTATGGACCCAACTTCCCCAGTGTGTGGCAATAGATAAACTTAAGGAAGTGCAAATCAT CAAATTTAATTATACTTGAGGAACATTTAAAAAAACAAGAAGGAATTCGATCATAATTCTAACATAAGGTACAGATG TAGAGGAAAAGAAGGATGGGATACACACAGTCTGCATAAATGGGAAGGATGGGATCCAGAAGATGGAACTGCTCAATGG CACAAATACAATTATGCCCACCTCCACCTCAGATTCCCAATTCTCACAATATGACAACCACACTGAATTATCGGGA TGGAGAAAAAGTATCTGTTCTTTGCCAAGAAAATTATCTAATTCAGGAAGGAGAAGAAATTACATGCAAAGATGG AAGATGGCAGTCAATACCACTCTGTGTTGAAAAAATTCCATGTTCACAACCACCTCAGATAGAACACGGAACCAT TAATTCATCCAGGTCTTCACAAGAAAGTTATGCACATGGGACTAAATTGAGTTATACTTGTGAGGGTGGTTTCAGG ATATCTGAAGAAATGAAACAACATGCTACATGGGAAAATGGAGTTCTCCACCTCAGTGTGAAGGCCTTCCTTGT AAATCTCCACCTGAGATTTCTCATGGTGTTGTAGCTCACATGTCAGACAGTTATCAATGTAGGGAAGAAGTTACGT ACAAATGTTTTGAAGGTTTTGGAATTGATGGGCCTGCAATTGCAAAATGCTTAGGAGAAAAATGGTCTCACCCTC CATCATGCATAAAAACAGATTGTCTCAGTTTACCTAGCTTTGAAAATGCCATACCCATGGGAGAGAAGAAGGATG TGTATAAGGCGGGTGAGCAAGTGACTTACACTTGTGCAACATATTACAAAATGGATGGAGCCAGTAATGTAACAT GCATTAATAGCAGATGGACAGGAAGGCCAACATGCAGAGACACCTCCTGTGTGAATCCGCCCACAGTACAAAAT GCTTATATAGTGTCGAGCACAGATGAGTAAATATCCATCTGGTGAGAGAGTACGTTATCAATGTAGGAGCCCTTATG AAATGTTTGGGGATGAAGAAGTGATGTGTTTTAAATGGAAAACTGGACGGAACCACCTCAATGCAAAGATTCTACA GGAAAATGTGGGCCCCCTCCACCTATTGACAATGGGGACATTACTTCATTCCCGTTGTCAGTATATGCTCCAGCTT CATCAGTTGAGTACCAATGCCAGAACTTGTATCAACTTGAGGGTAACAAGCGAATAACATGTAGAAATGGACAAT GGTCAGAACCACCAAAATGCTTACATCCGTGTGTAATATCCCGAGAAATTATGGAAAATTATAACATAGCATTAAG GTGGACAGCCAAACAGAAGCTTTATTCGAGAACAGGTGAATCAGTTGAATTTGTGTGTAAACGGGGATATCGTCT TTCATCACGTTCTCACACATTGCGAACAACATGTTGGGATGGGAAACTGGAGTATCCAACTTGTGCAAAAAGATA G |
| SEQ ID NO: 5 | MRLLAKIICLMLWAICVAEDCNELPPRRNTEILTGSWSDQTYPEGTQAIYKCRPGYRSLGNVIMVCRKGEWVALNPL RKCQKRPCGHPGDTPFGTFTLTGGNVFEYGVKAVYTCNEGYQLLGEINYRECDTDGWTNDIPICEVVKCLPVTAPEN GKIVSSAMEPDREYHFGQAVRFVCNSGYKIEGDEEMHCSDDGFWSKEKPKCVEISCKSPDVINGSPISQKIIYKENERF QYKCNMGYEYSERGDAVCTESGWRPLPSCEEKSCDNPYIPNGDYSPLRIKHRTGDEITYQCRNGFYPATRGNTAKCT STGWIPAPRCTLKPCDYPDIKHGGLYHENMRRPYFPVAVGKYYSYYCDEHFETPSGSYWDHIHCTQDGWSPAVPCLR KCYFPYLENGYNQNYGRKFVQGKSIDVACHPGYALPKAQTTVTCMENGWSPTPRCIRVSFTL |
| SEQ ID NO: 6 | ATGAGACTTCTAGCAAAGATTATTTGCCTTATGTTATGGGCTATTTGTGTAGCAGAAGATTGCAATGAACTTCCTCC AAGAAGAAATACAGAAATTCTGACAGGTTCCTGGTCTGACCAAACATATCCAGAAGGCACCCAGGCTATCTATAA ATGCCGCCCTGGATATAGATCTCTTGGAAATGTAATAATGGTATGCAGGAAGGGAGAATGGGTTGCTCTTAATCCA TTAAGGAAATGTCAGAAAAGGCCCTGTGGACATCCTGGAGATACTCCTTTTGGTACTTTTACCCTTACAGGAGGA AATGTGTTTGAATATGGTGTAAAAGCTGTGTATACATGTAATGAGGGGTATCAATTGCTAGGTGAGATTAATTACCG TGAATGTGACACAGATGGATGGACCAATGATATTCCTATATGTGAAGTTGTGAAGTGTTTACCAGTGACAGCACC AGAGAATGGAAAAATTGTCAGTAGTGCAATGGAACCAGATCGGGAATACCATTTTGGACAAGCAGTACGGTTTG TATGTAACTCAGGCTACAAGATTGAAGGAGATGAAGAAATGCATTGTTCAGACGATGGTTTTTGGAGTAAAGAG AAACCAAAGTGTGTGGAAATTTCATGCAAATCCCCAGATGTTATAAATGGATCTCCTATATCTCAGAAGATTATTTA TAAGGAGAATGAACGATTTCAATATAAATGTAACATGGGTTATGAATACAGTGAAAGAGGAGATGCTGTATGCAC TGAATCTGGATGGCGTCCGTTGCCTTCATGTGAAGAAAAATCATGTGATAATCCTTATATTCCAAATGGTGACTAC TCACCTTTAAGGATTAAACACAGAACTGGAGATGAAATCACGTACCAGTGTAGAAATGGTTTTTATCCTGCAACC CGGGGAAATACAGCAAAATGCACAAGTACTGGCTGGATACCTGCTCCGAGATGTACCTTGAAACCTTGTGATTAT CCAGACATTAAACATGGAGGTCTATATCATGAGAATATGCGTAGACCATACTTTCCAGTAGCTGTAGGAAAATATT ACTCCTATTACTGTGATGAACATTTTGAGACTCCGTCAGGAAGTTACTGGGATCACATTCATTGCACACAAGATGG ATGGTCGCCAGCAGTACCATGCCTCAGAAAATGTTATTTTCCTTATTTGGAAAATGGATATAATCAAAATCATGGA AGAAAGTTTGTACAGGGTAAATCTATAGACGTTGCCTGCCATCCTGGCTACGCTCTTCCAAAAGCGCAGACCACA GTTACATGTATGGAGAATGGCTGGTCTCCTACTCCCAGATGCATCCGTGTCAGCTTTACCCTCTGA |

(continued)

| SEQ ID NO: 7 | KSCPNPGEIRNGQIDVPGGILFGATISFSCNTGYKLFGSTSSFCLISGSSVQWSDPLPECRE |
|---|---|
| SEQ ID NO: 8 | CPNPGEIRNGQIDVPGGILFGATISFSCNTGYKLFGSTSSFCLISGSSVQWSDPLPECRE |
| SEQ ID NO: 9 | IYCPAPPQIDNGIIQGERDHYGYRQSVTYACNKGFTMIGEHSIYCTVNNDEGEWSGPPPECRG |
| SEQ ID NO: 10 | KSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRG |
| SEQ ID NO: 11 | CPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRG |
| SEQ ID NO: 12 | EDCNELPPRRNTEILTGSWSDQTYPEGTQAIYKCRPGYRSLGNVIMVCRKGEWVALNPLRKCQKRP |
| SEQ ID NO: 13 | EDCNELPPRRNTEILTGSWSDQTYPEGTQAIYKCRPGYRSLGNVIMVCRKGEWVALNPLRKCQK |
| SEQ ID NO: 14 | EDCNELPPRRNTEILTGSWSDQTYPEGTQAIYKCRPGYRSLGNIIMVCRKGEWVALNPLRKCQKRP |
| SEQ ID NO: 15 | EDCNELPPRRNTEILTGSWSDQTYPEGTQAIYKCRPGYRSLGNIIMVCRKGEWVALNPLRKCQK |
| SEQ ID NO: 16 | GHHHHHH |
| SEQ ID NO: 17 | MGWSCIILFLVATATGVHS |
| SEQ ID NO: 18 | EDCNELPPRRNTEILTGSWSDQTYPEGTQAIYKCRPGYRSLGNVIMVCRKGEWVALNPLRKCQKRPCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRG |
| SEQ ID NO: 19 | EDCNELPPRRNTEILTGSWSDQTYPEGTQAIYKCRPGYRSLGNVIMVCRKGEWVALNPLRKCQKKSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRG |
| SEQ ID NO: 20 | EDCNELPPRRNTEILTGSWSDQTYPEGTQAIYKCRPGYRSLGNIIMVCRKGEWVALNPLRKCQKKSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRG |
| SEQ ID NO: 21 | EDCNELPPRRNTEILTGSWSDQTYPEGTQAIYKCRPGYRSLGNIIMVCRKGEWVALNPLRKCQKRPCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRG |
| SEQ ID NO: 22 | EDCNELPPRRNTEILTGSWSDQTYPEGTQAIYKCRPGYRSLGNVIMVCRKGEWVALNPLRKCQKRPCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGGHHHHHH |
| SEQ ID NO: 23 | EDCNELPPRRNTEILTGSWSDQTYPEGTQAIYKCRPGYRSLGNVIMVCRKGEWVALNPLRKCQKKSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGGHHHHHH |
| SEQ ID NO: 24 | EDCNELPPRRNTEILTGSWSDQTYPEGTQAIYKCRPGYRSLGNIIMVCRKGEWVALNPLRKCQKKSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGGHHHHHH |
| SEQ ID NO: 25 | EDCNELPPRRNTEILTGSWSDQTYPEGTQAIYKCRPGYRSLGNIIMVCRKGEWVALNPLRKCQKRPCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGGHHHHHH |
| SEQ ID NO: 26 | MGWSCIILFLVATATGVHSEDCNELPPRRNTEILTGSWSDQTYPEGTQAIYKCRPGYRSLGNVIMVCRKGEWVALNPLRKCQKRPCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGGHHHHHH |
| SEQ ID NO: 27 | MGWSCIILFLVATATGVHSEDCNELPPRRNTEILTGSWSDQTYPEGTQAIYKCRPGYRSLGNVIMVCRKGEWVALNPLRKCQKKSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGGHHHHHH |
| SEQ ID NO: 28 | MGWSCIILFLVATATGVHSEDCNELPPRRNTEILTGSWSDQTYPEGTQAIYKCRPGYRSLGNIIMVCRKGEWVALNPLRKCQKKSCPNPGEIRNGQIDVPGGILFGATISFSCNTGYKLFGSTSSFCLISGSSVQWSDPLPECREIYCPAPPQIDNGIIQGERDHYGYRQSVTYACNKGFTMIGEHSIYCTVNNDEGEWSGPPPECRGGHHHHHH |

(continued)

| SEQ ID NO: 29 | MGWSCIILFLVATATGVHSEDCNELPPRRNTEILTGSWSDQTYPEGTQAIYKCRPGYRSLGNIIMVCRKGEWVALNPL RKCQKRPCPNPGEIRNGQIDVPGGILFGATISFSCNTGYKLFGSTSSFCLISGSSVQWSDPLPECREIYCPAPPQIDNGIIQ GERDHYGYRQSVTYACNKGFTMIGEHSIYCTVNNDEGEWSGPPPECRGGHHHHHH |
|---|---|
| SEQ ID NO: 30 | MGWSCIILFLVATATGVHSEDCNELPPRRNTEILTGSWSDQTYPEGTQAIYKCRPGYRSLGNVIMVCRKGEWVALNPL RKCQKRPCPNPGEIRNGQIDVPGGILFGATISFSCNTGYKLFGSTSSFCLISGSSVQWSDPLPECREIYCPAPPQIDNGIIQ GERDHYGYRQSVTYACNKGFTMIGEHSIYCTVNNDEGEWSGPPPECRG |
| SEQ ID NO: 31 | MGWSCIILFLVATATGVHSEDCNELPPRRNTEILTGSWSDQTYPEGTQAIYKCRPGYRSLGNVIMVCRKGEWVALNPL RKCQKKSCPNPGEIRNGQIDVPGGILFGATISFSCNTGYKLFGSTSSFCLISGSSVQWSDPLPECREIYCPAPPQIDNGIIQ GERDHYGYRQSVTYACNKGFTMIGEHSIYCTVNNDEGEWSGPPPECRG |
| SEQ ID NO: 32 | MGWSCIILFLVATATGVHSEDCNELPPRRNTEILTGSWSDQTYPEGTQAIYKCRPGYRSLGNIIMVCRKGEWVALNPL RKCQKKSCPNPGEIRNGQIDVPGGILFGATISFSCNTGYKLFGSTSSFCLISGSSVQWSDPLPECREIYCPAPPQIDNGIIQ GERDHYGYRQSVTYACNKGFTMIGEHSIYCTVNNDEGEWSGPPPECRG |
| SEQ ID NO: 33 | MGWSCIILFLVATATGVHSEDCNELPPRRNTEILTGSWSDQTYPEGTQAIYKCRPGYRSLGNIIMVCRKGEWVALNPL RKCQKRPCPNPGEIRNGQIDVPGGILFGATISFSCNTGYKLFGSTSSFCLISGSSVQWSDPLPECREIYCPAPPQIDNGIIQ GERDHYGYRQSVTYACNKGFTMIGEHSIYCTVNNDEGEWSGPPPECRG |
| SEQ ID NO: 34 | QCNAPEWLPFARPTNLTDEFEFPIGTYLKYECRPGYYGRPFSIICLKNSVWTGAKDRCRRKSCRNPPDPVNGMVHVI KDIQFGSQIKYSCTKGYRLIGSSSATCIISGNTVIWDNETPICDRIPCGLPPTITNGDFISTNRENFHYGSVVTYRCNPGS GGRKVFELVGEPSIYCTSNDDQVGIWSGPAPQCIGHHHHHH |
| SEQ ID NO: 35 | SCEVPTRLNSASLKQPYITQNYFPVGTVVEYECRPGYRREPSLSPKLTCLQNLKWSTAVEFCKKKSCPNPGEIRNGQID VPGGILFGATISFSCNTGYKLFGSTSSFCLISGSSVQWSDPLPECREIYCPAPPQIDNGIIQGERDHYGYRQSVTYACNK GFTMIGEHSIYCTVNNDEGEWSGPPPECRGGHHHHHH |
| SEQ ID NO: 36 | EDCNELPPRRNTEILTGSWSDQTYPEGTQAIYKCRPGYRSLGNIIMVCRKGEWVALNPLRKCQKRPCGHPGDTPFGT FTLTGGNVFEYGVKAVYTCNEGYQLLGEINYRECDTDGWTNDIPICEVVKCLPVTAPENGKIVSSAMEPDREYHFGQ AVRFVCNSGYKIEGDEEMHCSDDGFWSKEKPKCVEISCKSPDVINGSPISQKIIYKENERFQYKCNMGYEYSERGDAV CTESGWRPLPSCEEKSCDNPYIPNGDYSPLRIKHRTGDEITYQCRNGFYPATRGNTAKCTSTGWIPAPRCTLKPGHHH HHH |
| SEQ ID NO: 37 | GHCQAPDHFLFAKLKTQTNASDFPIGTSLKYECRPEYYGRPFSITCLDNLVWSSPKDVCKRKSCPNPGEIRNGQIDVP GGILFGATISFSCNTGYKLFGSTSSFCLISGSSVQWSDPLPECREIYCPAPPQIDNGIIQGERDHYGYRQSVTYACNKGF TMIGEHSIYCTVNNDEGEWSGPPPECRGGHHHHHH |
| SEQ ID NO: 38 | QEQTYVISAPKIFRVGASENIVIQVYGYTEAFDATISIKSYPDKKFSYSSGHVHLSSENKFQNSAILTIQPKQLPGGQNP VSYVYLEVVSKHFSKSKRMPITYDNGFLFIHTDKPVYTPDQSVKVRVYSLNDDLKPAKRETVLTFIDPEGSEVDMVE EIDHIGIISFPDFKIPSNPRYGMWTIKAKYKEDFSTTGTAYFEVKEYVLPHFSVSIEPEYNFIGYKNFKNFEITIKARYFY NKVVTEADVYITFGIREDLKDDQKEMMQTAMQNTMLINGIAQVTFDSETAVKELSYYSLEDLNNKYLYIAVTVIEST GGFSEEAEIPGIKYVLSPYKLNLVATPLFLKPGIPYPIKVQVKDSLDQLVGGVPVTLNAQTIDVNQETSDLDPSKSVTR VDDGVASFVLNLPSGVTVLEFNVKTDAPDLPEENQAREGYRAIAYSSLSQSYLYIDWTDNHKALLVGEHLNIIVTPKS PYIDKITHYNYLILSKGKIIHFGTREKFSDASYQSINIPVTQNMVPSSRLLVYYIVTGEQTAELVSDSVWLNIEEKCGNQ LQVHLSPDADAYSPGQTVSLNMATGMDSWVALAAVDSAVYGVQRGAKKPLERVFQFLEKSDLGCGAGGGLNNAN VFHLAGLTFLTNANADDSQENDEPCKEILRPRRTLQKKIEEIAAKYKHSVVKKCCYDGACVNNDETCEQRAARISLG PRCIKAFTECCVVASQLRANISHKDMQLGRLHMKTLLPVSKPEIRSYFPESWLWEVHLVPRRKQLQFALPDSLTTWEI QGVGISNTGICVADTVKAKVFKDVFLEMNIPYSVVRGEQIQLKGTVYNYRTSGMQFCVKMSAVEGICTSESPVIDHQ GTKSSKCVRQKVEGSSSHLVTFTVLPLEIGLHNINFSLETWFGKEILVKTLRVVPEGVKRESYSGVTLDPRGIYGTISR RKEFPYRIPLDLVPKTEIKRILSVKGLLVGEILSAVLSQEGINILTHLPKGSAEAELMSVVPVFYVFHYLETGNHWNIFH SDPLIEKQKLKKKLKEGMLSIMSYRNADYSYSVWKGGSASTWLTAFALRVLGQVNKYVEQNQNSICNSLLWLVENY QLDNGSFKENSQYQPIKLQGTLPVEARENSLYLTAFTVIGIRKAFDICPLVKIDTALIKADNFLLENTLPAQSTFTLAISA YALSLGDKTHPQFRSIVSALKREALVKGNPPIYRFWKDNLQHKDSSVPNTGTARMVETTAYALLTSLNLKDINYVNP VIKWLSEEQRYGGGFYSTQDTINAIEGLTEYSLLVKQLRLSMDIDVSYKHKGALHNYKMTDKNFLGRPVEVLLNDD LIVSTGFGSGLATVHVTTVVHKTSTSEEVCSFYLKIDTQDIEASHYRGYGNSDYKRIVACASYKPSREESSSGSSHAVM DISLPTGISANEEDLKALVEGVDQLFTDYQIKDGHVILQLNSIPSSDFLCVRFRIFELFEVGFLSPATFTVYEYHRPDKQ CTMFYSTSNIKIQKVCEGAACKCVEADCGQMQEELDLTISAETRKQTACKPEIAYAYKVSITSITVENVFVKYKATLL DIYKTGEAVAEKDSEITFIKKVTCTNAELVKGRQYLIMGKEALQIKYNFSFRYIYPLDSLTWIEYWPRDTTCSSCQAFL ANLDEFAEDIFLNGC |
| 39 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSS |
| 40 | GYTFTNY |
| 41 | NTYTGE |
| 42 | TKVPHYFAYAMDY |
| 43 | ASTKGPSVFPLAPCSRSTSESTAALGCLVKDYFPEPVTVSWNSGALTSGVHTFPAVLQSSGLYSLSSVVTVPSSSLGTK TYTCNVDHKPSNTKVDKRVESKYGPPCPPCPAPEFLGGPSVFLFPPKPKDTLMISRTPEVTCVVVDVSQEDPEVQFNW YVDGVEVHNAKTKPREEQFNSTYRVVSVLTVLHQDWLNGKEYKCKVSNKGLPSSIEKTISKAKGQPREPQVYTLPPS QEEMTKNQVSLTCLVKGFYPSDIAVEWESNGQPENNYKTTPPVLDSDGSFFLYSRLTVDKSRWQEGNVFSCSVLHEA LHAHYTQKSLSLSLGK |

| | |
|---|---|
| 44 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGK |
| 45 | DIVLTQSPSSLSASVGDRVTITCKASQSVDYHGDSYLNWYQQKPGKPPKLLIYAASNLESGIPSRFSGSGSGTDFTLTIS SLQPEDFATYYCQQSNEDPSTFGGGTKLEIK |
| 46 | KASQSVDYHGDSYLN |
| 47 | AASNLES |
| 48 | QQSNEDPST |
| 49 | RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC |
| 50 | DIVLTQSPSSLSASVGDRVTITCKASQSVDYHGDSYLNWYQQKPGKPPKLLIYAASNLESGIPSRFSGSGSGTDFTLTIS SLQPEDFATYYCQQSNEDPSTFGGGTKLEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTKSFNRGEC |
| 51 | EDCNELPPRRNTEILTGSWSDQTYPEGTQAIYKCRPGYRSLGNIIMVCRKGEWVALNPLRKCQKRPCGHPGDTPFGT FTLTGGNVFEYGVKAVYTCNEGYQLLGEINYRECDTDGWTNDIPICEVVKCLPVTAPENGKIVSSAMEPDREYHFGQ AVRFVCNSGYKIEGDEEMHCSDDGFWSKEKPKCVEISGHHHHHH |
| 52 | DCGLPPDVPNAQPALEGRTSFPEDTVITYKCEESFVKIPGEKDSVICLKGSQWSDIEEFCNRSCEVPTRLNSASLKQPYI TQNYFPVGTVVEYECRPGYRREPSLSPKLTCLQNLKWSTAVEFCKKKSCPNPGEIRNGQIDVPGGILFGATISFSCNTG YKLFGSTSSFCLISGSSVQWSDPLPECREIYCPAPPQIDNGIIQGERDHYGYRQSVTYACNKGFTMIGEHSIYCTVNND EGEWSGPPPECRGKSGHHHHHH |
| 53 | EDCNELPPRRNTEILTGSWSDQTYPEGTQAIYKCRPGYRSLGNIIMVCRKGEWVAENPSRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS*GHHHHHH* |
| 54 | EDCNELPPRRNTEILTGSWSDQTYPEGTQAIYKCRPGYRSLGNIIMVCRKGEWVAENPDRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS*GHHHHHH* |
| 55 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPGGILFG ATISFSCNTGYKLFGSTSSFCLISGSSVQWSDPLPECREIYCPAPPQIDNGIIQGERDHYGYRQSVTYACNKGFTMIGEH SIYCTVNNDEGEWSGPPPECRG |
| 56 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRG**KS***GHHHHHH* |
| 57 | EDCNELPPRRNTEILTGSWSDQTYPEGTQAIYKCRPGYRSLGNIIMVCRKGEWVA**E**NPLRKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRG**KS***GHHHHHH* |
| 58 | EDCNELPPRRNTEILTGSWSDQTYPEGTQAIYKCRPGYRSLGNIIMVCRKGEWVALNP**D**RKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRG**KS***GHHHHHH* |
| 59 | APEFLGGPSVFLFPPKPKDTLMISRTPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMTKNQVSLTCLVKGFYPSDIAVEWESN GQPENNYKTTPPVLDSDGSFFLYSRLTVDKSRWQEGNVFSCSVLHEALHAHYTQKSLSLSLGK |
| 60 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRGKS |

(continued)

| 61 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPGGILFG ATISFSCNTGYKLFGSTSSFCLISGSSVQWSDPLPECREIYCPAPPQIDNGIIQGERDHYGYRQSVTYACNKGFTMIGEH SIYCTVNNDEGEWSGPPPECRGKS |
| 62 | EDCNELPPRRNTEILTGSWSDQTYPEGTQAIYKCRPGYRSLGNIIMVCRKGEWVAENPLRKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRGKS |
| 63 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVAENPLRKCQKKSCPNPGEIRNGQIDVPGGILFG ATISFSCNTGYKLFGSTSSFCLISGSSVQWSDPLPECREIYCPAPPQIDNGIIQGERDHYGYRQSVTYACNKGFTMIGEH SIYCTVNNDEGEWSGPPPECRGKS |
| 64 | EDCNELPPRRNTEILTGSWSDQTYPEGTQAIYKCRPGYRSLGNIIMVCRKGEWVALNPDRKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRGKS |
| 65 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPDRKCQKKSCPNPGEIRNGQIDVPGGILFG ATISFSCNTGYKLFGSTSSFCLISGSSVQWSDPLPECREIYCPAPPQIDNGIIQGERDHYGYRQSVTYACNKGFTMIGEH SIYCTVNNDEGEWSGPPPECRGKS |
| 66 | EDCNELPPRRNTEILTGSWSDQTYPEGTQAIYKCRPGYRSLGNIIMVCRKGEWVALNPSRKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRGKS |
| 67 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPSRKCQKKSCPNPGEIRNGQIDVPGGILFG ATISFSCNTGYKLFGSTSSFCLISGSSVQWSDPLPECREIYCPAPPQIDNGIIQGERDHYGYRQSVTYACNKGFTMIGEH SIYCTVNNDEGEWSGPPPECRGKS |
| 68 | EDCNELPPRRNTEILTGSWSDQTYPEGTQAIYKCRPGYRSLGNIIMVCRKGEWVALNPLRKCQKKSCPNPGEIRNGQI DVPDGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRGKS |
| 69 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPDGILFG ATISFSCNTGYKLFGSTSSFCLISGSSVQWSDPLPECREIYCPAPPQIDNGIIQGERDHYGYRQSVTYACNKGFTMIGEH SIYCTVNNDEGEWSGPPPECRGKS |
| 70 | EDCNELPPRRNTEILTGSWSDQTYPEGTQAIYKCRPGYRSLGNIIMVCRKGEWVALNPLRKCQKKSCPNPGEIRNGQI DVPGGILYGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRGKS |

(continued)

| | |
|---|---|
| 71 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPGGILYG ATISFSCNTGYKLFGSTSSFCLISGSSVQWSDPLPECREIYCPAPPQIDNGIIQGERDHYGYRQSVTYACNKGFTMIGEH SIYCTVNNDEGEWSGPPPECRGKS |
| 72 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSECLISGSSVQWSDPLPECREIYCPAPPQIDNGIIQGERDHYGYRQSVTYACN KGFTMIGEHSIYCTVNNDEGEWSGPPPECRGKS |
| 73 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPGGILFG ATISFSCNTGYKLFGSTSSECLISGSSVQWSDPLPECREIYCPAPPQIDNGIIQGERDHYGYRQSVTYACNKGFTMIGEH SIYCTVNNDEGEWSGPPPECRGKS |
| 74 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFCDISGSSVQWSDPLPECREIYCPAPPQIDNGIIQGERDHYGYRQSVTYACN KGFTMIGEHSIYCTVNNDEGEWSGPPPECRGKS |
| 75 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPGGILFG ATISFSCNTGYKLFGSTSSFCDISGSSVQWSDPLPECREIYCPAPPQIDNGIIQGERDHYGYRQSVTYACNKGFTMIGEH SIYCTVNNDEGEWSGPPPECRGKS |
| 76 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFCEISGSSVQWSDPLPECREIYCPAPPQIDNGIIQGERDHYGYRQSVTYACN KGFTMIGEHSIYCTVNNDEGEWSGPPPECRGKS |
| 77 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPGGILFG ATISFSCNTGYKLFGSTSSFCEISGSSVQWSDPLPECREIYCPAPPQIDNGIIQGERDHYGYRQSVTYACNKGFTMIGEH SIYCTVNNDEGEWSGPPPECRGKS |
| 78 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS |
| 79 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKKSCPNPGEIRNGQIDVPGGILFG ATISFSCNTGYKLFGSTSSFCLTSGSSVQWSDPLPECREIYCPAPPQIDNGIIQGERDHYGYRQSVTYACNKGFTMIGEH SIYCTVNNDEGEWSGPPPECRGKS |
| 80 | EDCNELPPRRNTEILTGSWSDQTYPEGTQAIYKCRPGYRSLGNIIMVCRKGEWVALNPDRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS |

| | |
|---|---|
| 81 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPDRKCQKKSCPNPGEIRNGQIDVPDGILFG ATISFSCNTGYKLFGSTSSFCLTSGSSVQWSDPLPECREIYCPAPPQIDNGIIQGERDHYGYRQSVTYACNKGFTMIGEH SIYCTVNNDEGEWSGPPPECRGKS |
| 82 | EDCNELPPRRNTEILTGSWSDQTYPEGTQAIYKCRPGYRSLGNIIMVCRKGEWVALNPSRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS |
| 83 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPSRKCQKKSCPNPGEIRNGQIDVPDGILFG ATISFSCNTGYKLFGSTSSFCLTSGSSVQWSDPLPECREIYCPAPPQIDNGIIQGERDHYGYRQSVTYACNKGFTMIGEH SIYCTVNNDEGEWSGPPPECRGKS |
| 84 | EDCNELPPRRNTEILTGSWSDQTYPEGTQAIYKCRPGYRSLGNIIMVCRKGEWVAENPDRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS |
| 85 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVAENPDRKCQKKSCPNPGEIRNGQIDVPDGILFG ATISFSCNTGYKLFGSTSSFCLTSGSSVQWSDPLPECREIYCPAPPQIDNGIIQGERDHYGYRQSVTYACNKGFTMIGEH SIYCTVNNDEGEWSGPPPECRGKS |
| 86 | EDCNELPPRRNTEILTGSWSDQTYPEGTQAIYKCRPGYRSLGNIIMVCRKGEWVAENPSRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS |
| 87 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVAENPSRKCQKKSCPNPGEIRNGQIDVPDGILFG ATISFSCNTGYKLFGSTSSFCLTSGSSVQWSDPLPECREIYCPAPPQIDNGIIQGERDHYGYRQSVTYACNKGFTMIGEH SIYCTVNNDEGEWSGPPPECRGKS |
| 88 | EDCNELPPRRNTEILTGSWSDQTYPEGTQAIYKCRPGYRSLGNIIMVCRKGEWVALNP**S**RKCQKKSCPNPGEIRNGQI DVPGGILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRG**KS***HHHHHH* |
| 89 | EDCNELPPRRNTEILTGSWSDQTYPEGTQAIYKCRPGYRSLGNIIMVCRKGEWVALNPLRKCQKKSCPNPGEIRNGQI DVP**D**GILFGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRG**KS***HHHHHH* |
| 90 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFCL**T**SGSSVQWSDPLPECREIYCPAPPQIDNGIIQGERDHYGYRQSVTYACN KGFTMIGEHSIYCTVNNDEGEWSGPPPECRG**KS***HHHHHH* |
| 91 | EDCNELPPRRNTEILTGSWSDQTYPEGTQAIYKCRPGYRSLGNIIMVCRKGEWVALNPLRKCQKKSCPNPGEIRNGQI DVPGGILYGATISFSCNTGYKLFGSTSSFCLISGSSVQWSDPLPECREIYCPAPPQIDNGIIQGERDHYGYRQSVTYACN KGFTMIGEHSIYCTVNNDEGEWSGPPPECRGKS*GHHHHHH* |
| 92 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSEC**L**ISGSSVQWSDPLPECREIYCPAPPQIDNGIIQGERDHYGYRQSVTYACN KGFTMIGEHSIYCTVNNDEGEWSGPPPECRGKS*GHHHHHH* |
| 93 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFC**D**ISGSSVQWSDPLPECREIYCPAPPQIDNGIIQGERDHYGYRQSVTYACN KGFTMIGEHSIYCTVNNDEGEWSGPPPECRGKS*GHHHHHH* |

(continued)

| 94 | EDCNELPPRRNTEILTGSWSDQTYPEGTQAIYKCRPGYRSLGNIIMVCRKGEWVALNPLRKCQKKSCPNPGEIRNGQI DVPGGILFGATISFSCNTGYKLFGSTSSFC**E**ISGSSVQWSDPLPECREIYCPAPPQIDNGIIQGERDHYGYRQSVTYACN KGFTMIGEHSIYCTVNNDEGEWSGPPPECRGKS*GHHHHHH* |
| 95 | EDCNELPPRRNTEILTGSWSDQTYPEGTQAIYKCRPGYRSLGNIIMVCRKGEWVALNPDRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS*GHHHHHH* |
| 96 | EDCNELPPRRNTEILTGSWSDQTYPEGTQAIYKCRPGYRSLGNIIMVCRKGEWVALNPSRKCQKKSCPNPGEIRNGQI DVPDGILFGATISFSCNTGYKLFGSTSSFCLTSGSSVQWSDPLPECREIYCPAPPQIDNGIIQGERDHYGYRQSVTYACN KGFTMIGEHSIYCTVNNDEGEWSGPPPECRGKS*GHHHHHH* |
| 97 | QVQLVQSGAEVKKPGASVKVSCKASGYTFTNYGMNWVRQAPGQGLEWMGWINTYTGEPTYADDFKGRFTFTLDT STSTAYLELSSLRSEDTAVYYCARTKVPHYFAYAMDYWGQGTLVTVSSASTKGPSVFPLAPCSRSTSESTAALGCLVK DYFPEPVTVSWNSGALTSGVHTFPAVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVLHEALHAHYTQKSLSLSLGKGSGEDCNELPPR RNTEILTGSWSDQTYPEGTQAIYKCRPGYRSLGNIIMVCRKGEWVALNPLRKCQKRPCGHPGDTPFGTFTLTGGNVF EYGVKAVYTCNEGYQLLGEINYRECDTDGWTNDIPICEVVKCLPVTAPENGKIVSSAMEPDREYHFGQAVRFVCNSG YKIEGDEEMHCSDDGFWSKEKPKCVEISCKSPDVINGSPISQKIIYKENERFQYKCNMGYEYSERGDAVCTESGWRPL PSCEEKSCDNPYIPNGDYSPLRIKHRTGDEITYQCRNGFYPATRGNTAKCTSTGWIPAPRCTLKP |

## Claims

1. A hybrid protein mutant, wherein the parent hybrid protein comprises or consists of

   (a) CCP1 of human complement factor H (CFH),
   (b) CCP3 and CCP4 of human decay-accelerating factor (DAF), and
   optionally a signal peptide and/or a tag;
   wherein compared to the parent hybrid protein, the mutant comprises one or more mutations selected from the group consisting of:

   mutations at position 56, 59, 82, 86, 107, 109, and/or 110, e.g., substitutions, e.g., substitutions to an amino acid that is more hydrophilic than the original amino acid; and/or mutations comprised at the C-terminus, e.g., addition of one or more amino acids at the C-terminus;
   wherein the positions of amino acid mutations are numbered corresponding to the amino acid positions as set forth in SEQ ID NO:20.

2. The hybrid protein mutant of claim 1, wherein the optimized hybrid protein comprises substitutions at position 59, 82 and 110 numbered corresponding to the amino acid positions as set forth in SEQ ID NO:20, and/or addition of one or more amino acids at the C-terminus.

3. The hybrid protein mutant of claim 2 comprising substitutions at position 56, 86, 107 and/or 109 numbered corresponding to the amino acid positions as set forth in SEQ ID NO:20.

4. The hybrid protein mutant of claim 2 comprising substitutions at position 56, 59, 82 and/or 110 numbered corresponding to the amino acid positions as set forth in SEQ ID NO:20, e.g., substitutions at 59, 82 and 110, or substitutions at 56, 59, 82 and 110.

5. The hybrid protein mutant of any one of claims 1-4, wherein the mutation(s) are selected from the group consisting of:

   substitutions of the amino acids at position 56, 59, 82, 86, 107, 109 and/or 110 numbered corresponding to the amino acid position as set forth in SEQ ID NO:20, to amino acids which is more hydrophilic than the original amino acids, for example to D, E, S, Y or T; or
   addition of 1-5 amino acids at the C-terminus, for example 2 amino acids.

6. The hybrid protein mutation of any one of claims 1-5, wherein the mutation(s) are selected from the group consisting of: 56D/E, 59D/S, 82D, 86Y, 107E, 109D/E, 110T or an addition of the amino acid KS at the C-terminus, or a combination thereof.

7. The hybrid protein mutant of claim 6, comprising

56E;
59D/S;
82D;
86Y;
107E;
109D/E;
110T;
59D-82D-110T;
59S-82D-110T;
59D-82D-110T-56E;
59S-82D-110T-56E, or
an addition of the amino acids KS at the C-terminus.

8. The hybrid protein mutant of any one of claims 1-7, wherein the CCP3 and CCP4 of DAF are directly linked together to form CCP3-4.

9. The hybrid protein mutant of any one of claims 1-8, wherein the CCP1 of human CFH comprises or consists of the amino acid sequence at positions 19-82 of the human CFH protein, optionally the CCP1 comprises a V62I mutation, wherein the amino acid positions are numbered corresponding to the amino acid positions as set forth in SEQ ID NO:3.

10. The hybrid protein mutant of any one of claims 1-9, wherein the CCP1 of human CFH comprises or consists of the amino acid sequence at positions 19-84 of the human CFH protein, optionally, CCP1 comprises a V62I mutation, wherein the amino acid positions are numbered corresponding to the amino acid positions as set forth in SEQ ID NO:3.

11. The hybrid protein mutant of any one of claims 1-10, wherein the CCP3 of human DAF comprises or consists of the amino acid sequence at positions 161-222 of the human DAF protein, and/or the CCP4 of human DAF comprises or consists of the amino acid sequence at positions 223-285 of the human DAF protein, wherein the amino acid positions are numbered corresponding to the amino acid positions as set forth in SEQ ID NO: 1.

12. The hybrid protein mutant of any one of claims 1-11, wherein the CCP3 of human DAF comprises or consists of the amino acid sequence at positions 163-222 of the human DAF protein, and/or the CCP4 of human DAF comprises or consists of the amino acid sequence at positions 223-285 of the human DAF protein, wherein the amino acid positions are numbered corresponding to the amino acid positions as set forth in SEQ ID NO: 1.

13. The hybrid protein mutant of any one of claims 1-12, wherein the CCP3-4 of human DAF comprises or consists of the amino acid sequence at positions 161-285 of the human DAF protein, wherein the amino acid positions are numbered corresponding to the amino acid positions as set forth in SEQ ID NO: 1.

14. The hybrid protein mutant of any one of claims 1-13, wherein the CCP3-4 of human DAF comprises or consists of the amino acid sequence at positions 163-285 of the human DAF protein, wherein the amino acid positions are numbered corresponding to the amino acid positions as set forth in SEQ ID NO: 1.

15. The hybrid protein mutant of any one of claims 1-14, wherein

(1) the CCP1 of human CFH comprises or consists of the amino acid sequences at positions 19-82 of the human CFH protein, and the CCP3 of human DAF comprises or consists of the amino acid sequence at positions 161-222 of the human DAF protein, and the CCP4 of human DAF comprises or consists of the amino acid sequence at positions 223-285 of the human DAF protein;
(2) the CCP1 of human CFH comprises or consists of the amino acid sequences at positions 19-84 of the human CFH protein, and the CCP3 of human DAF comprises or consists of the amino acid sequence at positions 163-222 of the human DAF protein, and the CCP4 of human DAF comprises or consists of the amino acid sequence at positions 223-285 of the human DAF protein;
(3) the CCP1 of human CFH comprises or consists of the amino acid sequence at positions 19-82 of the human CFH protein, and the CCP3-4 of human DAF comprises or consists of the amino acid sequence at positions 161-285 of the human DAF protein; or
(4) the CCP1 of human CFH comprises or consists of the amino acid sequence at positions 19-84 of the human CFH protein, and the CCP3-4 of human DAF comprises or consists of the amino acid sequence at positions 163-285 of the human DAF protein;

wherein the amino acid positions of the human CFH protein are numbered corresponding to the amino acid positions as set forth in SEQ ID NO: 3 and the amino acid positions of the human DAF protein are numbered corresponding to the amino acid positions as set forth in SEQ ID NO: 1.

16. The hybrid protein mutant of any one of claims 1-15, wherein the CCP1 of human CFH has the V62I mutation.

17. The hybrid protein mutant of any oen of claims 1-16, wherein the human CFH protein is a human native CFH protein or comprises

(i) the amino acid sequence as set forth in SEQ ID NO: 3 or 5;
(ii) the amino acid sequence encoded by the nucleotide sequence as set forth in SEQ ID NO: 4 or 6;
(iii) the amino acid sequence having at least 95%, 96%, 97%, 98%, or 99% identity to the amino acid sequence as set forth in (i) or (ii);

or consists of the amino acid sequence as set forth in any one of (i) to (iii).

18. The hybrid protein mutant of any one of claims 1-17, wherein the human DAF protein is a native human DAF protein or comprises

(i) the amino acid sequence as set forth in SEQ ID NO: 1;
(ii) the amino acid sequence encoded by the nucleotide sequence as set forth in SEQ ID NO: 2;
(iii) the amino acid sequence having at least 95%, 96%, 97%, 98%, or 99% identity to the amino acid sequence as set forth in (i) or (ii);

or consists of the amino acid sequence as set forth in any one of (i) to (iii).

19. The hybrid protein mutant of any one of claims 1-18, wherein

the CCP1 of human CFH comprises or consists of the amino acid sequence as set forth in SEQ ID NO: 12, 13, 14, or 15, or an amino acid sequence having at least 95%, 96%, 97%, 98%, or 99% identity to the amino acid sequence as set forth in SEQ ID NO: 12, 13, 14, or 15;
the CCP3 of human DAF comprises or consists of the amino acid sequence as set forth in SEQ ID NO: 7 or 8, or an amino acid sequence having at least 95%, 96%, 97%, 98%, or 99% identity to the amino acid sequence as set forth in SEQ ID NO: 7 or SEQ ID NO: 8;
the CCP4 of human DAF comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9; and/or
the CCP3-4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.

20. The hybrid protein mutant of any one of claims 1-19, wherein the tag is, for example, a purification tag such as a hexahistidine tag or a biotin marker, comprising, e.g., the amino acid sequence as set forth in SEQ ID NO: 16.

21. The hybrid protein mutant of any one of claims 1-20, wherein the signal peptide is a secretory signal peptide, comprising, e.g., the amino acid sequence as set forth in SEQ ID NO: 17.

22. The hybrid protein mutant of any one of claims 1-21, wherein the parent hybrid protein comprises or consists of the amino acid sequence of any one of SEQ ID NOs: 18-33, or an amino acid sequence having at least a 95%, 96%, 97%, 98%, or 99% identity to the amino acid sequence.

23. The hybrid protein mutant of claim 22, wherein the parent hybrid protein comprises or consists of the amino acid sequence of SEQ ID NO:20, or an amino acid sequence having at least a 95%, 96%, 97%, 98%, or 99% identity to the amino acid sequence.

24. The hybrid protein mutant of any one of claims 1-23, comprising or consisting of the amino acid sequence of any one of SEQ ID NOs: 53-54, 56-58, 60, 62, 64, 66, 68, 70, 72, 74, 76, 78, 80, 82, 84, 86, 88-96, or an amino acid sequence having at least a 95%, 96%, 97%, 98%, or 99% identity to the amino acid sequence.

25. The hybrid protein mutant of any one of claims 1-23,

(1) comprising an amino acid sequence having at least 95%, 96%, 97%, 98%, 99% identity with the amino acid sequence as set forth in SEQ ID NO:60, and comprising an addition of KS at the C-terminus;
(2) comprising an amino acid sequence having at least 95%, 96%, 97%, 98%, 99% identity with the amino acid sequence as set forth in SEQ ID NO:62, and comprising an addition of KS at the C-terminus and a mutation of 56E;
(3) comprising an amino acid sequence having at least 95%, 96%, 97%, 98%, 99% identity with the amino acid sequence as set forth in SEQ ID NO:64, and comprising an addition of KS at the C-terminus and a mutation of 59D;
(4) comprising an amino acid sequence having at least 95%, 96%, 97%, 98%, 99% identity with the amino acid sequence as set forth in SEQ ID NO:66, and comprising an addition of KS at the C-terminus and a mutation of 59S;
(5) comprising an amino acid sequence having at least 95%, 96%, 97%, 98%, 99% identity with the amino acid sequence as set forth in SEQ ID NO:68, and comprising an addition of KS at the C-terminus and a mutation of 82D;
(6) comprising an amino acid sequence having at least 95%, 96%, 97%, 98%, 99% identity with the amino acid sequence as set forth in SEQ ID NO:70, and comprising an addition of KS at the C-terminus and a mutation of 86Y;
(7) comprising an amino acid sequence having at least 95%, 96%, 97%, 98%, 99% identity with the amino acid sequence as set forth in SEQ ID NO:72, and comprising an addition of KS at the C-terminus and a mutation of 107E;
(8) comprising an amino acid sequence having at least 95%, 96%, 97%, 98%, 99% identity with the amino acid sequence as set forth in SEQ ID NO:74, and comprising an addition of KS at the C-terminus and a mutation of 109D;
(9) comprising an amino acid sequence having at least 95%, 96%, 97%, 98%, 99% identity with the amino acid sequence as set forth in SEQ ID NO:76, and comprising an addition of KS at the C-terminus and a mutation of 109E;
(10) comprising an amino acid sequence having at least 95%, 96%, 97%, 98%, 99% identity with the amino acid sequence as set forth in SEQ ID NO:78, and comprising an addition of KS at the C-terminus and a mutation of 110T;
(11) comprising an amino acid sequence having at least 95%, 96%, 97%, 98%, 99% identity with the amino acid sequence as set forth in SEQ ID NO:80, and comprising KS at the C-terminus and a mutation of 59D-82D-110T;
(12) comprising an amino acid sequence having at least 95%, 96%, 97%, 98%, 99% identity with the amino acid sequence as set forth in SEQ ID NO:82, and comprising KS at the C-terminus and a mutation of 59S-82D-110T;
(13) comprising an amino acid sequence having at least 95%, 96%, 97%, 98%, 99% identity with the amino acid sequence as set forth in SEQ ID NO:84, and comprising KS at the C-terminus and a mutation of 59D-82D-110T-56E;
(14) comprising an amino acid sequence having at least 95%, 96%, 97%, 98%, 99% identity with the amino acid sequence as set forth in SEQ ID NO:86, and comprising KS at the C-terminus and a mutation of 59S-82D-110T-56E.

26. A fusion protein comprising an anti-C5 antibody or antigen-binding fragment thereof and the hybrid protein mutant of any one of claims 1-25, wherein the antibody or antigen-binding fragment thereof is linked to the hybrid protein mutant with or without a linker.

27. The fusion protein of claim 26, wherein the one or more hybrid protein mutants (at their N-terminus or at their C-terminus) are respectively connected to the N-terminus and/or C-terminus of the heavy chain and/or light chain of the anti-C5 antibody, with or without a linker.

28. The fusion protein of any one of claims 1-27, wherein the anti-C5 antibody is a humanized or chimeric antibody.

29. The fusion protein of any one of claims 1-28, wherein the anti-C5 antibody is a monoclonal antibody.

30. The fusion protein of any one of claims 1-29, wherein the antigen-binding fragment of the anti-C5 antibody is selected from Fab, Fab', Fab'-SH, Fv, singel chain antibody(e.g., scFv), (Fab')$_2$, single domain antibody e.g., VHH, dAb(domain antibody), or a linear antibody.

31. The fusion protein of any one of claims 1-30, wherein the anti-C5 antibody or antigen-binding fragment thereof comprises an Fc region, preferably, wherein the Fc region is connected at its C-terminus to the N-terminus of the hybrid protein, with or without a linker.

32. The fusion protein of any one of claims 1-31, wherein the linker is selected from one or more glycine $(G)_n$, GS, $G_nS$, $G_nS_n$, $(G_nS)_n$ or $(GSG)_n$ or $(G_4S)_n$, wherein n is an integer equal to or greater than 1, for example, n is an integer of 2, 3, 4, 5, 6 or 7, for example, the linker is G, GSG or $G_4S$.

33. The fusion protein of any one of claims 1-32, comprising the full-length anti-C5 antibody and the hybrid protein, wherein

the anti-C5 antibody is connected at the C-terminus of its Fc region to the N-terminus of the hybrid protein to form the heavy chain of the fusion protein (with or without a linker);
the light chain of the anti-C5 antibody forms the light chain of the fusion protein.

34. The fusion protein of any one of claims 1-33, wherein said anti-C5 antibody or antigen-binding fragment thereof is selected from

i) the anti-C5 antibody or the antigen-binding fragment thereof disclosed in CN113754763A; or
ii) Eculizumab, Ravulizumab, Pozelimab, Crovalimab, Tesidolumab or the antigen-binding fragment thereof.

35. The fusion protein of any one of claims 1-33, wherein the anti-C5 antibody or antigen-binding fragment thereof comprising three complementarity determining regions (CDRs) of a heavy chain variable region (VH), HCDR1, HCDR2, and HCDR3, and three CDRs of a light chain variable region (VL), LCDR1, LCDR2, and LCDR3, wherein the HCDR1, HCDR2, and HCDR3 are the three CDRs contained in the VH as set forth in SEQ ID NO: 39; and the LCDR1, LCDR2, and LCDR3 are the three CDRs contained in the VL as set forth in SEQ ID NO: 45.

36. The fusion protein of any one of claims 1-33, wherein the anti-C5 antibody or antigen-binding fragment thereof comprising:

- HCDR1 comprising or consisting of the amino acid sequence as set forth in SEQ ID NO: 40,
- HCDR2 comprising or consisting of the amino acid sequence as set forth in SEQ ID NO: 41,
- HCDR3 comprising or consisting of the amino acid sequence as set forth in SEQ ID NO: 42,
- LCDR1 comprising or consisting of the amino acid sequence as set forth in SEQ ID NO: 46,
- LCDR2 comprising or consisting of the amino acid sequence as set forth in SEQ ID NO: 47, and
- LCDR3 comprising or consisting of the amino acid sequence as set forth in SEQ ID NO: 48.

37. The fusion protein of claim 35 or 36, wherein the anti-C5 antibody or antigen-binding fragment thereof comprises a heavy chain variable region VH, wherein the heavy chain variable region

(i) comprises or consists of an amino acid sequence having at least 90%, 91%, 92%, 93%, 94%, 95%, 96%, 97%, 98%, or 99% identity with the amino acid sequence of SEQ ID NO: 39; or
(ii) comprises or consists of the amino acid sequence of SEQ ID NO: 39.

38. The fusion protein of any one of claims 35-37, wherein the anti-C5 antibody or antigen-binding fragment thereof comprises a light chain variable region VL, wherein the light chain variable region

(i) comprises or consists of an amino acid sequence having at least 90%, 91%, 92%, 93%, 94%, 95%, 96%, 97%, 98%, or 99% identity with the amino acid sequence of SEQ ID NO: 45; or
(ii) comprises or consists of the amino acid sequence of SEQ ID NO: 45.

39. The fusion protein of claim 35 or 36, wherein the anti-C5 antibody or antigen-binding fragment thereof comprises a heavy chain variable region VH and a light chain variable region VL, wherein

- the heavy chain variable region comprises or consists of the amino acid sequence of SEQ ID NO: 39 or an amino acid sequence having at least 90%, 91%, 92%, 93%, 94%, 95%, 96%, 97%, 98%, or 99% identity therewith; and
- the light chain variable region comprises or consists of the amino acid sequence of SEQ ID NO: 45 or an amino acid sequence having at least 90%, 91%, 92%, 93%, 94%, 95%, 96%, 97%, 98%, or 99% identity therewith.

40. The fusion protein of claim 35 or 36, wherein the anti-C5 antibody or antigen-binding fragment thereof comprises a heavy chain variable region VH and a light chain variable region VL, wherein the VH comprises or consists of the amino acid sequence of SEQ ID NO: 39 and the VL comprises or consists of the amino acid sequence of SEQ ID NO: 45.

41. The fusion protein of any one of claims 35-40, wherein the anti-C5 antibody or antigen-binding fragment thereof further comprises a heavy chain constant region HC, e.g., the heavy chain constant region (HC) is the heavy chain constant region of IgG1, IgG2, IgG3, or IgG4, preferably the heavy chain constant region of IgG2 or of IgG4, or the heavy chain constant region of an IgG2/IgG4 hybrid form.

42. The fusion protein of claim 41, the heavy chain constant region

(i) comprises or consists of an amino acid sequence having at least 90%, 91%, 92%, 93%, 94%, 95%, 96%, 97%, 98%, or 99% identity with SEQ ID NO: 43; or
(ii) comprises or consists of the amino acid sequence of SEQ ID NO: 43; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 43.

43. The fusion protein of claim 41 or 42, wherein the heavy chain constant region comprises one or more mutations enhancing FcRn binding, e.g., YTE mutaion (M252Y/S254T/T256E), LA mutation(M428L/N434A), or LS mutation (M428L/N434S)), preferably comprises LA mutation; and/or comprises mutations enhancing stability, e.g., S228P.

44. The fusion protein of any one of claims 35-43, wherein the anti-C5 antibody or antigen-binding fragment thereof comprises a light chain constant region, e.g., said light chain constant region is a lambda or a kappa constant region.

45. The antibody or antigen-binding fragment thereof according to claim 44, wherein the light chain constant region:

(i) comprises or consists of an amino acid sequence having at least 90%, 91%, 92%, 93%, 94%, 95%, 96%, 97%, 98%, or 99% identity with the amino acid sequence of SEQ ID NO: 49; or
(ii) comprises or consists of the amino acid sequence of SEQ ID NO: 49; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 49.

46. The fusion protein of any one of claims 35-45, wherein the anti-C5 antibody or antigen-binding fragment thereof comprises a heavy chain, wherein the heavy chain

(i) comprises or consists of an amino acid sequence having at least 90%, 91%, 92%, 93%, 94%, 95%, 96%, 97%, 98%, or 99% identity with the amino acid sequence of SEQ ID NO: 44; or
(ii) comprises or consists of the amino acid sequence of SEQ ID NO: 44; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 44, preferably the amino acid change(s) occurs outside the CDR region, preferably, the amino acid change(s) occurs outside the heavy chain variable regions.

47. The fusion protein of any one of claims 35-46, wherein the anti-C5 antibody or antigen-binding fragment thereof comprises a light chain, wherein the light chain

(i) comprises or consists of an amino acid sequence having at least 90%, 91%, 92%, 93%, 94%, 95%, 96%, 97%, 98%, or 99% identity with the amino acid sequence of SEQ ID NO: 50; or
(ii) comprises or consists of the amino acid sequence of SEQ ID NO: 50; or
(iii) comprises or consists of an amino acid sequence having one or more (preferably no more than 10, more preferably no more than 5, 4, 3, 2, or 1) amino acid changes (preferably substitutions, more preferably conservative substitutions) compared to the amino acid sequence of SEQ ID NO: 50, preferably the amino acid change(s) occurs outside the CDR region, preferably, the amino acid change(s) occurs outside the heavy chain variable regions.

48. The fusion protein of claim 46 or 47, wherein the anti-C5 antibody or antigen-binding fragment thereof comprises a heavy chain and a light chain, wherein

- the heavy chain comprises or consists of the amino acid sequence of SEQ ID NO: 44 or an amino acid sequence having at least 85%, 90%, 91%, 92%, 93%, 94%, 95%, 96%, 97%, 98%, or 99% identity therewith; and
- the light chain comprises or consists of the amino acid sequence of SEQ ID NO: 50 or an amino acid sequence having at least 85%, 90%, 91%, 92%, 93%, 94%, 95%, 96%, 97%, 98%, or 99% identity therewith.

49. The fusion protein of claim 48, wherein the anti-C5 antibody or antigen binding fragment thereof comprises a heavy chain and a light chain, wherein the heavy chain comprises or consists of the amino acid sequence as set forth in SEQ ID NO: 44 and the light chain comprises or consists of the amino acid sequence as set forth in SEQ ID NO: 50.

50. The fusion protein of claim 33, wherein the fusion protein comprises two heavy chains and two light chains.

51. The fusion protein of claim 33 or 50, wherein the heavy chain of the fusion protein comprises or consists of the amino acid sequence of SEQ ID NO:61, 63, 65, 67, 69, 71, 73, 75, 77, 79, 81, 83, 85, or 87, or an amino acid sequence having at least 85%, 90%, 91%, 92%, 93%, 94%, 95%, 96%, 97%, 98% or 99% identity thereto; and/or the light chain of the fusion protein comprises or consists of the amino acid sequence of SEQ ID NO:50, or an amino acid sequence having at least 85%, 90%, 91%, 92%, 93%, 94%, 95%, 96%, 97%, 98% or 99% identity thereto.

52. A nucleic acid molecule encoding a hybrid protein mutant of any one of claims 1 to 25 or a fusion protein of any one of claims 26 to 51.

53. An expression vector comprising the nucleic acid molecule of claim 52, preferably the expression vector is pcDNA3.1.

54. A host cell comprising the nucleic acid molecule according to claim 52 or the expression vector according to claim 53, preferably, the host cell is prokaryotic or eukaryotic, such as a CHO cell, a 293 cell, such as an Expi293 cell.

55. A method for preparing the hybrid protein mutant of any one of claims 1-25 or the fusion protein of any one of claims 26-51, the method comprising culturing a host cell of claim 54 under conditions suitable for expression of the hybrid protein mutant or fusion protein, and optionally further comprising isolating the hybrid protein mutant or fusion protein from the host cell or the host cell culture medium, and/or purifying the hybrid protein mutant or fusion protein.

56. An immunoconjugate comprising the fusion protein of any one of claims 26-51, and other agents, such as anti-hemolytic drugs or labels.

57. A pharmaceutical composition or medicament or formulation comprising the hybrid protein mutant of any one of claims 1-25 or the fusion protein of any one of claims 26-51, and optionally a pharmaceutical excipient.

58. A pharmaceutical combination product comprising

the hybrid protein mutant of any one of claims 1-25 or the fusion protein of any one of claims 26-51; and
the additional therapeutic agents.

59. A method for preventing or treating a disease or condition associated with the complement system in a subject, comprising administering to the subject an effective amount of the hybrid protein mutant of any one of claims 1-25 or the fusion protein of any one of claims 26-51; or the immunoconjugate of claim 56 or the pharmaceutical composition or formuation of claim 57; or the pharmaceutical combination product of claim 58.

60. The method of claim 59, wherein the disease or condition is caused by abnormal activation of the complement system or dysregulation of the complement system or is a complement C5-associated disease or condition.

61. The method of claim 60, wherein the abnormal activation of the complement system or dysregulation of the complement system is due to, for example, microbial infection or an increase in autoimmune antibodies, or due to a reduction, loss, disability, or functional interference or blockage of complement regulatory proteins.

62. The method of claim 60, wherein the complement C5-associated disease or condition includes a disease phenotype caused by unregulated C5 function, such as due to dysregulated C5 activation, such as increased C5 activation.

63. The method of claim 60, wherein the complement C5-associated disease or condition is a disease or condition in which the subject has (e.g., elevated levels, such as nucleic acid or protein levels) complement C5 protein (e.g., compared to healthy subjects) or the subject has (e.g., elevated levels, such as nucleic acid or protein levels) complement C5 protein in the blood or blood cells (e.g., compared to the blood or blood cells of healthy subjects).

64. The method of claim 63, wherein the complement system-associated disease or disorder is selected from a disease requiring hemolysis inhibition, such as a disease requiring inhibition of hemolysis of the classical pathway of the complement immunity and/or the alternative pathway of the complement immunity; or a disease requiring inhibition of C3b deposition activity, such as dense deposition disease DDD.

65. A method for detecting the presence of complement C5 in a biological sample, comprising contacting the biological sample with the fusion protein according to any one of claims 26-51, under conditions that allow binding to complement C5, and detecting whether a complex is formed between the antibody or antigen-binding fragment thereof or the fusion protein and complement C5, wherein the formation of a complex indicates the presence of complement C5.

**CFH CCP1-4**
PDB: 5o35

CFH CCP1

**F1D34** (hybrid protein)

**CD97**
EGF-like domain 1-3

DAF CCP3-4

**DAF CCP1-4**
PDB: 7do4

Figure 1

Figure 2

**Figure 3**

## CP assay

| | CR13m | FH15 | F1D34-1 | F1D34-2i | CR15D34 |
|---|---|---|---|---|---|
| IC50 | 4.716 | 424.6 | 6.425 | 3.422 | |

Figure 4

## CP assay

| | F1D34-1 | F1D34-2i | D24 | CR15D34 | FH13 | D14 |
|---|---|---|---|---|---|---|
| IC50 | 4.98 | 2.592 | 6.231 | | | 92.52 |

Figure 4 (continued)

**AP assay**

| | CR13m | FH15 | F1D34-1 | F1D34-2i | CR15D34 |
|---|---|---|---|---|---|
| IC50 | 172.9 | 281.4 | 60.15 | 51.90 | 310.7 |

Figure 5

**AP assay**

| | F1D34-1 | F1D34-2i | D24 | CR15D34 | FH13 | D14 |
|------|---------|----------|-------|---------|------|------|
| IC50 | 25.79 | 31.05 | 220.1 | 120.6 | | 1646 |

Figure 5 (continued)

**C3b deposition assay**

| | CR13m | FH15 | F1D34-1 | F1D34-2i | CR15D34 |
|---|---|---|---|---|---|
| IC50 | 27.85 | 38.52 | 9.203 | 7.748 | 21.79 |

Figure 6

**C3b deposition assay**

| | F1D34-1 | F1D34-2i | D24 | CR15D34 | FH13 | D14 |
|---|---|---|---|---|---|---|
| IC50 | 9.413 | 11.19 | 135.5 | 62.16 | | 205.3 |

Figure 6 (continued)

Figure 7

Figure 8

Figure 9

N'-terminus

Aggregation

0.500
0.333
0.167
0.000
-0.167
-0.333
-0.500

C'-terminus

Figure 10

**AP assay (rRBC in 15% NHS)**

| | C'-KS | L56E | L59D | L59S | G82D | F86Y | F107E | L109D | L109E | I110T | Ab-F1D34 |
|---|---|---|---|---|---|---|---|---|---|---|---|
| IC50 | 3.027 | 1.979 | 1.862 | 3.101 | 2.398 | 1.896 | 2.197 | 2.766 | 3.575 | 2.209 | 2.745 |

Figure 11

**CP assay**
**( sRBC in 1% NHS)**

Legend: C'-KS, L56E, L59D, L59S, G82D, F86Y, L109D, L109E, I110T, Ab-F1D34

| | C'-KS | L56E | L59D | L59S | G82D | F86Y | L109D | L109E | I110T | Ab-F1D34 |
|---|---|---|---|---|---|---|---|---|---|---|
| IC50 | 0.1668 | 0.2126 | 0.1754 | 0.1498 | 0.1749 | 0.4649 | 0.3133 | 0.5831 | 0.1755 | 0.1925 |

**Figure 12**

## CP assay
## ( sRBC in 1% NHS)

|  | C'-KS | F107E | Ab-F1D34 |
|------|--------|--------|----------|
| IC50 | 0.1398 | 0.3701 | 0.1781 |

Figure 12 (continued)

**AP assay (rRBC in 10% NHS)**

| | 59D/82D/110T | 59S/82D/110T | 59D/82D/110T/56E | 59S/82D/110T/56E | Ab-F1D34 |
|---|---|---|---|---|---|
| IC50 | 9.946 | 10.57 | 7.789 | 8.339 | 9.210 |

**Figure 13**

## CP assay(sRBCs in 1% NHS)

| | 59D/82D/110T | 59S/82D/110T | 59D/82D/110T/56E | 59S/82D/110T/56E | Ab-F1D34 |
|---|---|---|---|---|---|
| IC50 | 0.1658 | 0.1500 | 0.2510 | 0.2278 | 0.2720 |

B

**Figure 13(continued)**

Figure 14

**AP assay (rRBC in 10% NHS)**

Figure 14 (continued)

Figure 15

## CP assay (sRBCs in 5%NHS)

| | 59D/82D/110T/56E | Ab-F15 |
|---|---|---|
| IC50 | 0.8537 | 8.158 |

B

Figure 15 (continued)

**C3b Deposition Assay (rRBCs in 10% C7-Dpl NHS)**

Legend:
- ● 59D/82D/110T/56E
- ■ Eculizumab

| | 59D/82D/110T/56E | Eculizumab |
|---|---|---|
| IC50 | 13.33 | 6.196 |

A

Figure 16

## C3b Deposition Assay (rRBCs in 10% C6-Dpl NHS)

| | 59D/82D/110T/56E | Ab-F15 |
|---|---|---|
| IC50 | 24.58 | 152.0 |

B

Figure 16 (continued)

## C3b Deposition Assay (Wieslab assay)

| | 59D/82D/110T/56E | Eculizumab | Ab-F15 |
|---|---|---|---|
| IC50 | 17.50 | 23.91 | 57.53 |

Figure 17

**LP assay (Wieslab assay)**

| | Eculizumab | 59D/82D/110T/56E | Ab-F15 |
|---|---|---|---|
| IC50 | 1.909 | 0.6730 | 5.338 |

Figure 18

Figure 19

Figure 19 (continued)

Figure 19 (continued)

**G** F1D34-KS-G82D

| RT [min] | Name | Area | Main peak% |
|---|---|---|---|
| 8.670 | | 4.9 | 0.060 |
| 8.770 | | 7.4 | 0.091 |
| 9.629 | | 21.8 | 0.268 |
| 10.807 | | 21.2 | 0.260 |
| 12.313 | | 30.2 | 0.372 |
| 12.912 | | 40.3 | 0.495 |
| 13.427 | | 137.0 | 1.684 |
| 15.026 | | 7797.7 | 95.871 |
| 16.138 | | 17.4 | 0.214 |
| 16.300 | | 55.7 | 0.685 |
| | Sum | 8133.5 | |

**H** F1D34-KS-I110T

| RT [min] | Name | Area | Main peak% |
|---|---|---|---|
| 8.793 | | 7.0 | 0.099 |
| 9.675 | | 9.8 | 0.140 |
| 10.805 | | 21.3 | 0.303 |
| 12.196 | | 24.7 | 0.352 |
| 12.940 | | 27.0 | 0.384 |
| 13.459 | | 90.9 | 1.296 |
| 15.049 | | 6816.0 | 97.110 |
| 16.089 | | 22.2 | 0.316 |
| | Sum | 7018.8 | |

Figure 19 (continued)

Figure 20

Figure 21

## CP assay

| | F1D34-1 | F1D34-2i | F1D34-KS | F1D34-KS-L56E | F1D34-KS-L59D | F1D34-KS-L59S | F1D34-KS-G82D | F1D34-KS-I110T |
|---|---|---|---|---|---|---|---|---|
| IC50 | 28.47 | 23.62 | 18.62 | 19.52 | 22.29 | 13.85 | 16.25 | 28.45 |

A

**Figure 22**

**AP assay**

| | F1D34-1 | F1D34-2i | F1D34-KS | F1D34-KS-L56E | F1D34-KS-L59D | F1D34-KS-L59S | F1D34-KS-G82D | F1D34-KS-I110T |
|---|---|---|---|---|---|---|---|---|
| IC50 | 39.99 | 43.07 | 61.62 | 67.03 | 53.78 | 30.11 | 42.96 | 54.79 |

**Figure 22(continued)**

## INTERNATIONAL SEARCH REPORT

International application No.

**PCT/CN2023/130258**

**A.   CLASSIFICATION OF SUBJECT MATTER**

C07K 19/00(2006.01)i;  C12N 15/62 (2006.01)i;  C12N 5/10(2006.01)i;  C12N 15/85(2006.01)i;  A61K 38/17(2006.01)i;  A61P 7/04(2006.01)i;  A61P 43/00(2006.01)i

According to International Patent Classification (IPC) or to both national classification and IPC

**B.   FIELDS SEARCHED**

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

CNTXT, CNABS, WPABSC, ENTXTC, ENTXT, DWPI, VEN, 中国专利生物序列检索系统, China Patent Biological Sequence Search System, STN, GenBank, EBI-EMBL, CNKI, 百度学术, Baidu Scholar, PubMed, ISI Web ofKnowledge: 申请人/发明人, applicant/inventor, 补体, complement, 因子H, 因子H, CFH, FH, 衰变加速因子, DAF, CD55, 杂合, 融合, 嵌合, chimera, hybrid, fusion, CCP1, SCR1, CCP3-4, SCR3-4, V62I, L56E, L59D/S, G82D, F86Y, F107E, L109D/E, I110T, KS, SEQ ID NOs: 18-33, 39-86

**C.   DOCUMENTS CONSIDERED TO BE RELEVANT**

| Category* | Citation of document, with indication, where appropriate, of the relevant passages | Relevant to claim No. |
|---|---|---|
| PA | CN 116891533 A (TIANCHEN BIOMEDICAL (SUZHOU) CO., LTD.) 17 October 2023 (2023-10-17)<br>        entire document | 1-65 |
| A | CN 109689084 A (UNIVERSITÄT ULM) 26 April 2019 (2019-04-26)<br>        entire document, in particular claims 1-24, and description, embodiments 1-6 | 1-65 |
| A | WO 2022134047 A1 (THE TRUSTEES OF THE UNIVERSITY OF PENNSYLVANIA et al.) 30 June 2022 (2022-06-30)<br>        entire document, in particular description, paragraphs 0158-0174 | 1-65 |
| A | CN 113754763 A (TIANCHEN BIOMEDICAL (SUZHOU) CO., LTD.) 07 December 2021 (2021-12-07)<br>        entire document, in particular claims 1-10 | 1-65 |
| A | CN 113365648 A (UNIVERSITÄT ULM) 07 September 2021 (2021-09-07)<br>        entire document | 1-65 |

☑ Further documents are listed in the continuation of Box C.    ☑ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **13 December 2023** | **20 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**EP 4 617 287 A1**

**INTERNATIONAL SEARCH REPORT**

| International application No. |
| --- |
| **PCT/CN2023/130258** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 113660944 A (Q32 BIOLOGICAL INC.) 16 November 2021 (2021-11-16)<br>entire document | 1-65 |
| A | CN 114072174 A (THE TRUSTEES OF THE UNIVERSITY OF PENNSYLVANIA et al.) 18 February 2022 (2022-02-18)<br>entire document | 1-65 |
| A | WO 2020041638 A1 (ALEXION PHARMA, INC.) 27 February 2020 (2020-02-27)<br>entire document | 1-65 |
| A | LUKAWSKA, E. et al. "The Role of the Alternative Pathway of Complement Activation in Glomerular Diseases"<br>*Clinical and Experimental Medicine,* Vol. vol. 18, 15 February 2018 (2018-02-15),<br>pages 297-318 | 1-65 |
| A | FORNERIS, F. et al. "Regulators of Complement Activity Mediate Inhibitory Mechanisms through a Common C3b-binding Mode"<br>*The EMBO Journal,* Vol. vol. 35, 24 March 2016 (2016-03-24),<br>pages 1133-1149 | 1-65 |

Form PCT/ISA/210 (second sheet) (July 2022)

107

## INTERNATIONAL SEARCH REPORT

| | |
|---|---|
| International application No. | |
| | **PCT/CN2023/130258** |

**Box No. I     Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| | International application No. |
|---|---|
| | **PCT/CN2023/130258** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **59-64**
   because they relate to subject matter not required to be searched by this Authority, namely:

   Claims 59-64 relate to a method for preventing or treating a complement system-related disease or condition in a subject, and thus do not comply with PCT Rule 39.1(iv). The present report is provided on the basis of the modification of the subject matter of claims 59-64 to a corresponding pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| | International application No. |
|---|---|
| | **PCT/CN2023/130258** |

| Patent document cited in search report | | | Publication date (day/month/year) | Patent family member(s) | | | Publication date (day/month/year) |
|---|---|---|---|---|---|---|---|
| CN | 116891533 | A | 17 October 2023 | WO | 2023197930 | A1 | 19 October 2023 |
| CN | 109689084 | A | 26 April 2019 | WO | 2018002131 | A1 | 04 January 2018 |
| | | | | AU | 2017289214 | A1 | 24 January 2019 |
| | | | | AU | 2017289214 | B2 | 18 November 2021 |
| | | | | CA | 3029106 | A1 | 04 January 2018 |
| | | | | MX | 2019000059 | A | 07 September 2020 |
| | | | | US | 2019202878 | A1 | 04 July 2019 |
| | | | | PL | 3474883 | T3 | 19 September 2022 |
| | | | | IL | 263949 | A | 31 January 2019 |
| | | | | JP | 2019523238 | A | 22 August 2019 |
| | | | | JP | 7068203 | B2 | 16 May 2022 |
| | | | | ES | 2925466 | T3 | 18 October 2022 |
| | | | | EP | 3474883 | A1 | 01 May 2019 |
| | | | | EP | 3474883 | B1 | 25 May 2022 |
| | | | | KR | 20190039090 | A | 10 April 2019 |
| | | | | KR | 102566634 | B1 | 14 August 2023 |
| | | | | DK | 3474883 | T3 | 29 August 2022 |
| WO | 2022134047 | A1 | 30 June 2022 | EP | 4267612 | A1 | 01 November 2023 |
| CN | 113754763 | A | 07 December 2021 | WO | 2021244627 | A1 | 04 June 2021 |
| | | | | US | 2023212272 | A1 | 06 July 2023 |
| CN | 113365648 | A | 07 September 2021 | US | 2021355178 | A1 | 18 November 2021 |
| | | | | WO | 2019243586 | A1 | 26 December 2019 |
| | | | | EP | 3810166 | A1 | 28 April 2021 |
| | | | | EP | 3586860 | A1 | 01 January 2020 |
| | | | | JP | 2021527439 | A | 14 October 2021 |
| CN | 113660944 | A | 16 November 2021 | WO | 2020123662 | A2 | 18 June 2020 |
| | | | | WO | 2020123662 | A3 | 23 July 2020 |
| | | | | JP | 2022512234 | A | 02 February 2022 |
| | | | | MX | 2021006930 | A | 17 November 2021 |
| | | | | MA | 54468 | A | 13 April 2022 |
| | | | | AU | 2019398214 | A1 | 01 July 2021 |
| | | | | AU | 2019398214 | B2 | 23 November 2023 |
| | | | | IL | 283841 | A | 29 July 2021 |
| | | | | EP | 3893916 | A2 | 20 October 2021 |
| | | | | EP | 3893916 | A4 | 29 March 2023 |
| | | | | CA | 3123004 | A1 | 18 June 2020 |
| | | | | SG | 11202106073 | QA | 29 July 2021 |
| | | | | KR | 20210148076 | A | 07 December 2021 |
| CN | 114072174 | A | 18 February 2022 | MX | 2021012997 | A | 04 March 2022 |
| | | | | US | 2022204602 | A1 | 30 June 2022 |
| | | | | CA | 3137907 | A1 | 29 October 2020 |
| | | | | SG | 11202111419 | QA | 29 November 2021 |
| | | | | WO | 2020219922 | A1 | 29 October 2020 |
| | | | | EP | 3958901 | A1 | 02 March 2022 |
| | | | | EP | 3958901 | A4 | 19 July 2023 |
| | | | | KR | 20220003001 | A | 07 January 2022 |
| | | | | AU | 2020261073 | A1 | 16 December 2021 |
| | | | | JP | 2022529527 | A | 22 June 2022 |
| | | | | IL | 287232 | A | 01 December 2021 |

Form PCT/ISA/210 (patent family annex) (July 2022)

## INTERNATIONAL SEARCH REPORT
### Information on patent family members

| Patent document cited in search report | Publication date (day/month/year) | Patent family member(s) | Publication date (day/month/year) |
|---|---|---|---|
| WO 2020041638 A1 | 27 February 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 113754763 A
- US 9988611 B
- US 20120128674 A1

### Non-patent literature cited in the description

- The Complement FactsBook.. Elsevier Ltd., 2018
- **KABAT et al.** Sequences of Proteins of Immunological Interest. National Institutes of Health, 1987
- **NORTH et al.** A New Clustering of Antibody CDR Loop Conformations. *Journal of Molecular Biology*, 2011, vol. 406, 228-256
- **KABAT et al.** Sequences of Proteins of Immunological Interest. National Institutes of Health, 1991
- **EISENBERG et al.** *J.Mol.Biol.*, 1984, vol. 179, 125-142
- **GESTUR VIDARSSON et al.** *IgG subclasses and allotypes: from structure to effector functions*, 20 October 2014
- **MERCHANT, A. M. et al.** An efficient route to human bispecific IgG.. *Nat Biotechnol*, 1998, 677-681
- **R.C. ROWE** ; **P.J. SESKEY** ; **S.C. OWEN**. Handbook of Pharmaceutical Excipients. Pharmaceutical Press
- **HUI-FEN ZHANG et al.** *THE JOURNAL OF BIOLOGICAL CHEMISTRY*, July 2001, vol. 276 (29), 27290-27295
- **MASHA FRIDKIS-HARELI et al.** *Blood.*, 27 October 2011, vol. 118 (17), 4705-4713
- **AGUSTÍN TORTAJADA et al.** *Hum Mol Genet.*, 15 September 2009, vol. 18 (18), 3452-3461
- **JÄGER, V. et al.** *BMC Biotechnol*, 2013, vol. 13, 52
- **CASIM A. SARKAR et al.** *Nat Biotechnol.*, September 2002, vol. 20 (9), 908-13
- **TOMOYUKI IGAWA et al.** *Nat Biotechnol.*, November 2010, vol. 28 (11), 1203-7